# EUROPÄISCHE PATENTSCHRIFT

(11) **EP 2 935 276 B1**
(45) Veröffentlichungstag und Bekanntmachung des Hinweises auf die Patenterteilung: **01.11.2017**
(21) Anmeldenummer: 13805770.8
(22) Anmeldetag: 27.11.2013
(51) Int. Cl.: C07D 487/10, C07D 209/86, C09K 11/06, H01L 51/00, H01L 27/32

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ORGANIQUES ÉLECTROLUMINESCENTS

(30) Priorität: 21.12.2012 EP 12008584
(43) Veröffentlichungstag der Anmeldung: 28.10.2015
(73) Patentinhaber: Merck Patent GmbH, 64293 Darmstadt (DE)
(72) Erfinder: JATSCH, Anja, D-60489 Frankfurt am Main (DE); PFLUMM, Christof, D-64291 Darmstadt (DE); PARHAM, Amir Hossain, D-60486 Frankfurt am Main (DE); EBERLE, Thomas, D-76829 Landau (DE); STOESSEL, Philipp, D-60487 Frankfurt am Main (DE); KROEBER, Jonas Valentin, D-60311 Frankfurt am Main (DE); LINGE, Rouven, D-64291 Darmstadt (DE)
(86) Internationale Anmeldenummer: PCT/EP2013/003583
(87) Internationale Veröffentlichungsnummer: WO 2014/094963

(56) Entgegenhaltungen:
- WO-A1-2011/000455
- WO-A2-2012/074210

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen enthaltend diese Materialien.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, jedoch immer noch Verbesserungsbedarf, beispielsweise im Hinblick auf Effizienz, Betriebsspannung und Lebensdauer.

Die Eigenschaften phosphoreszierender OLEDs werden nicht nur von den eingesetzten Triplettemittern bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen.

Gemäß dem Stand der Technik werden unter anderem Indolocarbazolderivate, z. B. gemäß WO 2007/063754 oder WO 2008/056746, Indenocarbazolderivate, z. B. gemäß WO 2010/136109, oder Fluoren- bzw. Spirobifluorenderivate, z. B. gemäß WO 2012/074210, als Matrixmaterialien für phosphoreszierende Emitter in organischen Elektrolumineszenzvorrichtungen eingesetzt. Hier sind weitere Verbesserungen wünschenswert, insbesondere in Bezug auf die Effizienz, die Lebensdauer und die thermische Stabilität der Materialien.

Aufgabe der vorliegenden Erfindung ist die Bereitstellung von Verbindungen, welche sich für den Einsatz in einer OLED eignen, insbesondere als Matrixmaterial für phosphoreszierende Emitter, aber auch als Lochblockiermaterial, als Elektronentransportmaterial oder gegebenenfalls als Lochtransport- und/oder Elektronenblockiermaterial. Eine weitere Aufgabe der vorliegenden Erfindung ist es, weitere organische Halbleiter für organische Elektrolumineszenzvorrichtungen bereitzustellen, um so dem Fachmann eine größere Wahlmöglichkeit an Materialien für die Herstellung von OLEDs zu ermöglichen.

Überraschend wurde gefunden, dass bestimmte, unten näher beschriebene Verbindungen diese Aufgabe lösen, sich gut für die Verwendung in OLEDs eignen und zu Verbesserungen der organischen Elektrolumineszenzvorrichtung führen. Dabei betreffen die Verbesserungen insbesondere die Lebensdauer und/oder die Betriebsspannung. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine Verbindung gemäß Formel (1) bzw. Formel (1A), 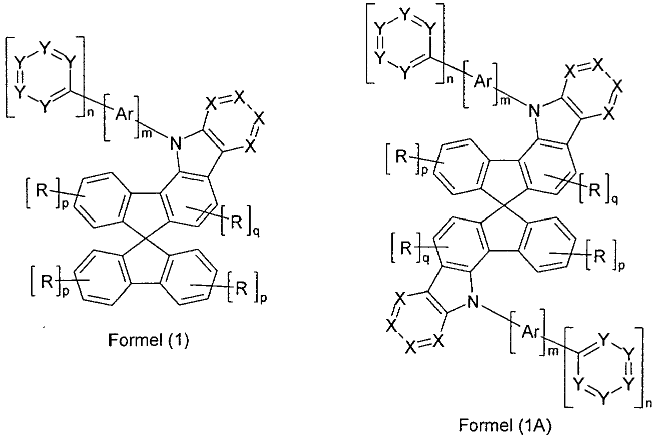 wobei für die verwendeten Symbole und Indizes gilt:
- Y: ist bei jedem Auftreten gleich oder verschieden CR¹ oder N, mit der Maßgabe, dass mindestens eine Gruppe Y für N steht;
- X: ist bei jedem Auftreten gleich oder verschieden CR¹ oder N; oder zwei benachbarte X stehen für S, O oder NR¹, so dass ein Fünfring entsteht; oder zwei benachbarte X stehen für eine Gruppe der folgenden Formel (2), (3) oder (4), 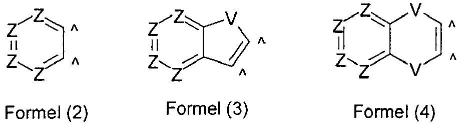 wobei ^ die entsprechenden benachbarten Gruppen X in der Formel (1) andeutet;
- V: ist bei jedem Auftreten gleich oder verschieden C(R¹)₂, NR¹, O, S, BR¹, Si(R¹)₂ oder C=O;
- Z: ist bei jedem Auftreten gleich oder verschieden CR¹ oder N;
- Ar: ist bei jedem Auftreten gleich oder verschieden ein aromatisches oder heteroaromatisches Ringsystem mit 5 bis 40 aromatischen Ringatomen, das mit einem oder mehreren Resten R¹ substituiert sein kann;
- R: ist bei jedem Auftreten gleich oder verschieden ausgewählt aus der Gruppe bestehend aus H, D, F, Cl, Br, I, CN, N(Ar¹)₂, einer geradkettigen Alkylgruppe mit 1 bis 40 C-Atomen oder einer verzweigten oder cyclischen Alkylgruppe mit 3 bis 40 C-Atomen, die jeweils mit einem oder mehreren Resten R² substituiert sein kann, wobei eine oder mehrere nicht-benachbarte CH₂-Gruppen durch R²C=CR², C=C oder O ersetzt sein können und wobei ein oder mehrere H-Atome durch D oder F ersetzt sein können, oder einem aromatischen Ringsystem mit 6 bis 60 aromatischen Ringatomen, das mit einem oder mehreren Resten R² substituiert sein kann, wobei das aromatische Ringsystem keine Heteroarylgruppen enthält; dabei können zwei benachbarte Substituenten R ein monocyclisches oder polycyclisches, aliphatisches oder aromatisches Ringsystem bilden, das mit einem oder mehreren Resten R² substituiert sein kann;
- R¹: ist bei jedem Auftreten gleich oder verschieden ausgewählt aus der Gruppe bestehend aus H, D, F, Cl, Br, I, CN, NO₂, N(Ar¹)₂, N(R²)₂, C(=O)Ar¹, C(=O)R², P(=O)(Ar¹)₂, P(Ar¹)₂, B(Ar¹)₂, Si(Ar¹)₃, Si(R²)₃, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C≡C, Si(R²)₂, C=O, C=S, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen oder heteroaromatischen Ring-system mit 5 bis 60 aromatischen Ringatomen, das jeweils mit einem oder mehreren Resten R² substituiert sein kann, einer Aryl-oxy- oder Heteroaryloxygruppe mit 5 bis 60 aromatischen Ring-atomen, die mit einem oder mehreren Resten R² substituiert sein kann; dabei können optional zwei benachbarte Substituenten R¹ ein monocyclisches oder polycyclisches, aliphatisches, aromatisches oder heteroaromatisches Ringsystem bilden, das mit einem oder mehreren Resten R² substituiert sein kann;
- Ar¹: ist bei jedem Auftreten gleich oder verschieden ein aromatisches oder heteroaromatisches Ringsystem mit 5-30 aromatischen Ringatomen, das mit einem oder mehreren nicht-aromatischen Resten R² substituiert sein kann; dabei können zwei Reste Ar¹, welche an dasselbe N-Atom oder P-Atom binden, auch durch eine Einfachbindung oder eine Brücke, ausgewählt aus N(R²), C(R²)₂, O oder S, miteinander verbrückt sein;
- R²: ist bei jedem Auftreten gleich oder verschieden ausgewählt aus der Gruppe bestehend aus H, D, F, CN, einem aliphatischen Kohlenwasserstoffrest mit 1 bis 20 C-Atomen, oder einem aromatischen oder heteroaromatischen Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R² miteinander ein mono- oder polycyclisches, aliphatisches Ringsystem bilden können;
- m, n: ist bei jedem Auftreten gleich oder verschieden 0 oder 1 mit der Maßgabe, dass m + n ≥ 1;
- p: ist bei jedem Auftreten gleich oder verschieden 0, 1, 2, 3 oder 4;
- q: ist 0, 1 oder 2.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthraxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Unter benachbarten Resten bzw. benachbarten Substituenten im Sinne der vorliegenden Anmeldung werden Substituenten verstanden, die an C-Atome gebunden sind, welche wiederum direkt aneinander gebunden sind, oder Substituenten, die an dasselbe C-Atom gebunden sind.

In einer bevorzugten Ausführungsform der Erfindung steht X gleich oder verschieden bei jedem Auftreten für CR¹ oder N, wobei maximal eine Gruppe X pro Cyclus für N steht; oder zwei benachbarte Gruppen X stehen für eine Gruppe der Formel (2) oder (3), insbesondere Formel (3), wobei Z gleich oder verschieden bei jedem Auftreten für CR¹ steht und V gleich oder verschieden bei jedem Auftreten für NR¹, C(R¹)₂, O oder S steht. Weiterhin bevorzugt bilden benachbarte Reste R¹, die an X vorhanden sind, keinen Ring miteinander. Besonders bevorzugt steht X gleich oder verschieden bei jedem Auftreten für CR¹.

Bevorzugte Ausführungsformen der Verbindungen gemäß Formel (1) sind die Verbindungen der folgenden Formeln (5) bis (11) und bevorzugte Ausführungsformen der Verbindungen gemäß Formel (1A) sind die Verbindungen der folgenden Formel (12), 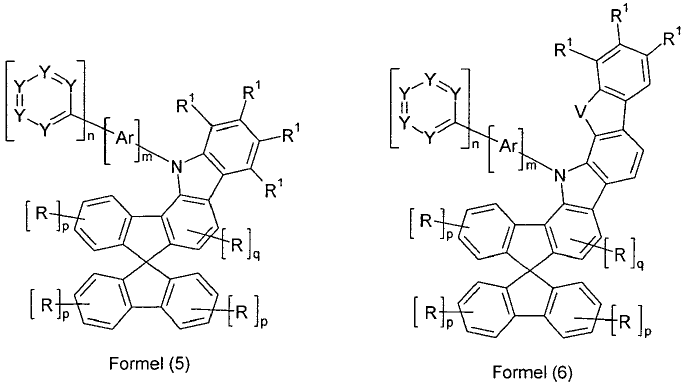 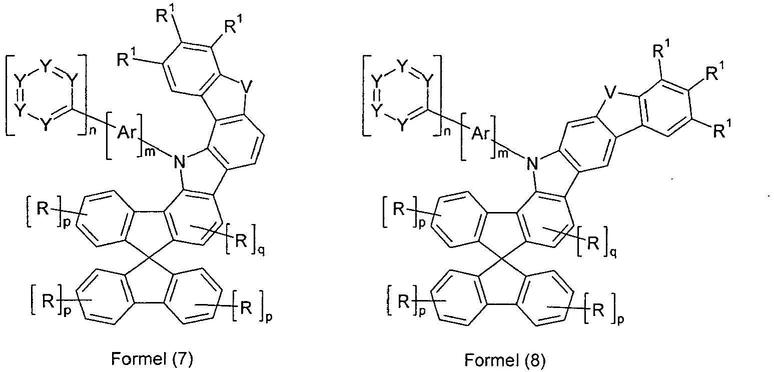 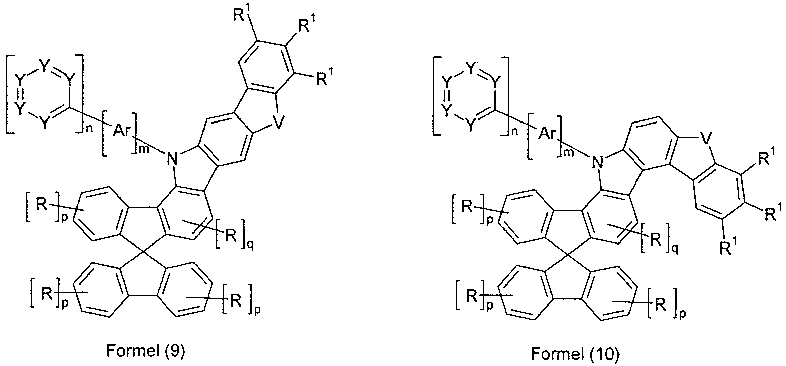 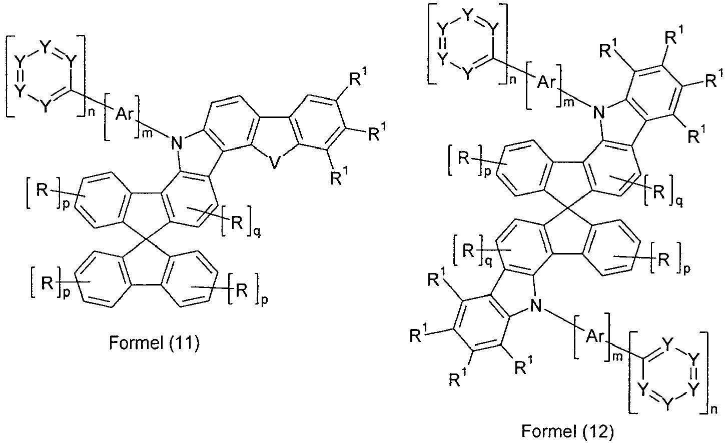 wobei die verwendeten Symbole und Indizes die oben genannten Bedeutungen aufweisen. Dabei steht V bevorzugt für NR¹, C(R¹)₂, O oder S. Es kann bevorzugt sein, wenn für V = C(R¹)₂ die beiden Reste R¹ miteinander einen Ring bilden und so ein Spirosystem aufspannen.

In einer bevorzugten Ausführungsform der Erfindung ist p bei jedem Auftreten gleich oder verschieden 0, 1 oder 2, besonders bevorzugt 0 oder 1 und ganz besonders bevorzugt gleich 0.

Weiterhin bevorzugt ist q gleich 0 oder 1, besonders bevorzugt gleich 0.

Besonders bevorzugte Ausführungsformen der Strukturen gemäß Formel (5) bis (12) sind die Strukturen der Formeln (5a) bis (12a), 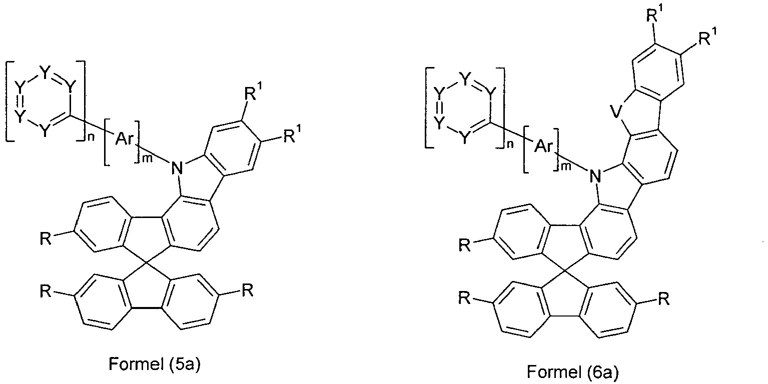 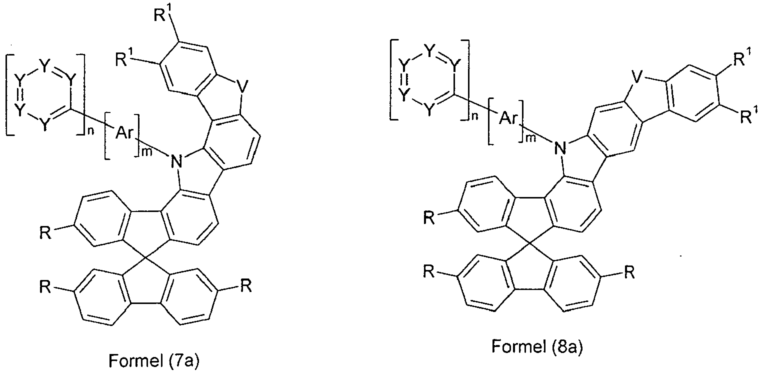 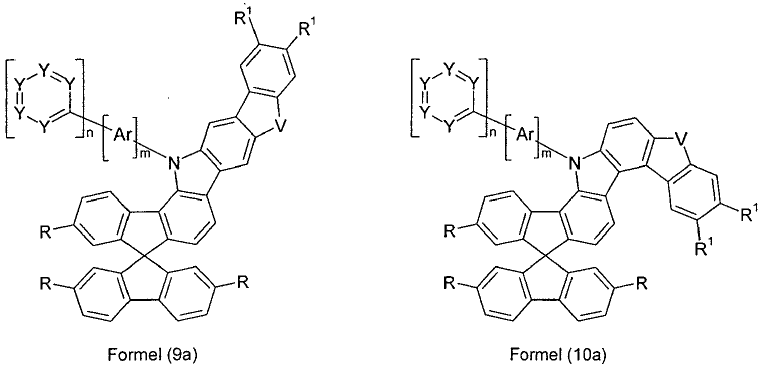 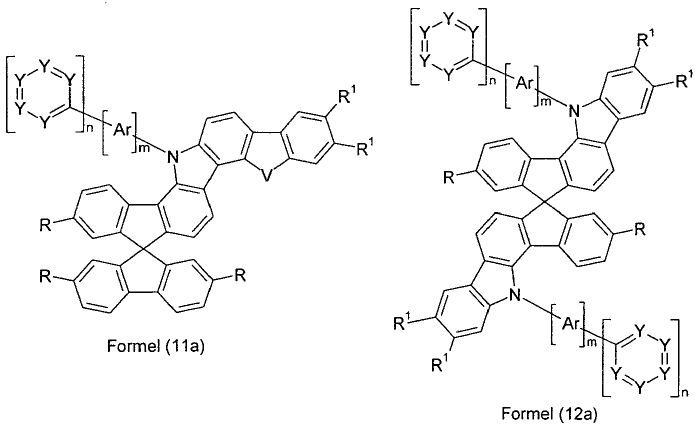 wobei die verwendeten Symbole und Indizes die oben genannten Bedeutungen aufweisen.

In einer bevorzugten Ausführungsform der Erfindung ist R gleich oder verschieden bei jedem Auftreten ausgewählt aus der Gruppe bestehend aus H, F, CN, N(Ar¹)₂, einer geradkettigen Alkylgruppe mit 1 bis 10 C-Atomen oder einer verzweigten oder cyclischen Alkylgruppe mit 3 bis 10 C-Atomen oder einem aromatischen Ringsystem mit 6 bis 30 aromatischen Ringatomen, das mit einem oder mehreren nicht-aromatischen Resten R² substituiert sein kann. In einer besonders bevorzugten Ausführungsform der Erfindung ist R gleich oder verschieden bei jedem Auftreten ausgewählt aus der Gruppe bestehend aus H, einer geradkettigen Alkylgruppe mit 1 bis 4 C-Atomen oder einer verzweigten oder cyclischen Alkylgruppe mit 3 bis 8 C-Atomen, insbesondere gleich H. Wenn die erfindungsgemäße Verbindung als Monomer zur Erzeugung eines Polymers eingesetzt wird, kann es auch bevorzugt sein, wenn zwei Substituenten R für Br oder I stehen und die Polymerisation über diese Gruppen durchgeführt wird.

In einer weiteren bevorzugten Ausführungsform der Erfindung ist R¹ gleich oder verschieden bei jedem Auftreten ausgewählt aus der Gruppe bestehend aus H, D, F, Br, CN, N(Ar¹)₂, C(=O)Ar¹, P(=O)(Ar¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenyl- oder Alkinylgruppe mit 2 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R² substituiert sein kann. Besonders bevorzugt ist R¹ gleich oder verschieden bei jedem Auftreten ausgewählt aus der Gruppe bestehend aus H, N(Ar¹)₂, einer geradkettigen Alkylgruppe mit 1 bis 4 C-Atomen oder einer verzweigten oder cyclischen Alkylgruppe mit 3 bis 8 C-Atomen, die jeweils mit einem oder mehreren Resten R² substituiert sein kann, oder einem aromatischen oder heteroaromatischen Ringsystem mit 5 bis 18 aromatischen Ringatomen, das jeweils mit einem oder mehreren Resten R² substituiert sein kann.

Wenn R für ein aromatisches Ringsystem steht bzw. wenn R¹ für ein aromatisches oder heteroaromatisches Ringsystem steht, dann ist dieses R bzw. R¹ bevorzugt gleich oder verschieden bei jedem Auftreten aus denselben Gruppen ausgewählt, wie unten als geeignete Gruppen für Ar angegeben.

Dabei haben in Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

In einer bevorzugten Ausführungsform der Erfindung ist n = 1 und m = 0. In einer weiteren bevorzugten Ausführungsform der Erfindung ist n = 0 und m = 1. In nochmals einer weiteren bevorzugten Ausführungsform der Erfindung ist n = m = 1.

Bevorzugte Gruppen Ar sind aromatische oder heteroaromatische Ringsysteme mit 5 bis 24 aromatischen Ringatomen, welche jeweils durch einen oder mehrere Reste R¹ substituiert sein können. Geeignete Gruppen Ar sind ausgewählt aus Benzol, ortho-, meta- oder para-Biphenyl, ortho-, meta-, para- oder verzweigtem Terphenyl, ortho-, meta-, para- oder verzweigtem Quaterphenyl, 1-, 2- oder 3-Fluorenyl, 1-, 2-, 3- oder 4-Spirobifluorenyl, 1- oder 2-Naphthyl, Pyrrol, Furan, Thiophen, Indol, Benzofuran, Benzothiophen, 1-, 2- oder 3-Carbazol, 1-, 2- oder 3-Dibenzofuran, 1-, 2- oder 3-Dibenzothiophen, Indenocarbazol, Indolocarbazol, 2-, 3- oder 4-Pyridin, 2-, 4- oder 5-Pyrimidin, Pyrazin, Pyridazin, Triazin, Anthracen, Phenanthren, Triphenylen, Pyren, Benzanthracen oder Kombinationen aus zwei oder drei dieser Gruppen, welche jeweils mit einem oder mehreren Resten R¹ substituiert sein können. Besonders bevorzugt steht Ar für ein aromatisches Ringsystem, insbesondere ausgewählt aus den Gruppen bestehend aus Benzol, ortho-, meta- oder para-Biphenyl, ortho-, meta-, para- oder verzweigtem Terphenyl und ortho-, meta-, para- oder verzweigtem Quaterphenyl. Wenn an die Gruppe Ar noch eine Gruppe (Het-Ar), wie unten genauer beschrieben, gebunden ist, wenn also n = m = 1 ist, dann ist die Gruppe (Het-Ar) an einer beliebigen Stelle an Ar gebunden.

In einer bevorzugten Ausführungsform der Erfindung ist Ar ein aromatisches Ringsystem, enthält also keine Heteroarylgruppen. Dies gilt sowohl, wenn n = 1 ist und an Ar noch eine Gruppe (Het-Ar), wie unten beschrieben, gebunden ist, als auch für n = 0.

In einer weiteren bevorzugten Ausführungsform der Erfindung sind die aromatischen Gruppen in der Gruppe Ar, wenn Ar mehr als eine Arylgruppe enthält, nicht para-verknüpft, d. h. es handelt sich bevorzugt nicht um para-Biphenyl, para-Terphenyl oder para-Quaterphenyl, sondern beispielsweise um die jeweiligen ortho- oder meta-verknüpften Strukturen.

Weiterhin ist es bevorzugt, wenn Ar eine Carbazol-, Pyrrol-, Imidazol- oder Benzimidazogruppe enthält, dass diese Gruppe nicht über ein Stickstoffatom, sondern über ein Kohlenstoffatom mit den anderen aromatischen Einheiten von Ar bzw. mit dem Stickstoffatom verknüpft ist.

Für n = 1 enthält die erfindungsgemäße Verbindung eine Heteroarylgruppe der folgenden Formel, welche im Folgenden mit (Het-Ar) abgekürzt wird:

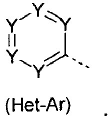

Diese Gruppe ist für n = 1 in der erfindungsgemäßen Verbindung vorhanden und ist für m = 1 an Ar bzw. für m = 0 an den Stickstoff gebunden. In der Gruppe (Het-Ar) stehen mindestens eine Gruppe Y und bevorzugt maximal drei Gruppen Y für N, und die anderen Gruppen Y stehen für CR¹.

Bevorzugte Ausführungsformen sind die Gruppen der folgenden Formeln (Het-Ar-1) bis (Het-Ar-10), 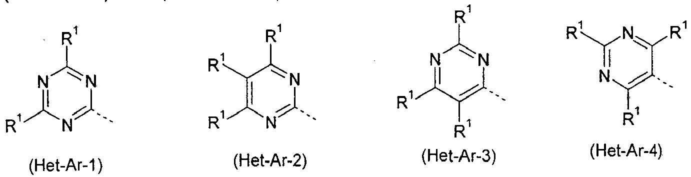 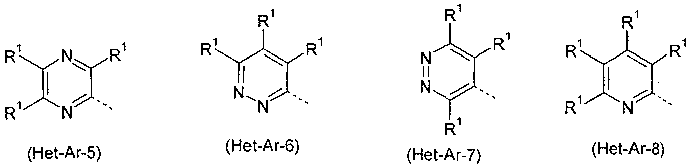 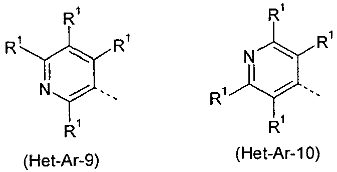 wobei die gestrichelte Bindung die Bindung an Ar bzw. für m = 0 die Bindung an den Stickstoff darstellt und die verwendeten Symbole die oben genannten Bedeutungen aufweisen.

Besonders bevorzugt sind die Gruppen der folgenden Formeln (Het-Ar-1a) bis (Het-Ar-10b), 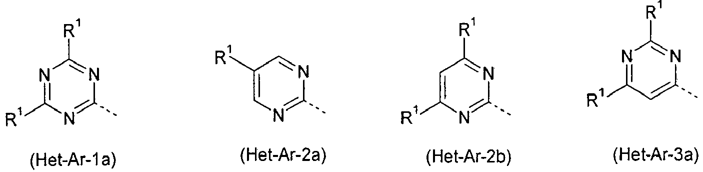 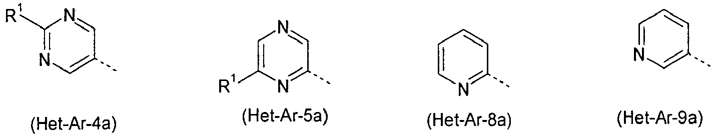 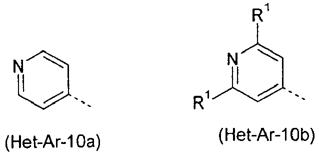 wobei die gestrichelte Bindung die Bindung an Ar bzw. für m = 0 die Bindung an den Stickstoff darstellt und die verwendeten Symbole die oben genannten Bedeutungen aufweisen.

Wenn (Het-Ar) für eine Gruppe (Het-Ar-1) bzw. (Het-Ar-1a) steht, dann stehen die beiden Substituenten R¹ in dieser Gruppe bevorzugt für ein aromatisches oder heteroaromatisches Ringsystem mit 5 bis 24 aromatischen Ringatomen, welches durch einen oder mehrere Reste R² substituiert sein kann, insbesondere für Phenyl, ortho-, meta- oder para-Biphenyl, ortho-, meta-, para- oder verzweigtes Terphenyl, ortho-, meta-, para- oder verzweigtes Quaterphenyl, 1-, 2-, 3- oder 4-Fluoren, 1-, 2-, 3- oder 4-Spirobifluoren, 1-, 2-, 3- oder 4-Dibenzofuran oder 1-, 2-, 3- oder 4-Carbazol.

Wenn (Het-Ar) für eine Gruppe (Het-Ar-2) bis (Het-Ar-10) bzw. (Het-Ar-2a) bis (Het-Ar-10a) steht, dann steht R¹ in diesen Gruppen bevorzugt gleich oder verschieden bei jedem Auftreten für H, D oder ein aromatisches oder heteroaromatisches Ringsystem mit 5 bis 24 aromatischen Ringatomen, welches durch einen oder mehrere Reste R² substituiert sein kann, insbesondere für H oder Phenyl, ortho-, meta- oder para-Biphenyl, ortho-, meta-, para- oder verzweigtes Terphenyl oder ortho-, meta-, para- oder verzweigtes Quaterphenyl.

Die oben genannten bevorzugten Ausführungsformen können beliebig miteinander kombiniert werden. In einer besonders bevorzugten Ausführungsform der Erfindung treten die oben genannten Bevorzugungen gleichzeitig auf.

Wenn die Verbindungen der Formel (1) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Verbindung keine kondensierten Aryl- bzw. Heteroarylgruppen enthält, in denen mehr als zwei Sechsringe direkt aneinander ankondensiert sind. Insbesondere ist es bevorzugt, wenn die Reste R, R¹, R² und Ar keine kondensierte Aryl- bzw. Heteroarylgruppe enthalten, in der zwei oder mehr Sechsringe direkt aneinander ankondensiert sind, und wenn zwei benachbarte Gruppen X nicht für eine Gruppe der Formel (2) stehen.

Beispiele für bevorzugte Verbindungen gemäß den oben aufgeführten Ausführungsformen sind die in der folgenden Tabelle aufgeführten Verbindungen.

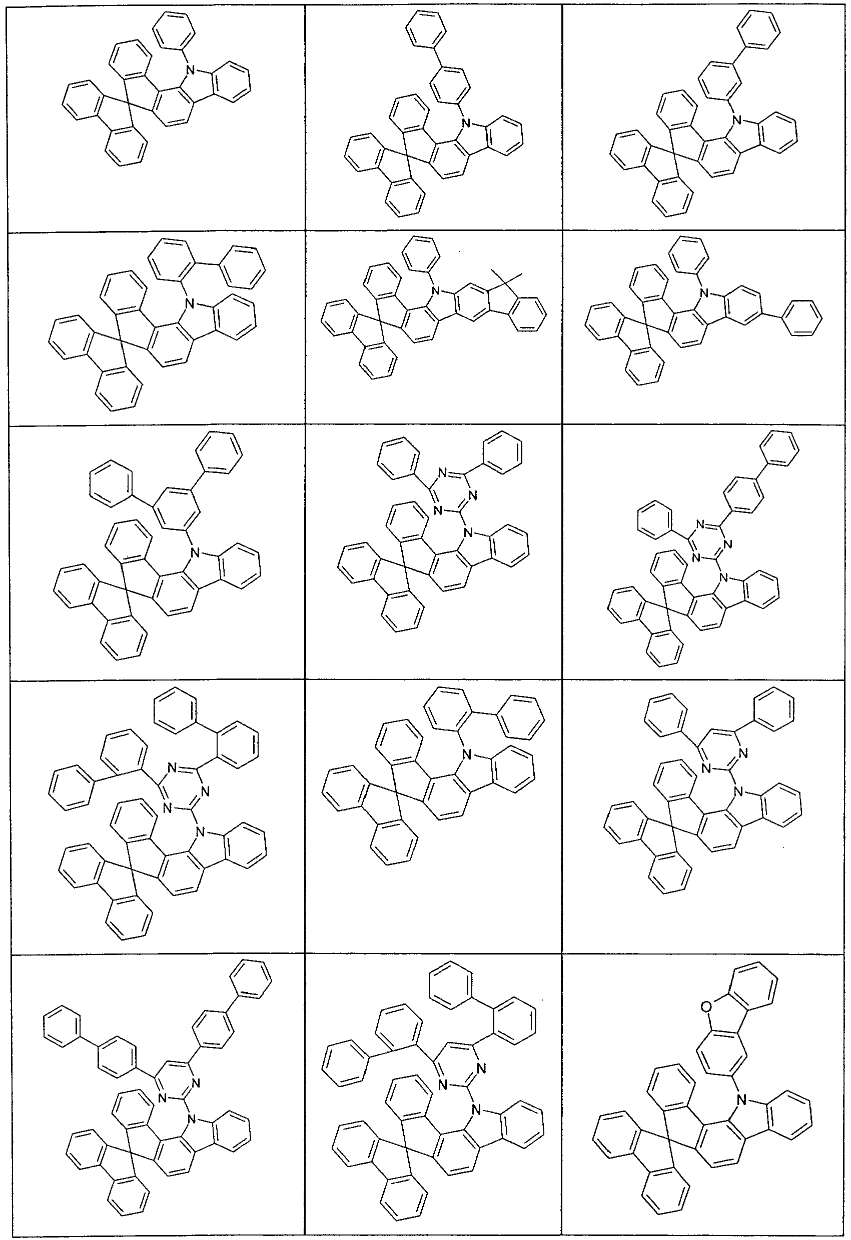

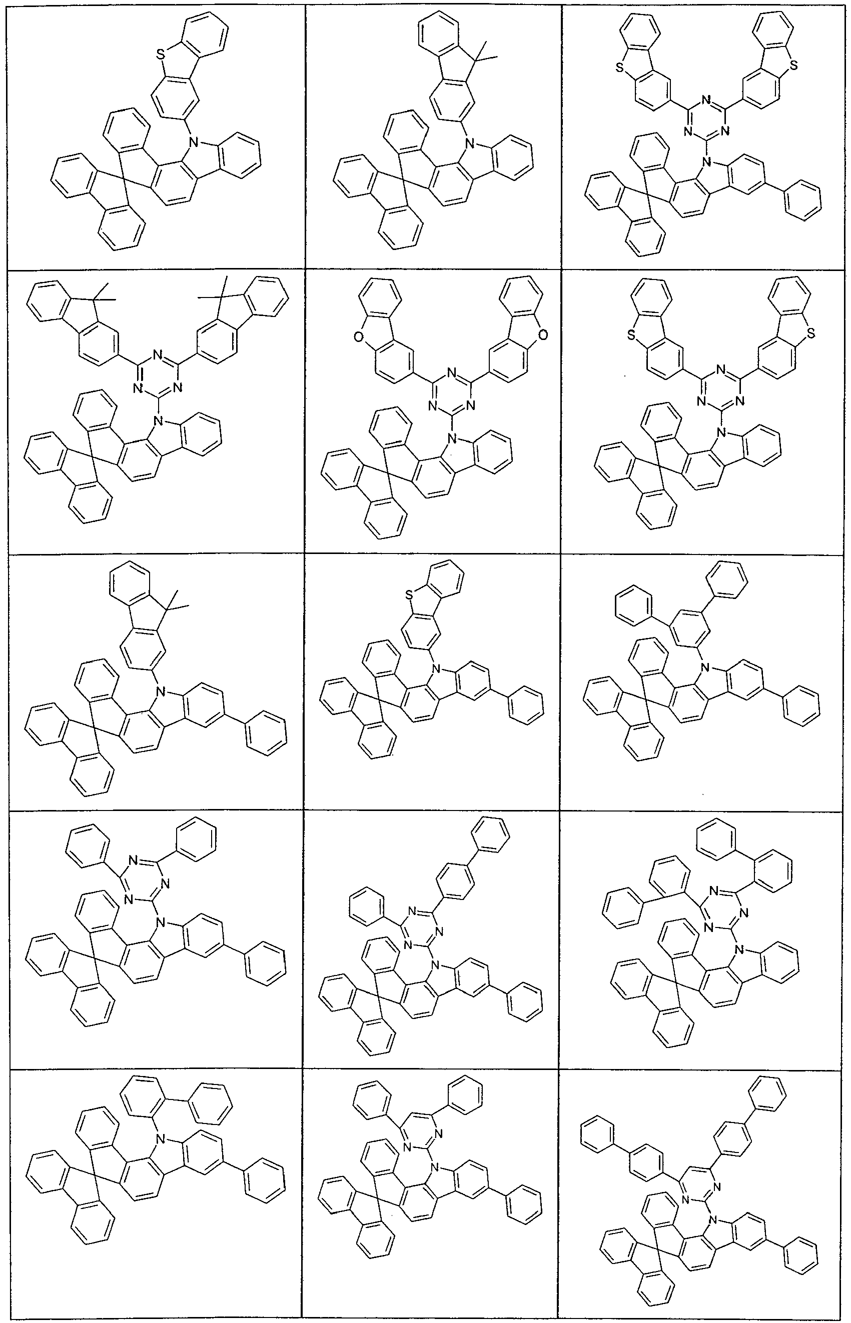

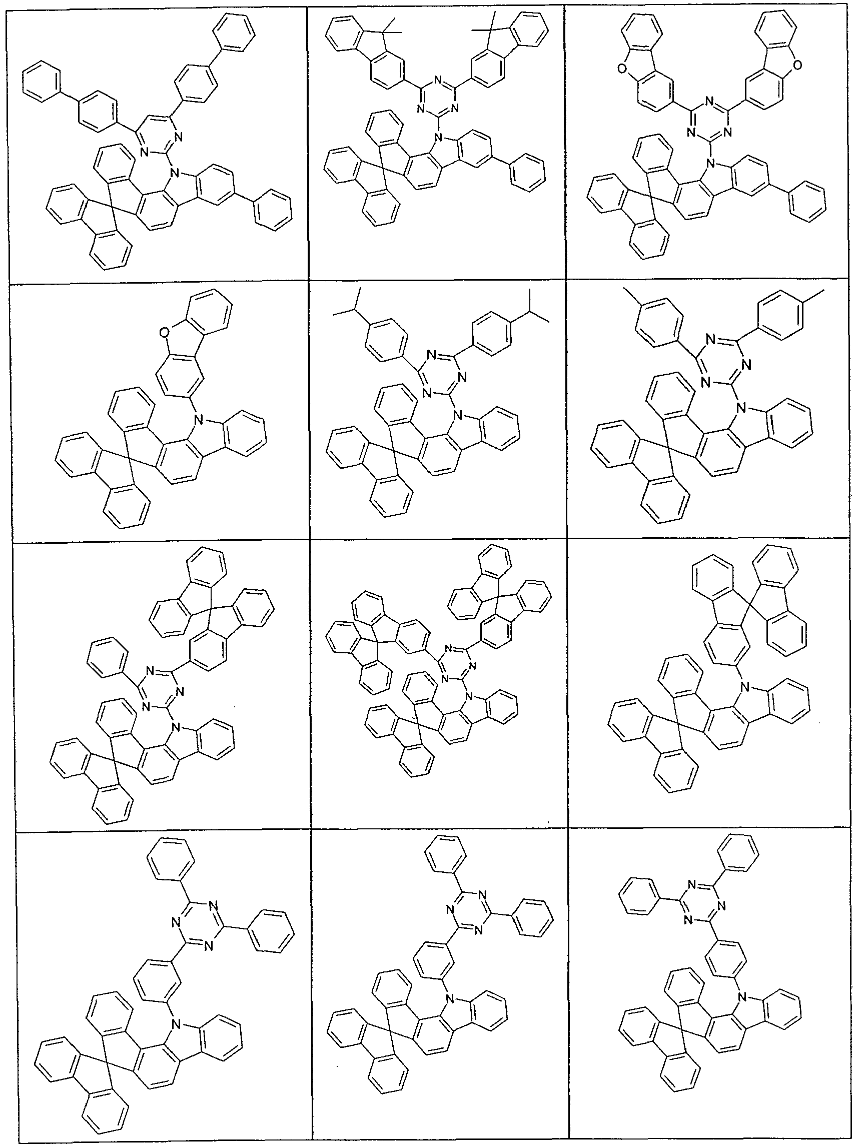

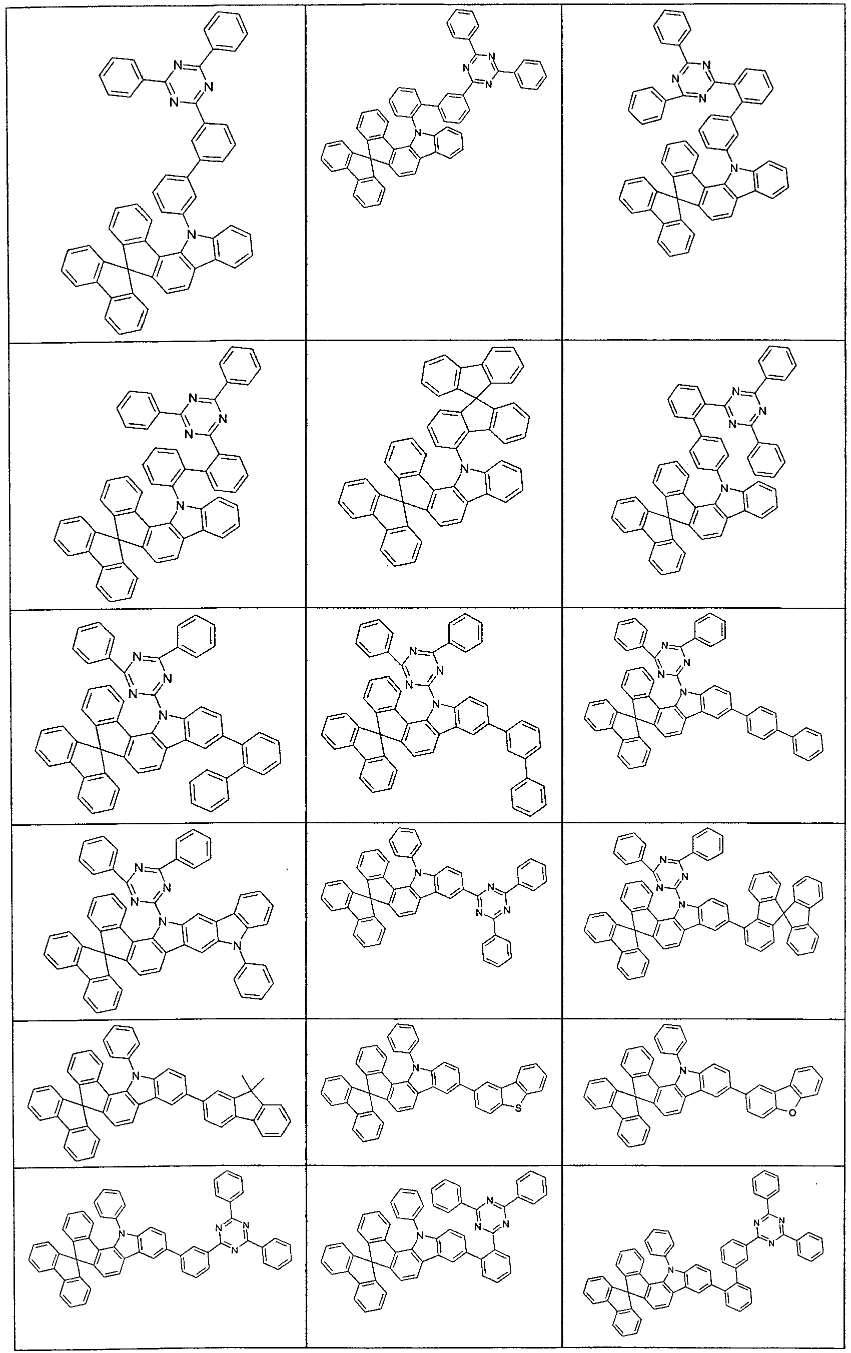

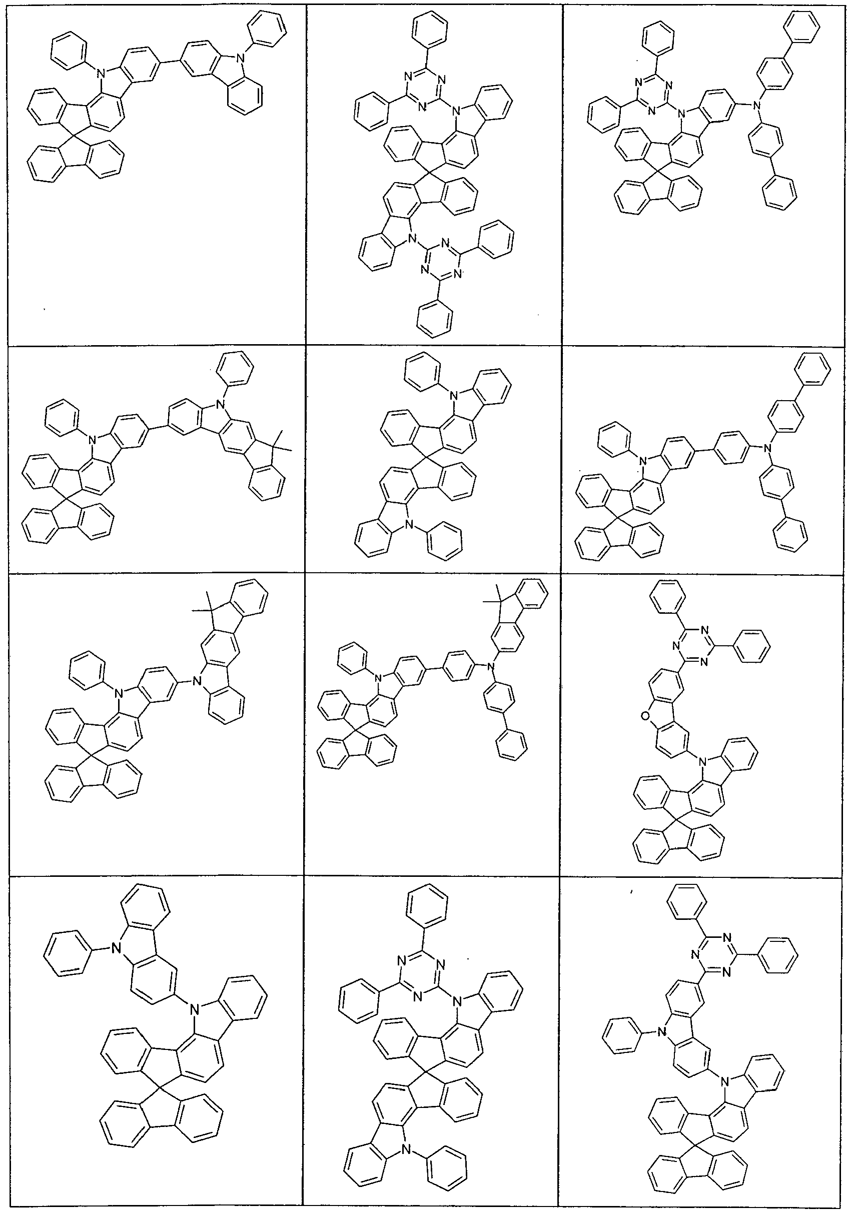

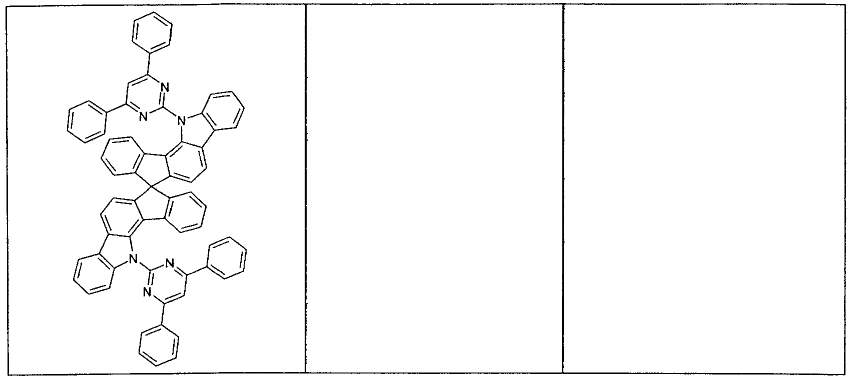

Die Grundstruktur der erfindungsgemäßen Verbindungen kann nach dem in Schema 1 skizzierten Weg dargestellt werden. Die Funktionalisierung kann gemäß Schema 2 erfolgen.

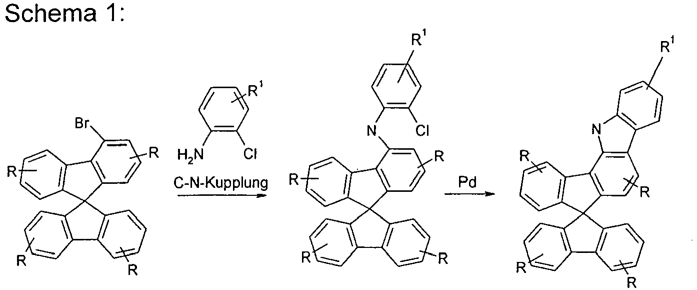

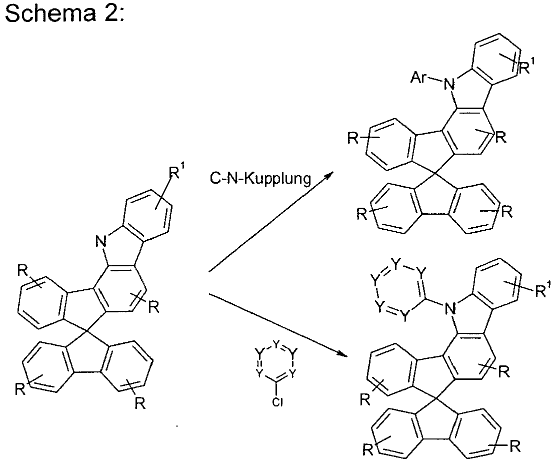

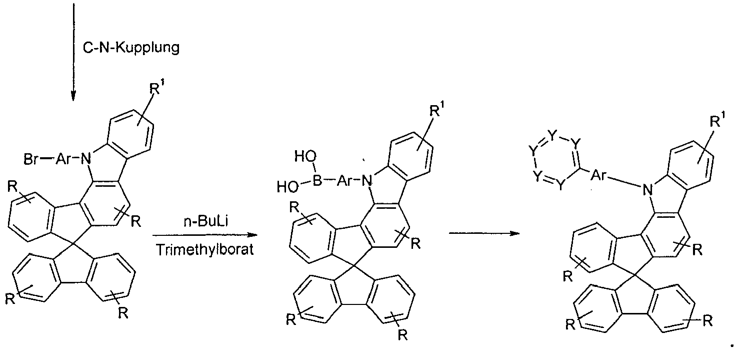

Dabei erfolgt die Synthese üblicherweise ausgehend vom literaturbekannten 4-Bromspirobifluoren (Organic Letters 2009, 11(12), 2607-2610) bzw. einem entsprechend substituierten 4-Bromspirobifluoren. Dieses wird mit einem ortho-Halogenaminobenzol in einer C-N-Kupplungsreaktion, beispielsweise unter Pd- oder Cu-Katalyse, umgesetzt, wobei das Halogen bevorzugt Cl, Br oder I ist. Ganz analog kann beispielsweise ein Naphthalin-, Fluoren-, Dibenzofuran- oder Dibenzothiophenderivat eingesetzt werden, wodurch man zu Verbindungen gelangt, welche Gruppen der Formel (2) oder (3) enthalten. Der Ringschluss zum entsprechenden Carbazolderivat erfolgt durch eine intramolekulare Pd-katalysierte Kupplungsreaktion.

Die Synthese von Verbindungen der Formel (1A) kann ganz analog ausgehend vom literaturbekannten 4,4'-Dibromspirobifluoren erfolgen.

Verbindungen der Formel (1) mit n = 0 und m =1 erhält man durch eine Kupplungsreaktion, beispielsweise Hartwig-Buchwald-Kupplung oder Ullmann-Kupplung, mit einem entsprechend funktionalisierten Aromaten oder Heteroaromaten, wobei die reaktive Gruppe bevorzugt Cl, Br oder I ist.

Verbindungen der Formel (1) mit n = 1 und m = 0 erhält man durch eine nukleophile aromatische Substitutionsreaktion oder durch eine Pd-katalysierte Kupplungsreaktion mit einer Gruppe (Het-Ar), die mit einer entsprechenden Abgangsgruppe, insbesondere Cl oder Br, substituiert ist.

Verbindungen der Formel (1) mit n = 1 und m = 1 erhält man durch eine Kupplungsreaktion, beispielsweise eine Hartwig-Buchwald-Kupplung oder Ullmann-Kupplung, mit einem difunktionalisierten Aromaten oder Heteroaromaten, wobei die reaktiven Gruppen bevorzugt eine Brom- und eine lodgruppe sind, gefolgt von einer Pd-katalysierten Kupplungsreaktion, beispielsweise einer Suzuki-, Negishi-, Yamamoto-, Grignard-Cross- oder Stille-Kupplung, gegebenenfalls nach Umwandlung einer Halogengruppe in ein Boronsäurederivat.

Ein weiterer Gegenstand der vorliegenden Erfindung ist ein Verfahren zur Herstellung einer Verbindung gemäß Formel (1) bzw. (1A), umfassend die Reaktionsschritte:
a) Synthese der Grundgerüsts der Verbindung (1) bzw. (1A), die noch keine Gruppe (Het-Ar) und/oder Ar enthält; und
b) Umsetzung der Grundgerüsts aus a) in einer C-C-Kupplung, wie Suzuki-, Negishi-, Yamamoto-, Grignard-Cross- oder Stille-Kupplung, etc., oder C-N-Kupplung, wie Buchwald- oder Ullmann-Kupplung.

Die oben beschriebenen erfindungsgemäßen Verbindungen, insbesondere Verbindungen, welche mit reaktiven Abgangsgruppen, wie Brom, lod, Chlor, Boronsäure oder Boronsäureester, oder mit reaktiven, polymerisierbaren Gruppen, wie Olefinen, Styrolen, Acrylaten oder Oxetanen, substituiert sind, können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten erfindungsgemäßen Verbindungen, wobei an einer oder mehreren Positionen statt Substituenten eine oder mehrere Bindungen der erfindungsgemäßen Verbindung zum Polymer, Oligomer oder Dendrimer vorhanden sind. Je nach Verknüpfung der erfindungsgemäßen Verbindung bildet diese eine Seitenkette des Oligomers oder Polymers oder ist in der Hauptkette verknüpft oder bildet den Kern eines Dendrimers. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Homopolymere oder Copolymere, wobei die Einheiten gemäß Formel (1) bzw. die oben ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/22026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten. Außerdem können die Polymere entweder einpolymerisiert oder als Blend eingemischt TriplettEmitter enthalten. Gerade die Kombination von den erfindungsgemäßen Oligomere, Polymeren oder Dendrimeren mit Triplett-Emittern führt zu besonders guten Ergebnissen.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich bei der erfindungsgemäßen organischen Elektrolumineszenzvorrichtung auch um eine Tandem-OLED handeln, insbesondere auch für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen als Matrixmaterial für phosphoreszierende oder fluoreszierende Emitter, insbesondere für phosphoreszierende Emitter, und/oder in einer elektronenblockierenden bzw. exzitonenblockierenden Schicht und/oder in einer Lochtransportschicht und/oder in einer Lochblockierschicht und/oder in einer Lochblockier- oder Elektronentransportschicht, je nach genauer Substitution.

In einer bevorzugten Ausführungsform der Erfindung wird die erfindungsgemäße Verbindung als Matrixmaterial für eine phosphoreszierende Verbindung in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Besonders geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, oder Triphenylenderivate, z. B. gemäß WO 2012/048781. Ebenso kann ein weiterer phosphoreszierender Emitter, weicher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 2005/033244, WO 2005/019373, US 2005/0258742, WO 2010/086089, WO 2011/157339, WO 2012/007086, WO 2012/163471, WO 2013/000531 und WO 2013/020631 entnommen werden. Weiterhin eignen sich beispielsweise die in den nicht offen gelegten Anmeldungen EP 12005187.5 und EP 12005715.3 offenbarten Metallkomplexe. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Die erfindungsgemäßen Verbindungen sind insbesondere auch geeignet als Matrixmaterialien für phosphoreszierende Emitter in organischen Elektrolumineszenzvorrichtungen, wie sie z. B. in US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft. Dabei wurde überraschend gefunden, dass die erfindungsgemäßen Verbindungen, wenn sie als Matrixmaterialien für das rote und/oder grüne Pixel eingesetzt werden, zusammen mit der aufgedampften blauen Emissionsschicht zu weiterhin sehr guter Emission führen.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In einer weiteren Ausführungsform der Erfindung wird die erfindungsgemäße Verbindung in einer Lochtransportschicht bzw. in einer Elektronenblockierschicht bzw. Exzitonenblockierschicht eingesetzt.

In nochmals einer weiteren bevorzugten Ausführungsform der Erfindung wird die erfindungsgemäße Verbindung als Elektronentransportmaterial in einer Elektronentransport- oder Elektroneninjektionsschicht eingesetzt. Dabei kann die emittierende Schicht fluoreszierend oder phosphoreszierend sein. Wenn die Verbindung als Elektronentransportmaterial eingesetzt wird, kann es bevorzugt sein, wenn sie dotiert ist, beispielsweise mit Alkalimetallkomplexen, wie z. B. LiQ (Lithiumhydroxychinolinat).

In nochmals einer weiteren bevorzugten Ausführungsform der Erfindung wird die erfindungsgemäße Verbindung in einer Lochblockierschicht eingesetzt. Unter einer Lochblockierschicht wird eine Schicht verstanden, die auf Kathodenseite direkt an eine emittierende Schicht angrenzt.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen, eingesetzt als Matrixmaterial für fluoreszierende oder phosphoreszierende Emitter, führen zu langen Lebensdauern. Dies gilt insbesondere, wenn die Verbindungen als Matrixmaterial für einen phosphoreszierenden Emitter eingesetzt werden.
2. Die erfindungsgemäßen Verbindungen führen zu sehr niedrigen Betriebsspannungen. Dies gilt insbesondere, wenn die Verbindungen als Matrixmaterial für einen phosphoreszierenden Emitter eingesetzt werden.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegeben Nummern sind die entsprechenden CAS-Nummern.

### Synthesebeispiele

### Beispiel 1a: Synthese von (2-Chlorphenyl)-4-spiro-9,9'-bifluorenyl-amin (Zwischenprodukt)

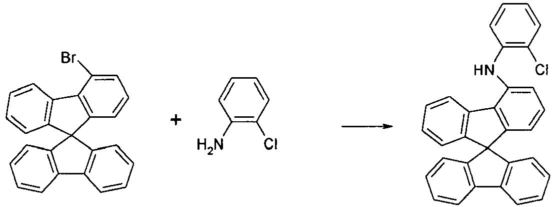

54 g (137 mmol) 4-Bromspiro-9,9'-bifluoren (1161009-88-6), 17.9 g (140 mmol) 2-Chloranilin, 68.2 g (710 mmol) Natrium-tert-butylat, 613 mg (3 mmol) Palladium(II)acetat und 3.03 g (5 mmol) dppf werden in 1.3 L Toluol gelöst und 5 h unter Rückfluss gerührt. Das Reaktionsgemisch wird auf Raumtemperatur abgekühlt, mit Toluol erweitert und über Celite filtriert. Das Filtrat wird im Vakuum eingeengt und der Rückstand aus Toluol/ Heptan kristallisiert. Das Produkt wird als farbloser Feststoff isoliert. Ausbeute: 52.2 g (118 mmol) 86% d.Th.

Analoa werden folgende Verbindungen als Zwischenprodukte hergestellt:

| | Edukt 1 | Edukt 2 | Produkt | Ausbeute |
|---|---|---|---|---|
| 1b | 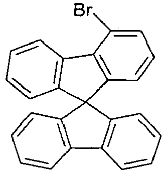 | 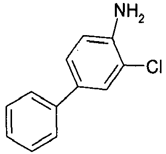 | 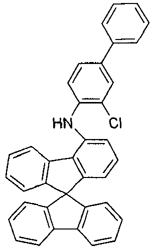 | 83% |
| | | 7285-66-7 | | |
| 1c | 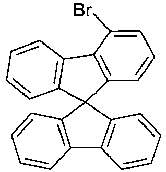 | 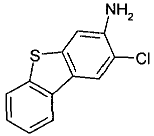 | 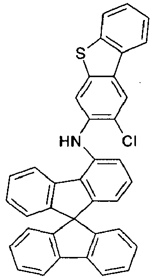 | 78% |
| | | 858426-71-8 | | |
| 1d | 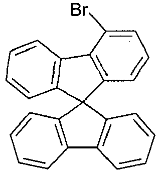 | 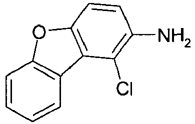 | 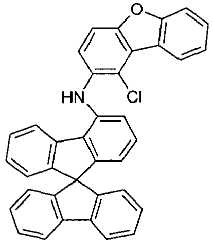 | 67% |
| | | 133617-97-7 | | |
| 1e | 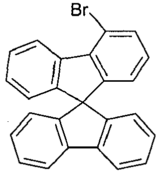 | 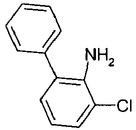 | 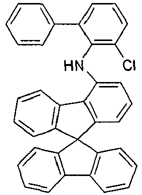 | 53% |
| | | 76838-82-9 | | |
| 1f | 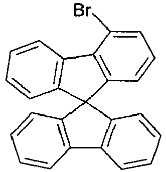 | 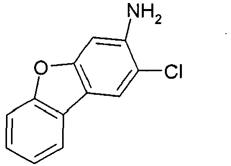 | 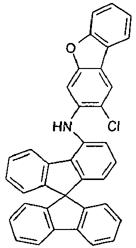 | 73% |
| | | 5833-88-5 | | |
| 1g | 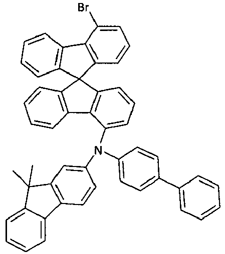 | 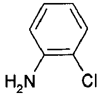 | 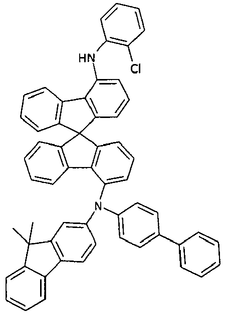 | 68% |
| | siehe Beispiel 11 | 95-51-2 | | |
| 1h | 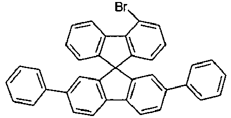 | 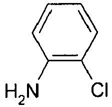 | 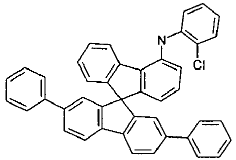 | 70% |
| | | 95-51-2 | | |
| 1i | 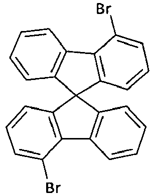 | 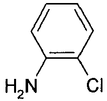 | 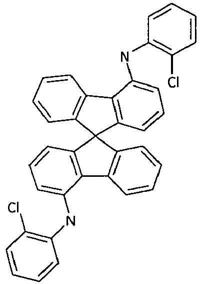 | 72% |
| | | 95-51-2 | | |
| 1j | 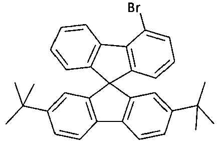 | 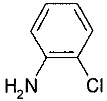 | 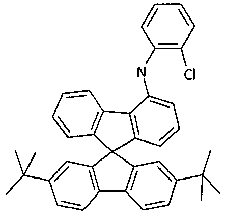 | 71% |
| | | 95-51-2 | | |
| 1k | 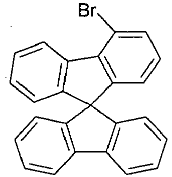 | 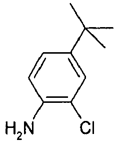 | 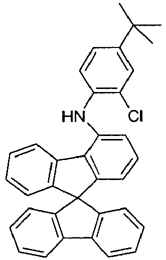 | 69% |
| | | 42265-67-8 | | |
| 1l | 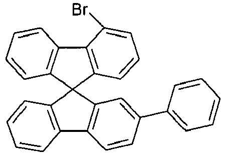 | 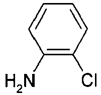 | 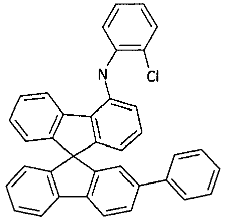 | 73% |
| | | 95-51-2 | | |
| 1m | 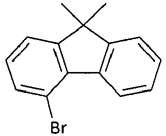 | 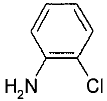 | 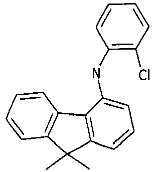 | 78% |
| | 942615-32-9 | 95-51-2 | | |
| 1n | 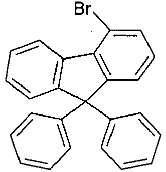 | 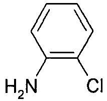 | 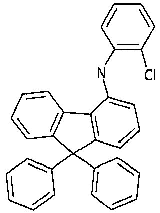 | 83% |
| | 713125-22-5 | 95-51-2 | | |

### Beispiel 2a: Synthese von Spiro[9H-fluoren-9,7'(1'H)-indeno[1,2-a]carbazol] (Zwischenprodukt)

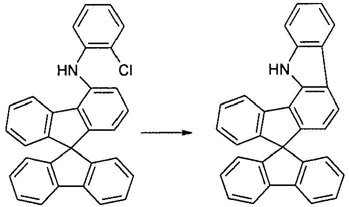

45 g (102 mmol) (2-Chlorphenyl)-4-spiro-9,9'-bifluorenyl-amin, 56 g (409 mmol) Kaliumcarbonat, 4.5 g (12 mmol) Tricyclohexylphosphonium-tetrafluoroborat und 1.38 g (6 mmol) Palladium(II)acetat werden in 500 mL Dimethylacetamid suspendiert und 6 h unter Rückfluss gerührt. Nach Erkalten wird die Reaktionmischung mit 300 ml Wasser und 600 mL Dichlormethan erweitert. Man rührt 30 min. nach, trennt die organische Phase ab, filtriert diese über ein kurzes Celite-Bett und entfernt dann das Lösungsmittel im Vakuum. Das Rohprodukt wird mit Toluol heiß extrahiert und aus Toluol umkristallisiert. Das Produkt wird als beigefarbener Feststoff isoliert (32.5 g, 80 mmol, entsprechend 78 % d.Th.).

werden folgende Verbindungen als Zwischenprodukte hergestellt:

| | Edukt | Produkt | Ausbeute |
|---|---|---|---|
| 2b | 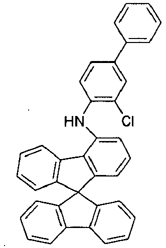 | 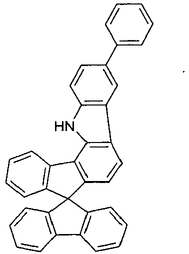 | 72% |
| 2c | 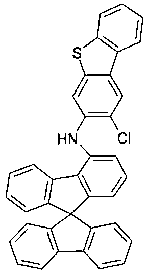 | 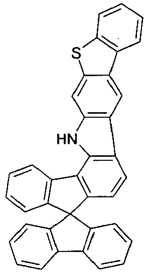 | 73% |
| 2d | 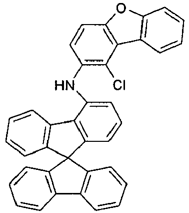 | 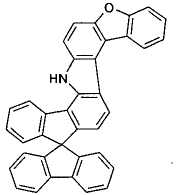 | 67% |
| 2e | 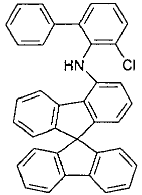 | 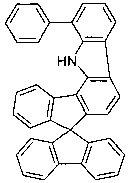 | 56% |
| 2f | 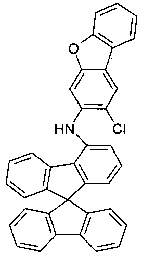 | 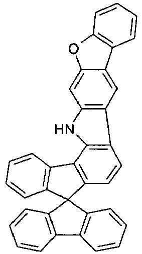 | 71% |
| 2g | 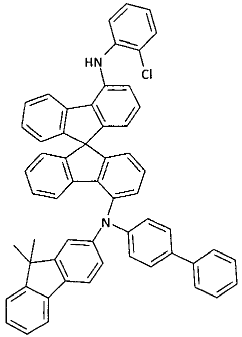 | 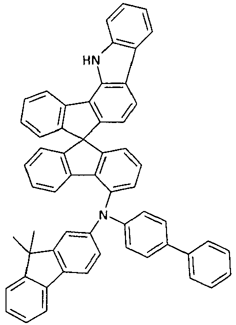 | 67% |
| 2h | 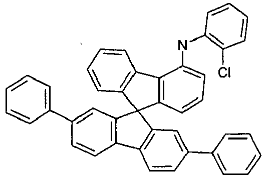 | 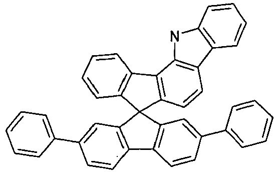 | 74% |
| 2i | 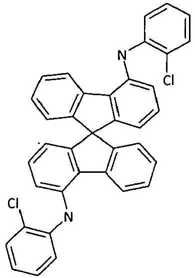 | 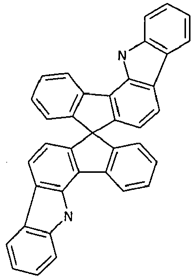 | 68% |
| 2j | 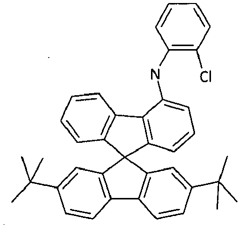 | 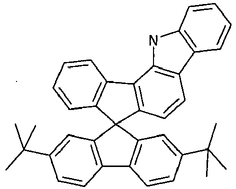 | 72% |
| 2k | 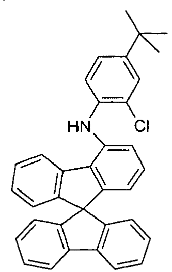 | 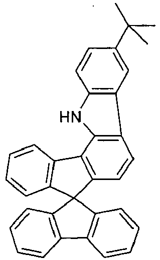 | 75% |
| 2l | 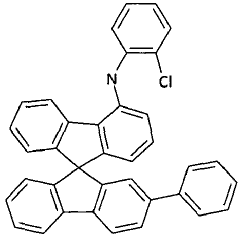 | 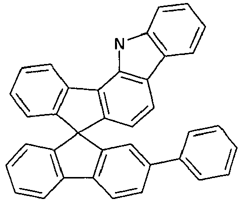 | 73% |
| 2m | 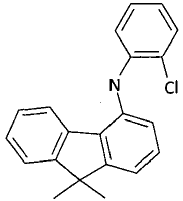 | 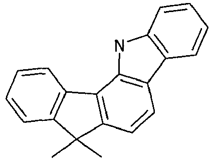 | 83% |
| 2n | 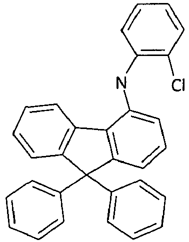 | 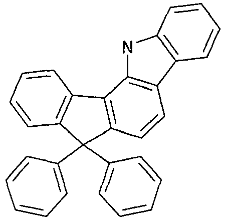 | 85% |

### Beispiel 3a: Synthese von Spiro[9H-fluoren-9,7'(12'H)-indeno[1,2-a]carbazol]-12'-[2-(4,6-diphenyl-1,3,5-triazin-2-yl)]

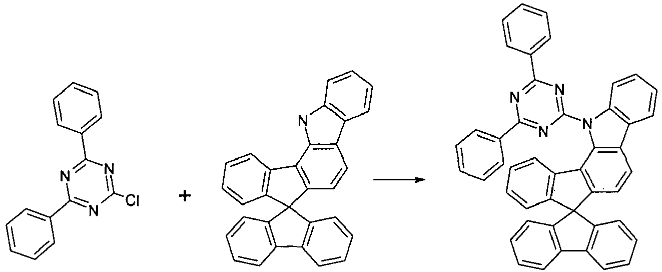

4.2 g NaH 60%ig in Mineralöl (0,106 mol) werden in 300 mL Dimethylformamid unter Schutzatmosphäre gelöst. 43 g (0.106 mol) Spiro[9H-fluoren-9,7'(1'H)-indeno[1,2-a]carbazol] werden in 250 mL DMF gelöst und zu der Reaktionsmischung zugetropft. Nach 1 h bei Raumtemperatur wird eine Lösung von 2-Chlor-4,6-Diphenyl-[1,3,5]triazin (34.5 g, 0.122 mol) in 200 mL THF zugetropft. Das Reaktionsgemischt wird dann 12 h bei Raumtemperatur gerührt. Nach dieser Zeit wird das Reaktionsgemisch auf Eis gegossen. Der dabei ausgefallene Feststoff wird nach Erwärmen auf Raumtemperatur filtriert und mit Ethanol und Heptan gewaschen. Der Rückstand wird mit Toluol heiß extrahiert, aus Toluol / n-Heptan umkristallisiert und abschließend im Hochvakuum sublimiert, Reinheit beträgt 99,9%. Die Ausbeute beträgt 28.4 g (44.5 mmol; 42 %).

Analog werden folgende Verbindungen hergestellt:

| | Edukt 1 | Edukt 2 | Produkt | Ausbeute |
|---|---|---|---|---|
| 3b | 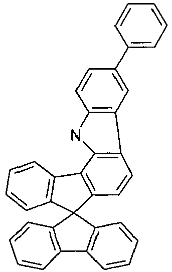 | 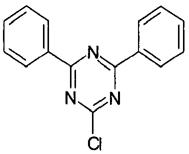 | 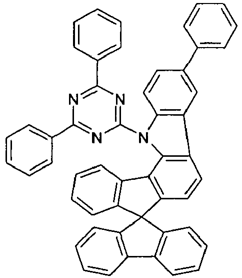 | 41% |
| | | 3842-55-5 | | |
| 3c | 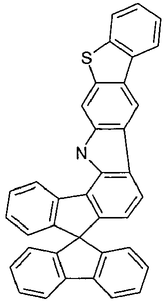 | 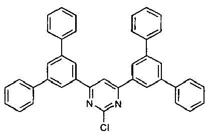 | 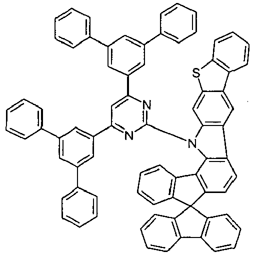 | 33% |
| | | 1384480-21-0 | | |
| 3d | 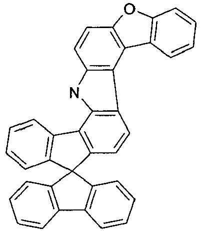 | 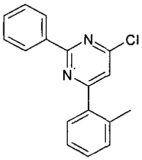 | 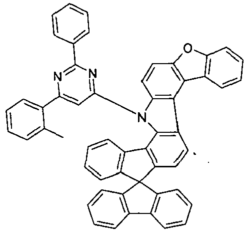 | 42% |
| | | 70484-36-5 | | |
| 3e | 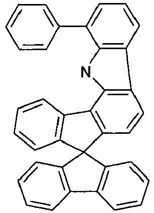 | 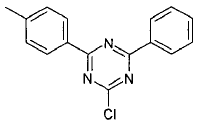 | 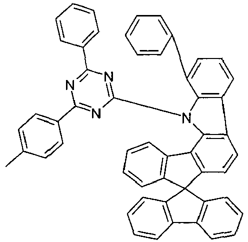 | 27% |
| | | 92853-85-5 | | |
| 3f | 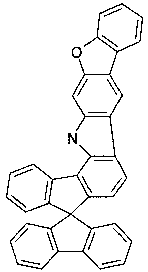 | 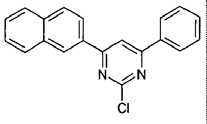 | 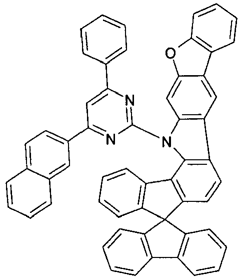 | 40% |
| | | 1260393-65-4 | | |
| 3g | 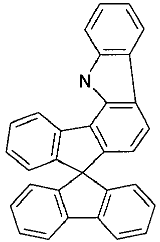 | 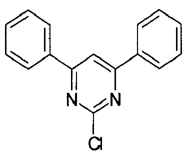 | 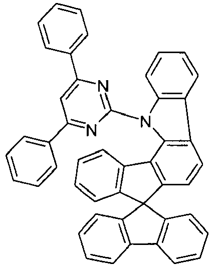 | 45% |
| | | 2915-16-4 | | |
| 3h | 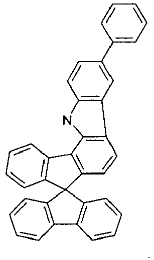 | 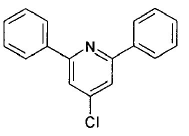 | 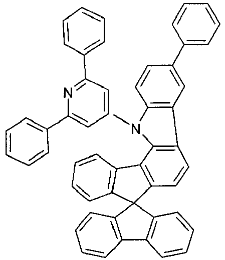 | 38% |
| | | 133785-60-1 | | |
| 3i | 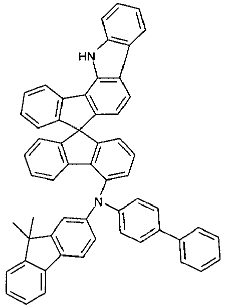 | 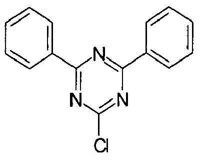 | 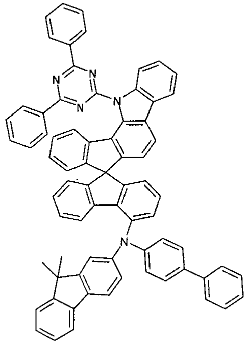 | 34% |
| | | 3842-55-5 | | |
| 3j | 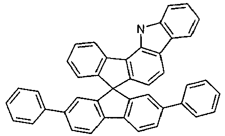 | 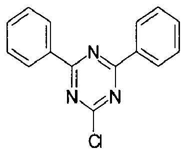 | 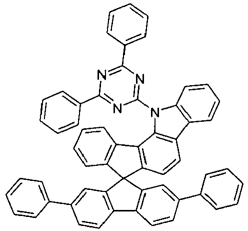 | 38% |
| | | 3842-55-5 | | |
| 3k | 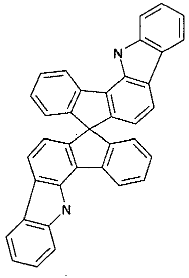 | 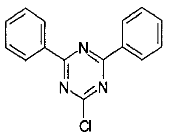 | 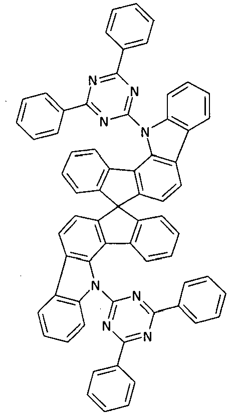 | 32% |
| | | 3842-55-5 | | |
| 3l | 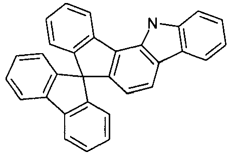 | 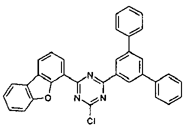 | 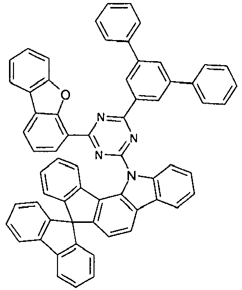 | 34% |
| 3m | 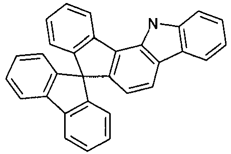 | 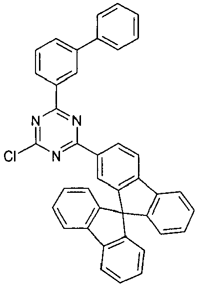 | 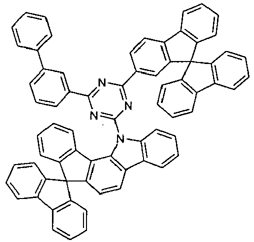 | 41% |
| 3n | 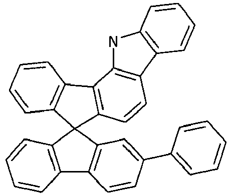 | 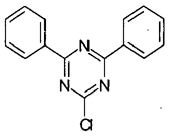 | 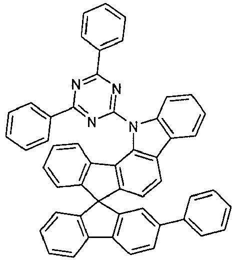 | 42% |
| | | 3842-55-5 | | |
| 3o | 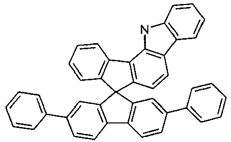 | 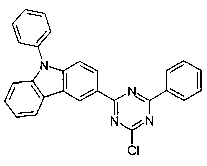 | 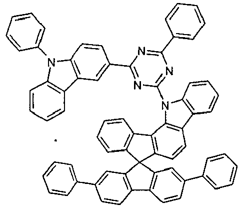 | 39% |
| 3p | 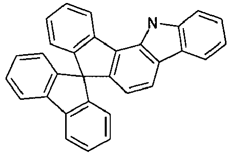 | 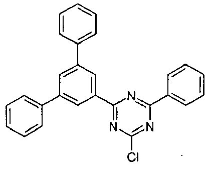 | 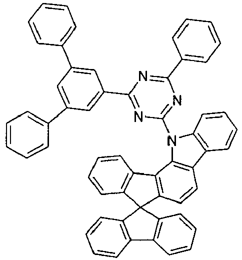 | 41% |
| 3q | 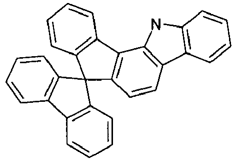 | 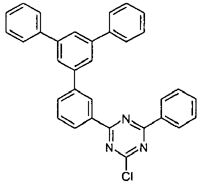 | 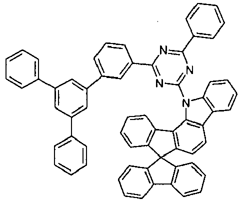 | 36% |
| 3r Vgl. | 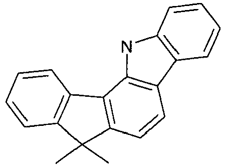 | 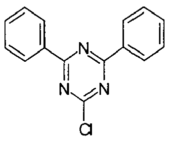 | 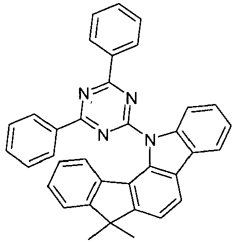 | 40% |
| | | 3842-55-5 | | |
| 3s Vgl. | 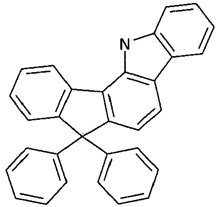 | 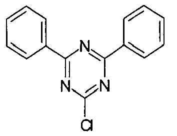 | 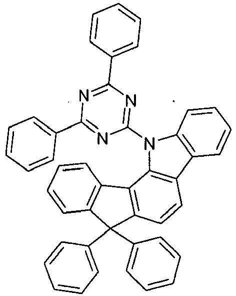 | 46% |
| | | 3842-55-5 | | |

### Beispiel 4a: Synthese von Spiro[9H-fluoren-9,7'(12'H)-indeno[1,2-a]carbazol]-12'-[4-bromphenyl]

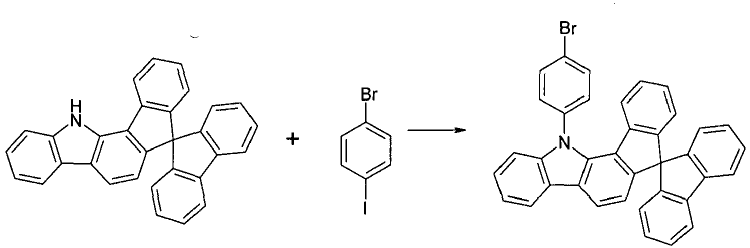

32.8 g (81 mmol) Spiro[9H-fluoren-9,7'(1'H)-indeno[1,2-a]carbazol], 115 g (406 mmol) 1-Brom-4-iodbenzol, 22.4 g (162 mmol) Kaliumcarbonat, 1.84 g (8.1 mmol) 1,3-Di(2-pyridyl)-1,3-propandion, 1.55 g (8.1 mmol) Kupferiodid und 1000 mL DMF werden 30 h unter Rückfluss erhitzt. Anschließend wird die Reaktionsmischung am Rotationsverdampfer bis zur Trockene eingeengt. Der Rückstand wird in THF gelöst und über ein kurzes Kieselgel-Bett filtriert, dann das Lösungsmittel im Vakuum entfernt. Anschließend wird der Feststoff aus Heptan/THF umkristallisiert und über Aluminiumoxid mit Heptan/Toluol heiß extrahiert. Der beim Erkalten ausgefallene Feststoff wird filtriert und getrocknet. Ausbeute:37g (66 mmol), 81 %.

Analog werden folgende Verbindungen hergestellt:

| | Edukt1 | Edukt 2 | Produkt | Ausbeute |
|---|---|---|---|---|
| 4b | 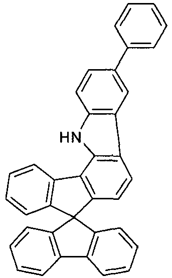 | 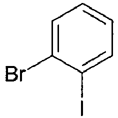 | 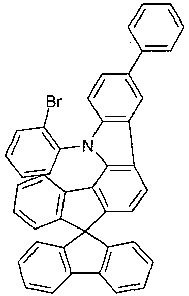 | 47% |
| | | 583-55-1 | | |
| 4c | 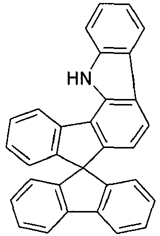 | 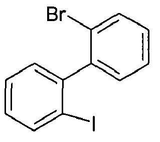 | 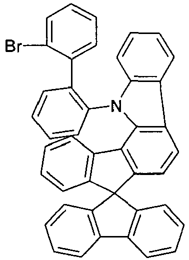 | 27% |
| | | 39655-12-4 | | |
| 4d | 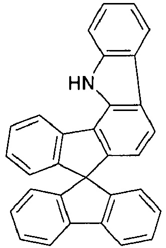 | 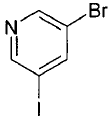 | 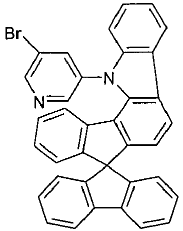 | 76% |
| | | 233770-01-9 | | |
| 4e | 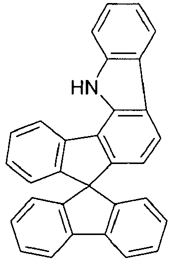 | 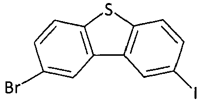 | 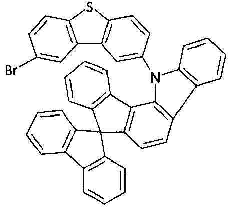 | 65% |
| | | 1206544-88-8 | | |
| 4f | 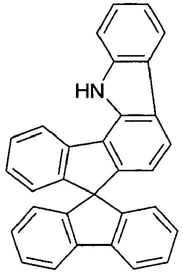 | 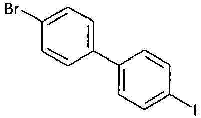 | 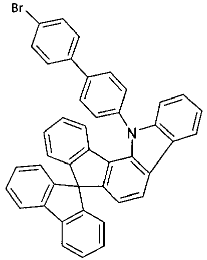 | 73% |
| | | 105946-82-5 | | |
| 4g | 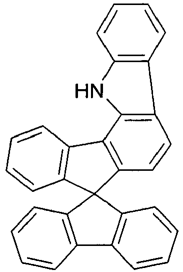 | 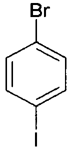 | 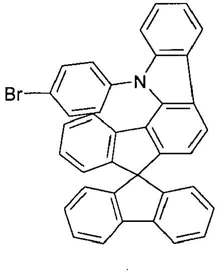 | 76% |
| | | 589-87-7 | | |
| 4h | 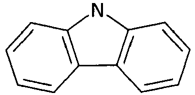 | 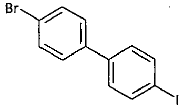 | 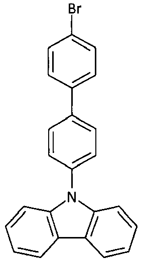 | 81% |
| | 86-74-8 | 105946-82-5 | | |
| 4i | 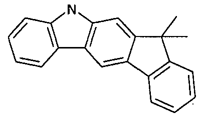 | 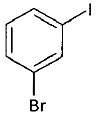 | 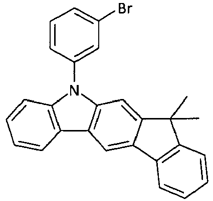 | 77% |
| | 1257220-47-5 | 591-18-4 | Zwischenprodukt | |

### Beispiel 5a: Synthese von Spiro[9H-fluoren-9,7'(12'H)-indeno[1,2-a]-carbazol]-12'-[4-phenylboronsäure] (Zwischenprodukt)

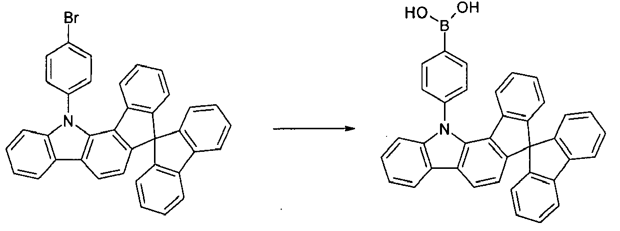

Eine auf -78 °C gekühlte Lösung von 73 g (130 mmol) 1-Brom-9-spirobifluoren in 1500 ml THF wird tropfenweise mit 55 ml (138 mmol) n-Butyllithium (2.5 M in Hexan) versetzt. Die Reaktionsmischung wird 30 min. bei -78 °C gerührt. Man lässt auf Raumtemperatur kommen, kühlt erneut auf -78 °C und versetzt dann schnell mit einer Mischung von 20 ml (176 mmol) Trimethylborat in 50 ml THF. Nach Erwärmen auf -10 °C wird mit 135 ml 2 N Salzsäure hydrolysiert. Die organische Phase wird abgetrennt, mit Wasser gewaschen, über Natriumsulfat getrocknet und zur Trockene eingeengt. Der Rückstand wird in 300 ml n-Heptan aufgenommen, der farblose Feststoff wird abgesaugt, mit n-Heptan gewaschen und im Vakuum getrocknet. Ausbeute: 94,5 g (255 mmol), 99 % d. Th.; Reinheit: 99 % nach HPLC.

Analog werden folgende Verbindungen als Zwischenprodukte hergestellt:

| | Edukt | Produkt | Ausbeute |
|---|---|---|---|
| 5b | 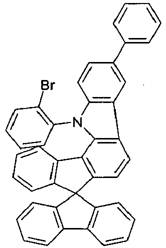 | 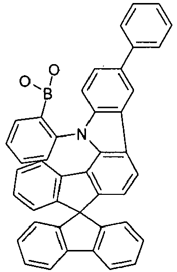 | 88% |
| 5c | 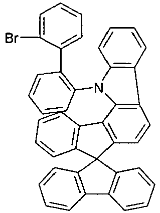 | 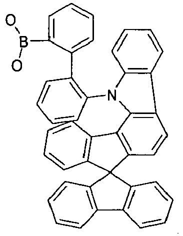 | 91% |
| 5d | 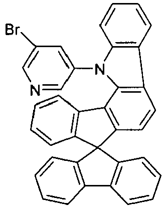 | 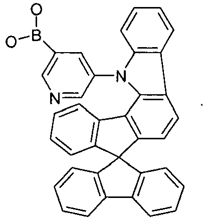 | 95% |
| 5e | 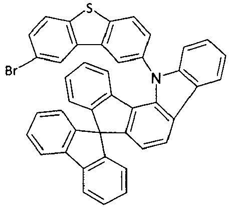 | 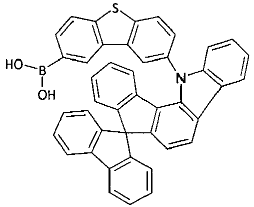 | 87% |
| 5f | 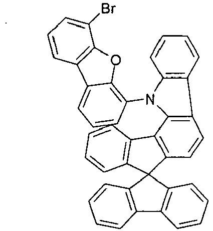 | 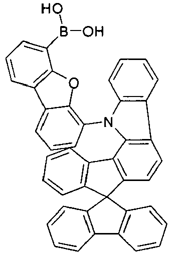 | 73% |
| 5g | 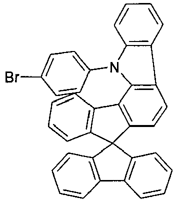 | 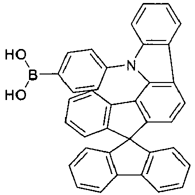 | 78% |
| 5h | 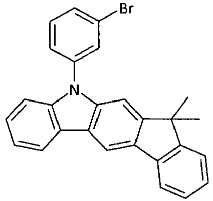 | 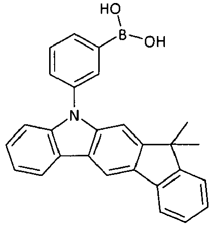 | 77% |

### Beispiel 6a

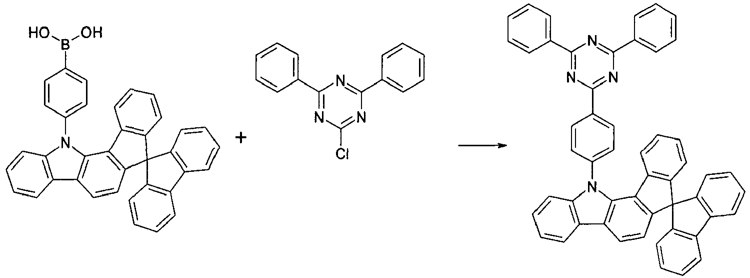

57.8 g (110 mmol) Spiro[9H-fluoren-9,7'(12'H)-indeno[1,2-a]carbazol]-12'-[4-phenylboronsäure], 29.5 g (110.0 mmol) 2-Chlor-4,6-diphenyl-1,3,5-triazin und 44.6 g (210.0 mmol) Trikaliumphosphat werden in 500 mL Toluol, 500 mL Dioxan und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol/Heptan umkristallisiert und abschließend im Hochvakuum (p = 5 x 10⁻⁵ mbar, T = 377 °C) sublimiert. Die Ausbeute beträgt 29.2 g (41 mmol), entsprechend 37 % der Theorie.

Analog werden folgende Verbindungen hergestellt:

| | Edukt1 | Edukt 2 | Produkt | Ausbeute |
|---|---|---|---|---|
| 6b | 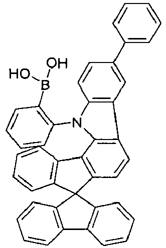 | 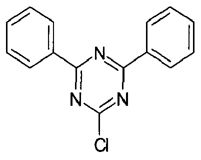 | 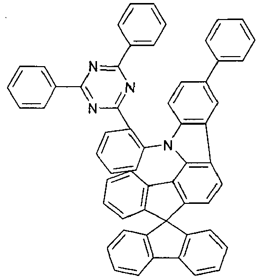 | 22% |
| | | 3842-55-5 | | |
| 6c | 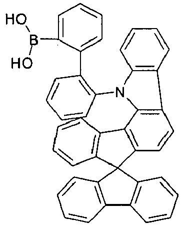 | 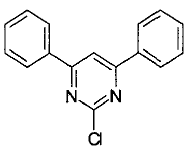 | 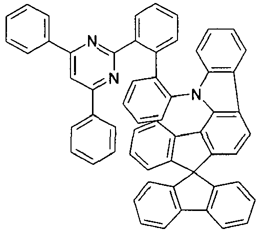 | 27% |
| | | 2915-16-4 | | |
| 6d | 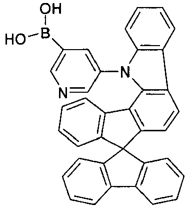 | 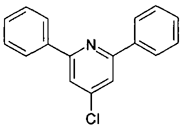 | 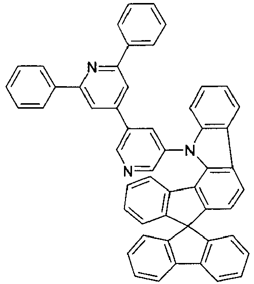 | 35% |
| | | 133785-60-1 | | |
| 6e | 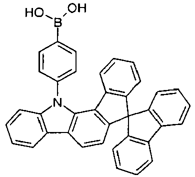 | 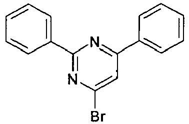 | 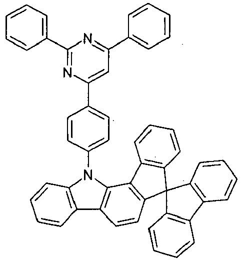 | 32% |
| | | 40734-4-5 | | |
| 6f | 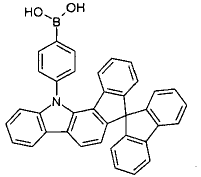 | 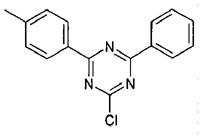 | 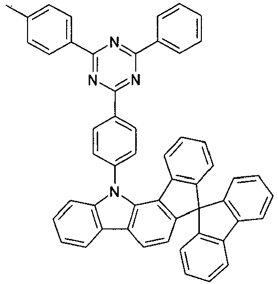 | 33% |
| | | 92853-85-5 | | |
| 6g | 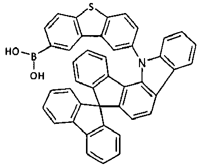 | 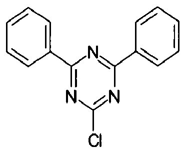 | 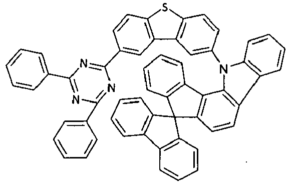 | 34% |
| | | 3842-55-5 | | |
| 6h | 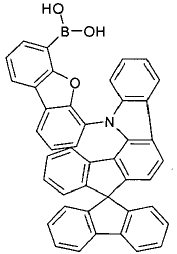 | 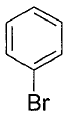 | 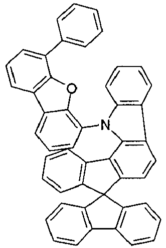 | 37% |
| | | 108-86-1 | | |
| 6i | 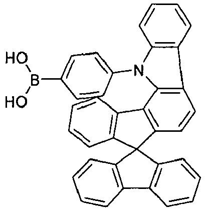 | 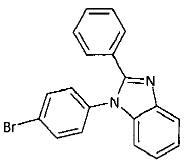 | 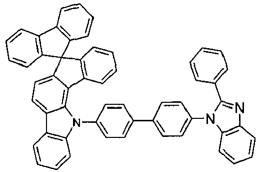 | 34% |
| | | 760212-58-6 | | |
| 6j | 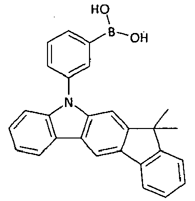 | 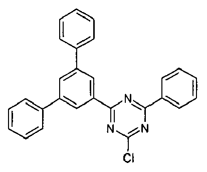 | 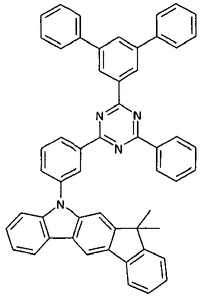 | 36% |
| | | | Vergleichsbeispiel | |

### Beispiel 7a

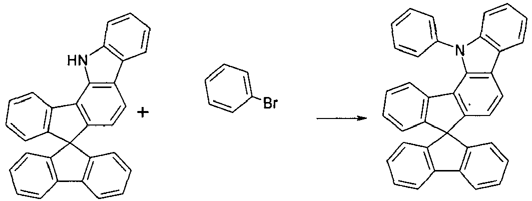

43 g (106 mmol) Spiro[9*H*-fluoren-9,7'(1'*H*)-indeno[1,2-*a*]carbazol], 17.9 g (114 mmol) Brombenzol und 30.5 g NaOtBu werden in 1.5 L p-Xylol suspendiert. Zu dieser Suspension werden 0.5 g (2.11 mmol) Pd(OAc)₂ und 4.2 ml einer 1M Tri-tert-butylphosphin-Lösung in Toluol gegeben. Die Reaktionsmischung wird 16 h unter Rückfluss erhitzt. Nach Erkalten wird die organische Phase abgetrennt, dreimal mit je 200 mL Wasser gewaschen und anschließend zur Trockene eingeengt. Der Rückstand wird mit Toluol heiß extrahiert, aus Toluol umkristallisiert und abschließend im Hochvakuum sublimiert, Reinheit beträgt 99.9% bei einer Ausbeute von 21.4 g (44.5 mmol; 42%).

Analog werden folgende Verbindungen hergestellt:

| | Edukt1 | Edukt 2 | Produkt | Ausbeute |
|---|---|---|---|---|
| 7b | 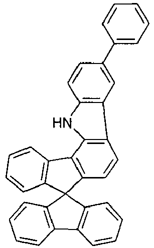 | 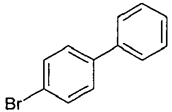 | 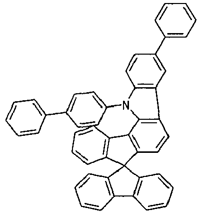 | 41% |
| | | 92-66-0 | | |
| 7c | 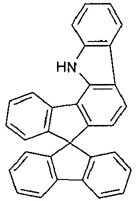 | 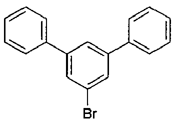 | 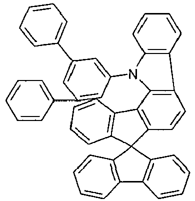 | 27% |
| | | 103068-20-8 | | |
| 7d | 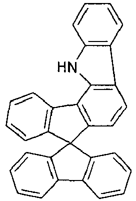 | 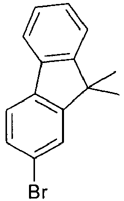 | 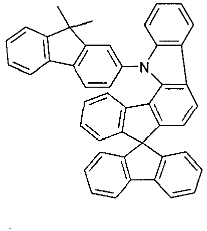 | 29% |
| | | 28320-31-2 | | |
| 7e | 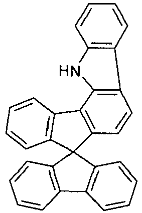 | 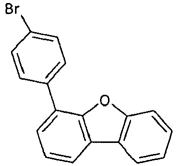 | 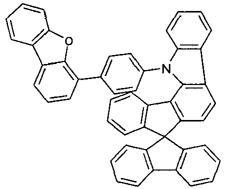 | 54% |
| | | 955959-84-9 | | |
| 7f | 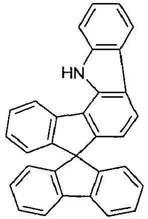 | 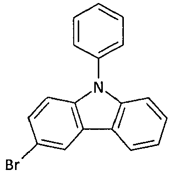 | 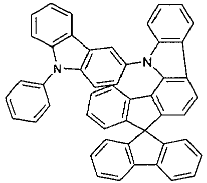 | 62% |
| | | 1153-85-1 | | |
| 7g | 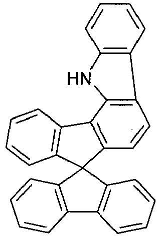 | 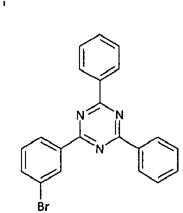 | 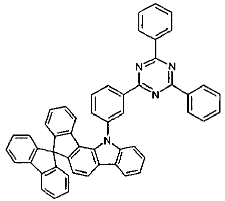 | 53% |
| | | 864377-31-1 | | |
| 7h | 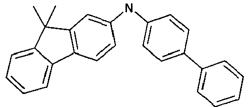 | 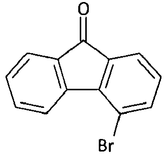 | 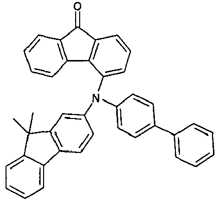 | 91% (ohne Sublimation) |
| | 1359833-90-1 | 4269-17-4 | Vergleichsbeispiel | |
| 7j | 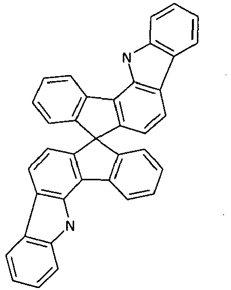 | 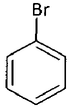 | 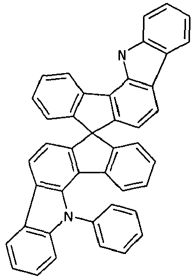 | 82% (ohne Sublimation) |
| | | 108-86-1 | Zwischenprodukt | |
| 7k | 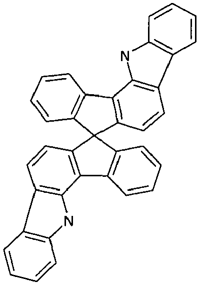 | 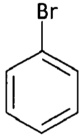 | 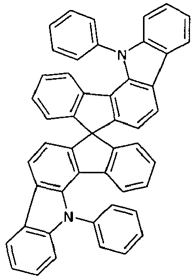 | 32% |
| | | 108-86-1 | | |
| 7l | 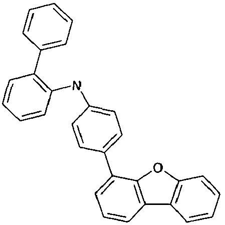 | 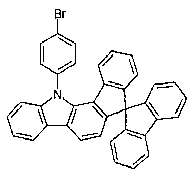 | 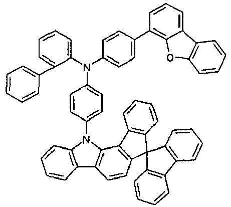 | 42% |
| 7m | 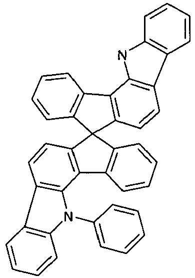 | 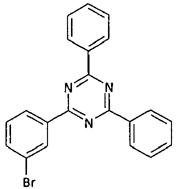 | 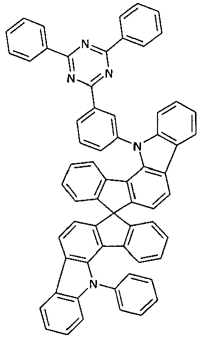 | 41% |
| | | 864377-31-1 | | |
| 7n | 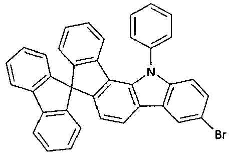 | 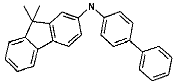 | 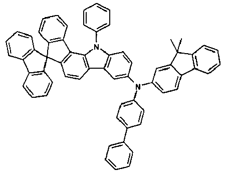 | 37% |
| | | 1359833-90-1 | | |
| 7o | 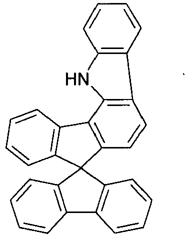 | 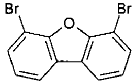 | 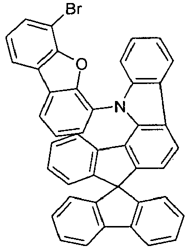 | 57% (ohne Sublimation) |
| | | 201138-91-2 | | |
| 7p | 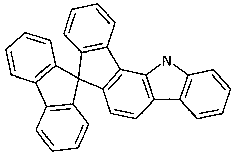 | 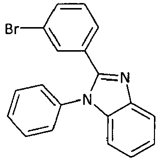 | 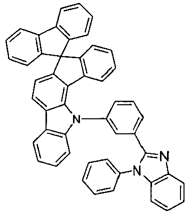 | 31% |
| 7q | 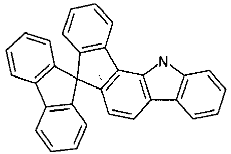 | 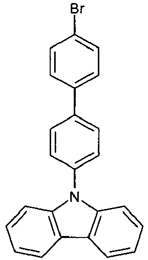 | 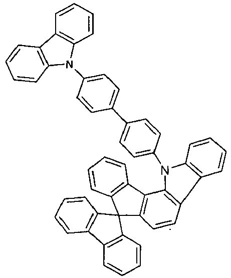 | 39% |
| 7r | 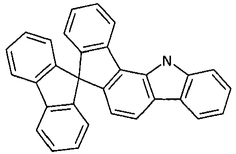 | 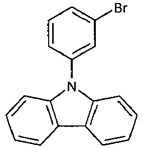 | 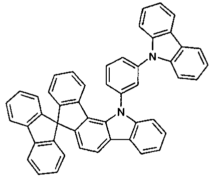 | 34% |
| | | 185112-61-2 | | |
| 7s | 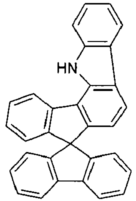 | 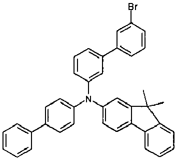 | 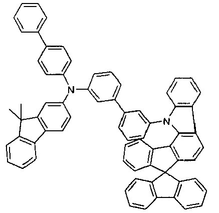 | 48% |

### Beispiel 8

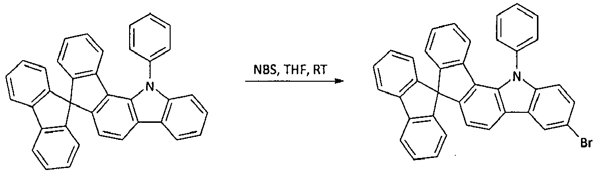

In einem 500 mL Vierhalskolben werden 7 g (14.5 mmol) Phenyl-4-spiro-carbazol und 2.7 g (15 mmol) NBS in 300 mL THF gelöst und 48 h bei Raumtemperatur bis zur vollständigen Umsetzung gerührt. Anschließend wird mit 50 mL Wasser hydrolysiert und die organischen Lösemittel unter vermindertem Druck entfernt. Der erhaltene Feststoff wird einmal mit 300 mL Ethanol heiß ausgerührt. Nach Abkühlen auf Raumtemperatur wird der Feststoff filtriert. Nach Trocknung unter vermindertem Druck wird das Produkt als farbloser Feststoff erhalten. Die Ausbeute beträgt 7.3 g (13 mmol, entspr. 90 % d.Th).

### Beispiel 9a

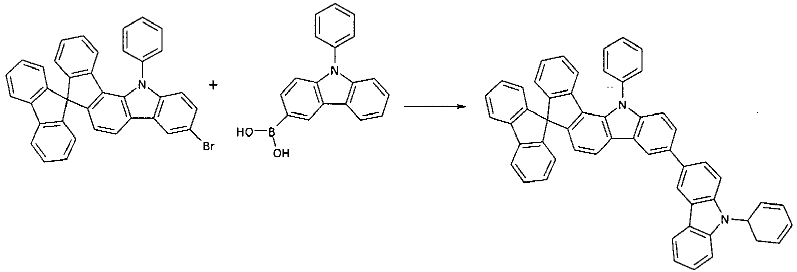

In einem 1-L-Vierhalskolben werden 17.5 g (31 mmol) **8a,** 9.2 g (32 mmol) Phenylcarbazolboronsäure, 26.5 g (125 mmol) Trikaliumphosphat in 120 mL Glyme, 170 mL Toluol und 120 mL Wasser gelöst und 30 min. Argon durchgeleitet. Anschließend werden 140 mg (0.6 mmol) Palladiumacetat und 380 mg (1.2 mmol) Tri-o-tolylphospin hinzugegeben und 16 h unter Rückfluss erhitzt. Nach vollständigem Umsatz wird das Reaktionsgemisch auf Raumtemperatur abgekühlt, der ausgefallene Feststoff filtriert und mit Ethanol und Heptan gewaschen. Anschließend wird der Feststoff zweimal mit Toluol heiß extrahiert, aus Heptan/Toluol umkristallisiert und bei 400 °C und 2x10⁻⁵ bar sublimiert. Das Produkt wird als farbloser Feststoff mit einer HPLC-Reinheit von 99.95% erhalten. Die Ausbeute beträgt 10.2 g (14 mmol entspr. 45 % d.Th.)

Analog werden folgende Verbindungen hergestellt:

| | Edukt 1 | Edukt 2 | Produkt | Ausbeute |
|---|---|---|---|---|
| 9b | 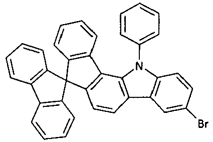 | 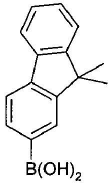 | 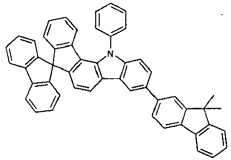 | 37% |
| | | 333432-28-3 | | |
| 9c | 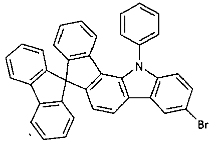 | 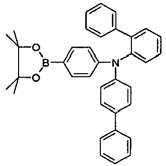 | 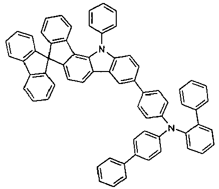 | 38% |
| | | s. Bsp 10 | | |
| 9d | 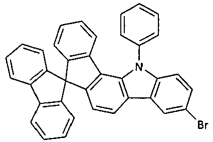 | 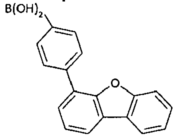 | 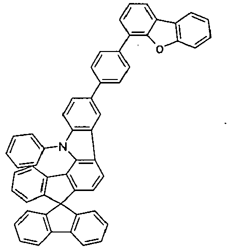 | 50% |
| | | 796071-96-0 | | |
| 9e | 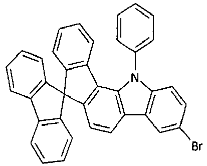 | 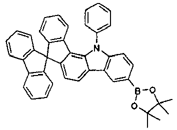 | 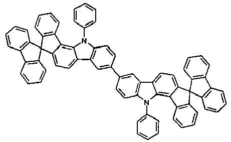 | 61% |
| | | s. Bsp 10 | | |

### Beispiel 10a (Zwischenprodukt)

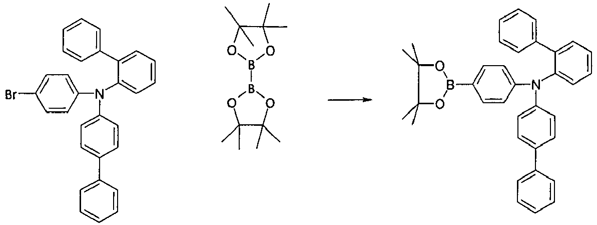

In einem 2-L-Vierhalskoben werden 33.8 g (71 mmol) Biphenyl-2-yl-biphenyl-4-yl-(4-bromphenyl)-amin (1371651-92-1), 21.9 g (86 mmol) Bispinacolatodiboran (73183-34-3), 21.7 g (221 mmol) Kaliumacetat und 1.7 g (2.1 mmol) 1,1-Bis(diphenylphosphino)ferrocen-dichloropalladium(II) Komplex mit DCM in 1000 mL wasserfreiem Dioxan 16 h bis zum vollständigen Umsatz unter Rückfluss erhitzt. Nach Abkühlen auf Raumtemperatur wird die organische Phase mit Ethylacetat erweitert, dreimal mit 300 mL Wasser gewaschen und mit Natriumsulfat getrocknet. Die vereinigten organischen Phasen werden bis zur Trockene einrotiert. Nach Umkristallisation aus Heptan wird das Produkt als Feststoff erhalten. Die Ausbeute beträgt 22.6 g (41 mmol; 61 %).

wird die folgende Verbindung als Zwischenprodukt hergestellt:

| | Edukt | Produkt | Ausbeute |
|---|---|---|---|
| 10b | 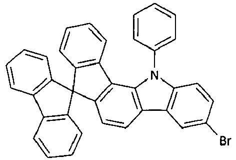 | 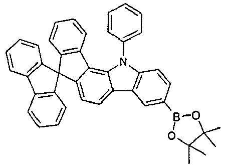 | 92% |

### Beispiel 11a (Zwischenprodukt)

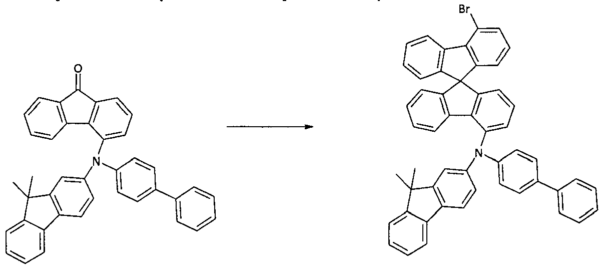

Aus 2.7 g (110 mmol) mit Jod aktivierten Magnesiumspänen und einer Mischung aus 25.6 g (110 mmol) 2-Brombiphenyl, 0.8 ml 1,2-Dichlorethan, 50 ml 1,2-Dimethoxyethan, 400 ml THF und 200 ml Toluol wird unter Begleitheizen mit einem 70 °C warmen Ölbad das entsprechende Grignard-Reagenz dargestellt. Nachdem das Magnesium vollständig abreagiert hat, lässt man auf Raumtemperatur abkühlen und tropft dann eine Lösung von 25.9 g (100 mmol) 4-[Biphenyl-4-yl-(9,9-dimethyl-9H-fluoren-2-yl)-amino]-fluoren-9-on **(7h)** in 500 ml THF zu, erwärmt die Reaktionsmischung für 4 h auf 50 °C und rührt dann 12 h bei Raumtemperatur nach. Man gibt 100 ml Wasser zu, rührt kurz nach, trennt die organische Phase ab und entfernt das Lösungsmittel im Vakuum. Der Rückstand wird in der Wärme bei 40 °C in 500 ml Eisessig suspendiert, die Suspension wird mit 0.5 ml konz. Schwefelsäure versetzt und anschließend 2 h bei 100 °C nachgerührt. Nach Erkalten saugt man vom ausgefallenen Feststoff ab, wäscht diesen einmal mit 100 ml Eisessig, dreimal mit je 100 ml Ethanol und kristallisiert abschließend aus Dioxan um. Ausbeute: 26.9 g (68 mmol), 68 %; Reinheit ca. 98 % n. ¹H-NMR.

Analog werden folgende Verbindungen als Zwischenprodukte hergestellt:

| | Edukt1 | Edukt 2 | Produkt | Ausbeute |
|---|---|---|---|---|
| 11b | 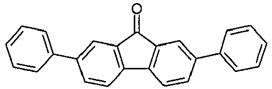 | 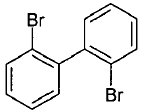 | 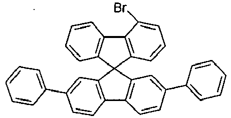 | 56% |
| | 115033-91-5 | 13029-09-9 | | |
| 11c | 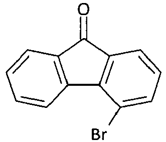 | 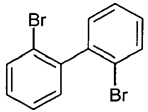 | 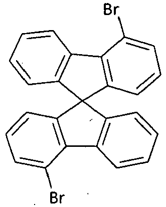 | 47% |
| | 4269-17-4 | 13029-09-9 | | |
| 11d | 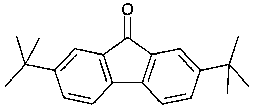 | 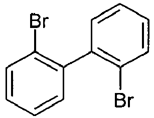 | 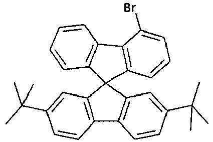 | 49% |
| | 58775-13-6 | 13029-09-9 | | |
| 11e | 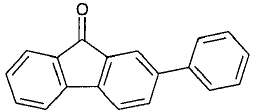 | 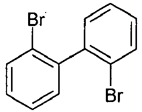 | 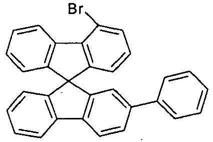 | 51% |
| | 3096-49-9 | 1329-09-9 | | |

### Beispiel 12: Synthese der Amin-Bausteine (Zwischenprodukt)

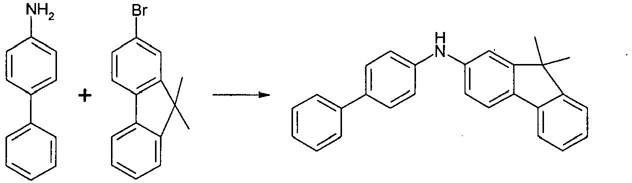

24.0 g (142 mmol, 1.2 eq.) 4-Aminobiphenyl (CAS 92-67-1) und 32.0 g (117 mmol, 1.0 eq.) 2-Brom-9,9'-dimethylfluoren (CAS 28320-31-2) werden in 950 ml Toluol vorgelegt und 30 Minuten mit Argon gesättigt. Anschließend werden 1.0 g (1.8 mmol, 0.02 eq.) 1,1'-Bis(diphenylphosphino)ferrocen (CAS 12150-46-8), 350 mg (1.6 mmol, 0.01 eq.) Palladium(II)-acetat (CAS 3375-31-3) und 29 g (300 mmol, 2.6 eq.) Natrium-*tert*-butylat (CAS 865-48-5) zugegeben und über Nacht unter Rückfluss erhitzt. Nach beendeter Reaktion wird der Ansatz mit 300 ml Toluol verdünnt und mit Wasser extrahiert. Die organische Phase wird über Natriumsulfat getrocknet und das Lösungsmittel am Rotationsverdampfer entfernt. Das braune Öl wird mit 50 ml Ethylacetat versetzt und in eine Mischung aus Heptan/Essigester 20:1 gegeben. Der entstandene Feststoff wird abgesaugt und mit Heptan gewaschen. Nach Trocknung werden 29 g (80mmol, 69 %) des gewünschten Produkts mit einer HPLC-Reinheit von 99.1% erhalten.

Analog werden folgende Verbindungen als Zwischenprodukte hergestellt:

| **Bsp.** | **Edukt 1** | **Edukt 2** | **Produkt** | **Ausbeute** |
|---|---|---|---|---|
| 12b | 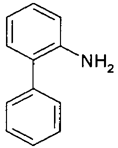 | 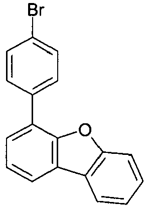 | 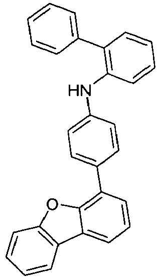 | 62% |
| | 90-41-5 | 955959-84-9 | | |

### Beispiel 13: Einführung der Brücke an den Aminbaustein (Zwischenprodukt)

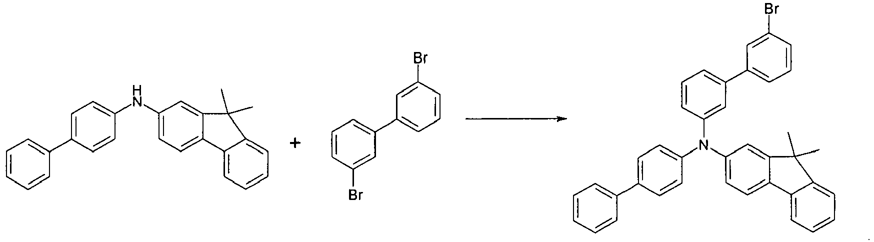

29 g (80 mmol, 1.0 eq.) des Zwischenprodukts **12a** und 25 g (80 mmol, 1.0 eq.) 3,3'-Dibrom-1,1'-biphenyl (CAS 16400-51-4) werden in 600 ml Toluol gelöst und für 30 Minuten entgast. Anschließend werden 45 g (240 mmol, 3.0 eq.) Natrium-*tert*-butylat, 890 mg (0.40 mmol, 0.050 eq.) Palladium(II)-acetat und 8 ml (8.0 mmol, 0.10 eq.) einer 1M tri-*tert*-Butylphosphin-Lösung zugegeben. Der Ansatz wird über Nacht unter Rückfluss erhitzt und nach beendeter Reaktion zweimal über Aluminiumoxid mit Toluol filtriert. Nach Entfernung des Lösungsmittels am Rotationsverdampfer wird das Öl in etwas THF gelöst und in Heptan eingetragen. Der entstandene Feststoff wird abgesaugt und mittels Heißextraktion in Heptan/Toluol 1:1 aufgereinigt. Es werden 16.6g (28mmol, 35%) des gewünschten Produkts erhalten.

### Beispiel 14: Synthese des Triazin-Bausteins (Zwischenprodukt) Stufe 1:

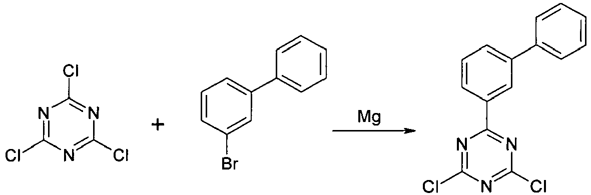

7.9 g (330 mmol, 1.2 eq.) Magnesiumspäne werden in einem 1L-Vierhalskolben vorgelegt und so langsam mit einer THF-Lösung aus 63 g (270 mmol, 1.0 eq.) 3-Brombiphenyl (CAS 2113-57-7) versetzt, um den Rückfluss des Reaktionsgemisches zu erhalten. Nach beendeter Zugabe wird der Ansatz für weitere 2 h unter Rückfluss erhitzt.

In einem 2L-Vierhalskolben werden 50 g (270 mmol, 1 eq.) 2,4,6-Trichlor-[1,3,5]triazin (CAS 108-77-0) in 500 ml THF auf -10 °C abgekühlt. Bei dieser Temperatur wird die Grignard-Lösung so langsam zugetropft, dass die Temperatur 0 °C nicht übersteigt und der Ansatz schließlich über Nacht bei Raumtemperatur gerührt. Zur Aufarbeitung werden 270 ml einer 1N Salzsäure zugetropft und das Gemisch für 1 h gerührt. Anschließend wird die wässrige Phase abgetrennt und mit Diethylether extrahiert. Die vereinten organischen Phasen werden über Natriumsulfat getrocknet und das Lösungsmittel am Rotationsverdampfer entfernt. Es werden 56 g (69%) eines farblosen Öls erhalten.

Analog werden folgende Verbindungen als Zwischenprodukte hergestellt:

| **Bsp.** | **Edukt** 1 | **Edukt 2** | **Produkt** | **Ausbeute** |
|---|---|---|---|---|
| 14b | 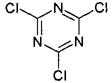 | 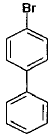 | 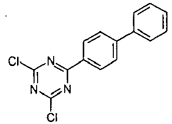 | 56% |
| | | 92-66-0 | | |
| 14c | 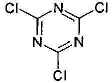 | 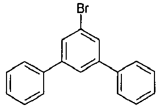 | 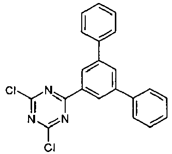 | 71% |
| | | 103068-20-8 | | |

### Stufe 2:

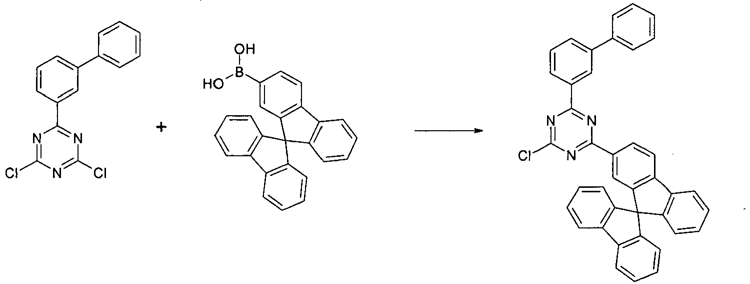

### Variante A:

18 g (50 mmol, 1 eq.) 9,9-Spirobifluoren-2-yl-boronsäure (CAS 236389-21-2), 15 g (50 mmol, 1 eq.) 2-Biphenyl-3-yl-4,6-dichlor-[1,3,5]triazin **14a** und 5.8 g (55 mmol, 1.1 eq.) Natriumcarbonat werden in einem Gemisch aus 200 ml Dioxan, 200 ml Toluol und 70 ml Wasser gelöst und für 30 Minuten entgast. Anschließend werden 580 mg (0.50 mmol, 1 mol-%) Tetrakis(triphenylphosphin) (CAS 14221-01-3) zugegeben und der Ansatz über Nacht am Rückfluss erhitzt. Das Reaktionsgemisch wird abgekühlt und mit 300 ml Wasser versetzt. Die wässrige Phase wird dreimal mit Ethylacetat extrahiert, die organischen Phasen vereint und das Lösungsmittel am Rotationsverdampfer entfernt. Nach Heißextraktion in Heptan/Toluol 4:1 werden 15g (26mmol, 51%) eines farblosen Feststoffs erhalten.

### Variante B: analog Stufe 1.

Analog werden folgende Verbindungen als Zwischenprodukte hergestellt:

| **Bsp.** | **Variante** | **Edukt 1** | **Edukt 2** | **Produkt** | **Ausbeute** |
|---|---|---|---|---|---|
| 15b | A | 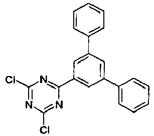 | 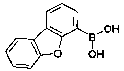 | 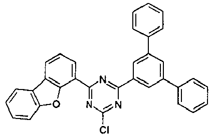 | 63% |
| | | | 100124-06-9 | | |
| 15c | A | 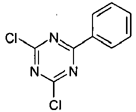 | 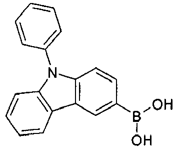 | 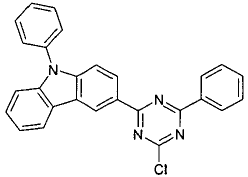 | 68% |
| | | 1700-02-3 | 854952-58-2 | | |
| 15d | B | 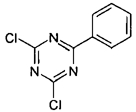 | 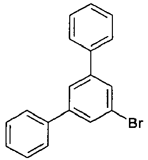 | 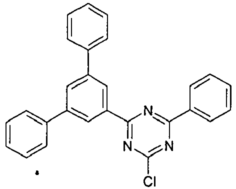 | 67% |
| | | 1700-02-3 | 103068-20-8 | | |
| 15e | B | 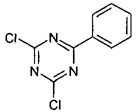 | 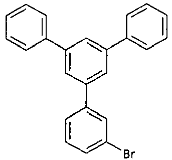 | 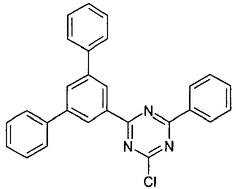 | 57% |
| | | 1700-02-3 | 1233200-57-1 | | |

### Beispiel 16: Herstellung der OLEDs

In den folgenden Beispielen V1 bis E77 (siehe Tabellen 1 und 2) werden die Daten verschiedener OLEDs vorgestellt.

**Vorbehandlung für die Beispiele V1-V7 und E1-E49:** Gereinigte Glasplättchen (Reinigung in Miele Laborspülmaschine, Reiniger Merck Extran), die mit strukturiertem ITO (Indium Zinn Oxid) der Dicke 50 nm beschichtet sind, werden zur verbesserten Prozessierung mit 20 nm PEDOT:PSS beschichtet (Poly(3,4-ethylendioxythiophen)poly(styrolsulfonat), bezogen als CLEVIOS™ P VP Al 4083 von Heraeus Precious Metals GmbH Deutschland, aus wässriger Lösung aufgeschleudert). Die Proben werden anschließend bei 180 °C für 10 min ausgeheizt. Diese beschichteten Glasplättchen bilden die Substrate, auf welche die OLEDs aufgebracht werden.

**Vorbehandlung für die Beispiele E50-E55:** Gereinigte Glasplättchen (Reinigung in Miele Laborspülmaschine, Reiniger Merck Extran), die mit strukturiertem ITO (Indium Zinn Oxid) der Dicke 50 nm beschichtet sind, werden 130 s lang mit einem Sauerstoffplasma behandelt. Diese mit Plasma behandelten Glasplättchen bilden die Substrate, auf welche die OLEDs aufgebracht werden. Die Substrate verbleiben vor der Beschichtung im Vakuum. Die Beschichtung beginnt innerhalb 10 min nach der Plasmabehandlung.

**Vorbehandlung für die Beispiele E56-E77:** Gereinigte Glasplättchen (Reinigung in Miele Laborspülmaschine, Reiniger Merck Extran), die mit strukturiertem ITO (Indium Zinn Oxid) der Dicke 50 nm beschichtet sind, werden 130 s lang mit einem Sauerstoffplasma und anschließend 150 s lang mit einem Argonplasma behandelt. Diese mit Plasma behandelten Glasplättchen bilden die Substrate, auf welche die OLEDs aufgebracht werden. Die Substrate verbleiben vor der Beschichtung im Vakuum. Die Beschichtung beginnt innerhalb 10 min nach der Plasmabehandlung.

Die OLEDs haben prinzipiell folgenden Schichtaufbau: Substrat / optionale Lochinjektionsschicht (HIL) / Lochtransportschicht (HTL) / Zwischenschicht (IL) / Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt. Eine Bezeichnung wie "3a" bezieht sich hierbei auf die in den Tabellen zu den Beispiel 3a gezeigten Materialien. Analoges gilt für die anderen Materialien.

Die Synthese der Verbindung WB1 erfolgt analog WO 2009/124627. Die Synthese der Verbindung TEG3 ist beschrieben in WO 2011/032626, von Verbindung TER2 in WO 2011/032626, von Verbindung IC4 in WO 2010/136109.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie ST1:7b:TEG1 (30%:58%:12%) bedeutet hierbei, dass das Material ST1 in einem Volumenanteil von 30%, 7b in einem Anteil von 58% und TEG1 in einem Anteil von 12% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Als Lebensdauer LD wird die Zeit definiert, nach der die Leuchtdichte bei Betrieb mit konstantem Strom von der Startleuchtdichte auf einen gewissen Anteil L1 absinkt. Eine Angabe von L0;j0 = 4000 cd/m² und L1 = 70 % in Tabelle X2 bedeutet, dass die in Spalte LD angegebene Lebensdauer der Zeit entspricht, nach der die Leuchtdichte von ihrem Anfangswert 4000 cd/m² auf 2800 cd/m² absinkt. Analog bedeutet L0;j0 = 20 mA/cm², L1 = 80 %, dass die Leuchtdichte bei Betrieb mit 20 mA/cm² nach der Zeit LD auf 80 % ihres Anfangswertes absinkt.

Die Daten der verschiedenen OLEDs sind in Tabelle 2 zusammengefasst. Die Beispiel V1-V7 sind Vergleichsbeispiele gemäß dem Stand der Technik, die Beispiele E1-E77 zeigen Daten von OLEDs mit erfindungsgemäßen Materialien.

Im Folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt. Wie sich der Tabelle entnehmen lässt, werden auch bei Verwendung der nicht näher ausgeführten erfindungsgemäßen Verbindungen deutliche Verbesserungen gegenüber dem Stand der Technik erzielt, teilweise in allen Parametern, in manchen Fällen ist aber nur eine Verbesserung von Effizienz oder Spannung oder Lebensdauer zu beobachten. Allerdings stellt bereits die Verbesserung einer der genannten Paramter einen signifikanten Fortschritt dar, weil verschiedene Anwendungen die Optimierung hinsichtlich unterschiedlicher Parameter erfordern.

### Verwendung von erfindungsgemäßen Verbindungen als Elektronentransportmaterialien

Verwendet man statt der Diphenyl-Fluoren Verbindung StdT2 gemäß dem Stand der Technik die analoge erfindungsgemäße Spiroverbindung 3a als Elektronentransportmaterial, so erhält man in Verbindung mit dem grün phosphoreszierenden Dotanden TEG1 eine deutliche Verbesserung der Leistungseffizienz um etwa 15% aufgrund der deutlich verbesserten Spannung und der verbesserten externen Quanteneffizienz. Die Lebensdauer wird ebenfalls leicht verbessert (Beispiele V6, E24). Auch in Kombination mit dem blau fluoreszierenden Dotanden D1 erhält man sehr gute Leistungsdaten (Beispiel E25).

### Verwendung von erfindungsgemäßen Verbindungen als Matrixmaterialien in phosphoreszierenden OLEDs

In Kombination mit dem grün phosphoreszierenden Dotanden TEG1 erhält man bei Einsatz der erfindungsgemäßen Verbindung 3a, bei der das Triazin über den Stickstoff angebunden ist, eine deutliche Verbesserung der Spannung um 0.3 V und Lebensdauer um 35% im Vergleich zur Verbindung StdT1, bei der die Triazingruppe am Fluorenteil des Spiros angebunden ist. Auch die externe Quanteneffizienz verbessert sich, was insgesamt zu einer deutlichen Verbesserung der Leistungseffizienz um fast 20% führt (Beispiele V1, E1). Im Vergleich zur Diphenyl-Fluoren Verbindung StdT2 ist vor allem eine Verbesserung der Lebensdauer zu beobachten (Beispiele V4, E1). Ähnliche Verbesserungen ergeben sich in Kombination mit dem rot phosphoreszierenden Dotanden TER1 bei Einsatz erfindungsgemäßer Verbindungen (Beispiele V5, E30, E31). Bei Kombination mit Verbindung IC3 in der Emissionsschicht erhält man ebenfalls deutliche Vorteile durch die erfindungsgemäßen Verbindungen (Beispiele V7, E21), vor allem eine Verbesserung der Lebensdauer um fast 40%.

Schließlich führt die Kombination von erfindungsgemäßen Verbindungen mit der Verbindung ST1 bzw IC2 in der Emissionsschicht zu sehr guten Leistungsdaten (Beispiele E39, E40, E41, E42).

**Tabelle 1: Aufbau der OLEDs**

| Bsp. | HIL | HTL | IL | EBL | EML | HBL | ETL | EIL |
|---|---|---|---|---|---|---|---|---|
| | Dicke | Dicke | Dicke | Dicke | Dicke | Dicke | Dicke | Dicke |
| V1 | --- | SpA1 | HATCN | SpMA1 | StdT1:TEG1 | --- | ST2:LiQ | --- |
| | | 70nm | 5nm | 90nm | (90%:10%) 30nm | | (50%:50%) 40nm | |
| V2 | --- | SpA1 | HATCN | SpMA1 | StdT2:TEG2 | ST2 | ST2:LiQ | --- |
| | | 70nm | 5nm | 90nm | (90%:17%) 30nm | 10nm | (50%:50%) 30nm | |
| V3 | --- | SpA1 | HATCN | SpMA1 | StdT3:TEG2 | --- | ST2:LiQ | --- |
| | | 70nm | 5nm | 90nm | (90%:17%) 30nm | | (50%:50%) 40nm | |
| V4 | --- | SpA1 | HATCN | SpMA1 | StdT2:TEG1 | --- | ST2:LiQ | --- |
| | | 70nm | 5nm | 90nm | (90%:10%) 30nm | | (50%:50%) 40nm | |
| V5 | --- | SpA1 | HATCN | SpMA1 | StdT1:TER1 | --- | ST2:LiQ | --- |
| | | 90nm | 5nm | 130nm | (92%:8%) 40nm | | (50%:50%) 40nm | |
| V6 | --- | SpA1 | HATCN | SpMA1 | IC1:TEG1 | --- | StdT2 | LiQ |
| | | 70nm | 5nm | 90nm | (90%:10%) 30nm | | 40nm | 4nm |
| V7 | --- | SpA1 | HATCN | SpMA1 | StdT2:IC3:TEG1 | IC1 | ST2:LiQ | --- |
| | | 70nm | 5nm | 90nm | (45%:45%:10%) 30nm | 10nm | (50%:50%) 30nm | |
| E1 | --- | SpA1 | HATCN | SpMA1 | 3a:TEG1 | --- | ST2:LiQ | --- |
| | | 70nm | 5nm | 90nm | (90%:10%) 30nm | | (50%:50%) 40nm | |
| E2 | --- | SpA1 | HATCN | SpMA1 | 3a:TEG2 | --- | ST2:LiQ | --- |
| | | 70nm | 5nm | 90nm | (90%:17%) 30nm | | (50%:50%) 40nm | |
| E3 | --- | SpA1 | HATCN | SpMA1 | 3a:TEG2 | ST2 | ST2:LiQ | --- |
| | | 70nm | 5nm | 90nm | (90%:17%) 30nm | 10nm | (50%:50%) 30nm | |
| E4 | --- | SpA1 | HATCN | SpMA1 | 3j:TEG1 | --- | ST2:LiQ | --- |
| | | 70nm | 5nm | 90nm | (90%:10%) 30nm | | (50%:50%) 40nm | |
| E5 | --- | SpA1 | HATCN | SpMA1 | 3b:TEG1 | --- | ST2:LiQ | --- |
| | | 70nm | 5nm | 90nm | (90%:10%) 30nm | | (50%:50%) 40nm | |
| E6 | --- | SpA1 | HATCN | SpMA1 | 3g:TEG1 | --- | ST2:LiQ | --- |
| | | 70nm | 5nm | 90nm | (90%:10%) 30nm | | (50%:50%) 40nm | |
| E7 | --- | SpA1 | HATCN | SpMA1 | 3k:TEG1 | --- | ST2:LiQ | --- |
| | | 70nm | 5nm | 90nm | (90%:10%) 30nm | | (50%:50%) 40nm | |
| E8 | --- | SpA1 | HATCN | SpMA1 | 31:TEG1 | --- | ST2:LiQ | --- |
| | | 70nm | 5nm | 90nm | (90%:10%) 30nm | | (50%:50%) 40nm | |
| E9 | --- | SpA1 | HATCN | SpMA1 | 3m:TEG1 | --- | ST2:LiQ | --- |
| | | 70nm | 5nm | 90nm | (90%:10%) 30nm | | (50%:50%) 40nm | |
| E10 | --- | SpA1 | HATCN | SpMA1 | 3n:TEG1 | --- | ST2:LiQ | --- |
| | | 70nm | 5nm | 90nm | (90%:10%) 30nm | | (50%:50%) 40nm | |
| E11 | --- | SpA1 | HATCN | SpMA1 | 3o:TEG1 | --- | ST2:LiQ | --- |
| | | 70nm | 5nm | 90nm | (90%:10%) 30nm | | (50%:50%) 40nm | |
| E12 | --- | SpA1 | HATCN | SpMA1 | 6g:TEG1 | --- | ST2:LiQ | --- |
| | | 70nm | 5nm | 90nm | (90%:10%) 30nm | | (50%:50%) 40nm | |
| E13 | --- | SpA1 | HATCN | SpMA1 | 6e:TEG1 | --- | ST2:LiQ | --- |
| | | 70nm | 5nm | 90nm | (90%:10%) 30nm | | (50%:50%) 40nm | |
| E14 | --- | SpA1 | HATCN | SpMA1 | 6e:7a:TEG1 | IC1 | ST2:LiQ | --- |
| | | 70nm | 5nm | 90nm | (30%:55%:15%) 30nm | 10nm | (50%:50%) 40nm | |
| E15 | --- | SpA1 | HATCN | SpMA1 | 7m:TEG1 | --- | ST2:LiQ | --- |
| | | 70nm | 5nm | 90nm | (90%:10%) 30nm | | (50%:50%) 40nm | |
| E16 | --- | SpA1 | HATCN | SpMA1 | IC2:7k:TEG1 | IC1 | ST2:LiQ | --- |
| | | 70nm | 5nm | 90nm | (40%:45%:15%) 30nm | 10nm | (50%:50%) 40nm | |
| E17 | --- | SpA1 | HATCN | SpMA1 | IC2:9b:TEG1 | IC1 | ST2:LiQ | --- |
| | | 70nm | 5nm | 90nm | (30%:55%:15%) 30nm | 10nm | (50%:50%) 40nm | |
| E18 | --- | SpA1 | HATCN | SpMA1 | IC2:9d:TEG1 | IC1 | ST2:LiQ | --- |
| | | 70nm | 5nm | 90nm | (30%:55%:15%) 30nm | 10nm | (50%:50%) 40nm | |
| E19 | --- | SpA1 | HATCN | SpMA1 | IC2:9h:TEG1 | IC1 | ST2:LiQ | --- |
| | | 70nm | 5nm | 90nm | (30%:55%:15%) 30nm | 10nm | (50%:50%) 40nm | |
| E20 | --- | SpA1 | HATCN | SpMA1 | IC1:TEG1 | 3a | ST2:LiQ | --- |
| | | 70nm | 5nm | 90nm | (90%:10%) 30nm | 10nm | (50%:50%) 30nm | |
| E21 | --- | SpA1 | HATCN | SpMA1 | 3a:IC3:TEG1 | IC1 | ST2:LiQ | --- |
| | | 70nm | 5nm | 90nm | (45%:45%:10%) 30nm | 10nm | (50%:50%) 30nm | |
| E22 | --- | SpA1 | HATCN | SpMA1 | IC1:TEG1 | --- | 3a | LiQ |
| | | 70nm | 5nm | 90nm | (90%:10%) 30nm | | 40nm | 4nm |
| E23 | --- | SpA1 | HATCN | SpMA1 | IC1:TEG1 | --- | 6i | LiF |
| | | 70nm | 5nm | 90nm | (90%:10%) 30nm | | 40nm | 1nm |
| E24 | --- | SpA1 | HATCN | SpMA1 | IC1:TEG1 | --- | 7p | LiF |
| | | 70nm | 5nm | 90nm | (90%:10%) 30nm | | 40nm | 1nm |
| E25 | HATCN | SpA1 | HATCN | SpMA1 | M1:D1 | --- | 3a | LiQ |
| | 5nm | 140nm | 5nm | 20nm | (95%:5%) 20nm | | 30nm | 4nm |
| E26 | SpA1 | HATCN | SpMA1 | 9c | IC2:TEG2 | --- | ST2:LiQ | --- |
| | 70nm | 5nm | 80nm | 10nm | (83%:17%) 30nm | | (50%:50%) 40nm | |
| E27 | SpA1 | HATCN | SpMA1 | 7s | IC2:TEG2 | --- | ST2:LiQ | --- |
| | 70nm | 5nm | 80nm | 10nm | (83%:17%) 30nm | | (50%:50%) 40nm | |
| E28 | SpA1 | HATCN | SpMA1 | 71 | IC2:TEG2 | --- | ST2:LiQ | --- |
| | 70nm | 5nm | 80nm | 10nm | (83%:17%) 30nm | | (50%:50%) 40nm | |
| E29 | SpA1 | HATCN | SpMA1 | 7n | IC2:TEG2 | --- | ST2:LiQ | --- |
| | 70nm | 5nm | 80nm | 10nm | (83%:17%) 30nm | | (50%:50%) 40nm | |
| E30 | --- | SpA1 | HATCN | SpMA1 | 3c:TER1 | --- | ST2:LiQ | --- |
| | | 90nm | 5nm | 130nm | (92%:8%) 40nm | | (50%:50%) 40nm | |
| E31 | --- | SpA1 | HATCN | SpMA1 | 3f:TER1 | --- | ST2:LiQ | --- |
| | | 90nm | 5nm | 130nm | (92%:8%) 40nm | | (50%:50%) 40nm | |
| E32 | --- | SpA1 | HATCN | SpMA1 | IC2:7q:TER1 | --- | ST2:LiQ | --- |
| | | 90nm | 5nm | 130nm | (92%:12%) 40nm | | (50%:50%) 40nm | |
| E33 | --- | SpA1 | HATCN | SpMA1 | IC2:7r:TER1 | --- | ST2:LiQ | --- |
| | | 90nm | 5nm | 130nm | (92%:12%) 40nm | | (50%:50%) 40nm | |
| E34 | --- | SpA1 | HATCN | SpMA1 | IC2:7f:TER1 | --- | ST2:LiQ | --- |
| | | 90nm | 5nm | 130nm | (92%:12%) 40nm | | (50%:50%) 40nm | |
| E35 | --- | SpA1 | HATCN | SpMA1 | 6a:TEG1 | --- | ST2:LiQ | --- |
| | | 70nm | 5nm | 90nm | (90%:10%) 30nm | | (50%:50%) 40nm | |
| E36 | --- | SpA1 | HATCN | SpMA1 | 6b:TEG1 | --- | ST2:LiQ | --- |
| | | 70nm | 5nm | 90nm | (90%:10%) 30nm | | (50%:50%) 40nm | |
| E37 | --- | SpA1 | HATCN | SpMA1 | 6c:TEG1 | --- | ST2:LiQ | --- |
| | | 70nm | 5nm | 90nm | (90%:10%) 30nm | | (50%:50%) 40nm | |
| E38 | --- | SpA1 | HATCN | SpMA1 | 6f:TEG1 | --- | ST2:LiQ | --- |
| | | 70nm | 5nm | 90nm | (90%:10%) 30nm | | (50%:50%) 40nm | |
| E39 | --- | SpA1 | HATCN | SpMA1 | ST1:7a:TEG1 | IC1 | ST2:LiQ | --- |
| | | 70nm | 5nm | 90nm | (30%:58%:12%) 30nm | 10nm | (50%:50%) 30nm | |
| E40 | --- | SpA1 | HATCN | SpMA1 | ST1:7b:TEG1 | IC1 | ST2:LiQ | --- |
| | | 70nm | 5nm | 90nm | (30%:58%:12%) 30nm | 10nm | (50%:50%) 30nm | |
| E41 | --- | SpA1 | HATCN | SpMA1 | ST1:7c:TEG1 | IC1 | ST2:LiQ | --- |
| | | 70nm | 5nm | 90nm | (30%:58%:12%) 30nm | 10nm | (50%:50%) 30nm | |
| E42 | --- | SpA1 | HATCN | SpMA1 | IC2:7d:TER1 | --- | ST2:LiQ | --- |
| | | 90nm | 5nm | 130nm | (30%:60%:10%) 40nm | | (50%:50%) 40nm | |
| E43 | --- | SpA1 | HATCN | SpMA1 | IC2:3i:TEG2 | ST2 | ST2:LiQ | --- |
| | | 70nm | 5nm | 90nm | (44%:44%:12%) 30nm | 10nm | (50%:50%) 30nm | |
| E44 | --- | SpA1 | HATCN | SpMA1 | 3i:TEG2 | ST2 | ST2:LiQ | --- |
| | | 70nm | 5nm | 90nm | (88%:12%) 30nm | 10nm | (50%:50%) 30nm | |
| E45 | --- | SpA1 | HATCN | SpMA1 | 3j:TEG2 | ST2 | ST2:LiQ | --- |
| | | 70nm | 5nm | 90nm | (83%:17%) 30nm | 10nm | (50%:50%) 30nm | |
| E46 | --- | SpA1 | HATCN | SpMA1 | IC2:7e:TEG2 | ST2 | ST2:LiQ | --- |
| | | 70nm | 5nm | 90nm | (28%:55%:17%) 30nm | 10nm | (50%:50%) 30nm | |
| E47 | --- | SpA1 | HATCN | SpMA1 | IC2:9a:TEG2 | ST2 | ST2:LiQ | --- |
| | | 70nm | 5nm | 90nm | (44%:44%:12%) 30nm | 10nm | (50%:50%) 30nm | |
| E48 | --- | SpA1 | HATCN | SpMA1 | IC2:9e:TEG2 | ST2 | ST2:LiQ | --- |
| | | 70nm | 5nm | 90nm | (44%:44%:12%) 30nm | 10nm | (50%:50%) 30nm | |
| E49 | --- | SpA1 | HATCN | SpMA1 | 7g:TEG2 | ST2 | ST2:LiQ | --- |
| | | 70nm | 5nm | 90nm | (80%:20%) 30nm | 10nm | (50%:50%) 30nm | |
| E50 | HATCN | --- | --- | SpMA2 | L2:3a:TEY1 | --- | ST1 | LiQ |
| | 5nm | | | 90m | (45%:45%:10%) 30nm | | 40nm | 3nm |
| E51 | HATCN | --- | --- | SpMA5 | L2:3a:TEY1 | --- | ST1 | LiQ |
| | 5nm | | | 90m | (45%:45%:10%) 30nm | | 40nm | 3nm |
| E52 | HATCN | --- | --- | SpMA4 | L2:3a:TEY1 | --- | ST1 | LiQ |
| | 5nm | | | 90m | (45%:45%:10%) 30nm | | 40nm | 3nm |
| E53 | HATCN | --- | --- | SpMA6 | L2:3a:TEY1 | --- | ST1 | LiQ |
| | 5nm | | | 90m | (45%:45%:10%) 30nm | | 40nm | 3nm |
| E54 | HATCN | --- | --- | SpMA2 | 3a:7e:TEY1 | --- | ST1 | LiQ |
| | 5nm | | | 90m | (45%:45%:10%) 30nm | | 40nm | 3nm |
| E55 | HATCN | SpMA1 | --- | SpMA2 | IC2:9a:TEY1 | --- | ST1 | LiQ |
| | 5nm | 70nm | | 10m | (55%:35%:10%) 25nm | | 45nm | 3nm |
| E56 | SpMA1:F4T | --- | SpMA1 | SpMA3 | 3a:L1:TEG2 | ST2 | ST2:LiQ | LiQ |
| | (95%:5%) 20nm | | 200nm | 20nm | (53%:30%:17%) 30nm | 10nm | (50%:50%) 30nm | 1nm |
| E57 | SpMA1:F4T | --- | SpMA1 | SpMA3 | 3a:L1:TEG2 | ST2 | ST2:LiQ | LiQ |
| | (95%:5%) 20nm | | 200nm | 20nm | (38%:50%:12%) 30nm | 10nm | (50%:50%) 30nm | 1nm |
| E58 | SpMA1:F4T | --- | SpMA1 | SpMA3 | 3a:L1:TEG2 | ST2 | ST2:LiQ | LiQ |
| | (95%:5%) 20nm | | 200nm | 20nm | (33%:50%:17%) 30nm | 10nm | (50%:50%) 30nm | 1nm |
| E59 | SpMA1:F4T | --- | SpMA1 | SpMA3 | 7g:L1:TEG2 | ST2 | ST2:LiQ | LiQ |
| | (95%:5%) 20nm | | 200nm | 20nm | (53%:30%:17%) 30nm | 10nm | (50%:50%) 30nm | 1nm |
| E60 | SpMA1:F4T | --- | SpMA1 | SpMA3 | 7g:L1:TEG2 | ST2 | ST2:LiQ | LiQ |
| | (95%:5%) 20nm | | 200nm | 20nm | (38%:50%:12%) 30nm | 10nm | (50%:50%) 30nm | 1nm |
| E61 | SpMA1:F4T | --- | SpMA1 | SpMA3 | L2:9a:TEG2 | ST2 | ST2:LiQ | LiQ |
| | (95%:5%) 20nm | | 200nm | 20nm | (60%:25%:15%) 30nm | 10nm | (50%:50%) 30nm | 1nm |
| E62 | SpMA1:F4T | --- | SpMA1 | SpMA4 | L2:9a:TEG2 | ST2 | ST2:LiQ | LiQ |
| | (95%:5%) 20nm | | 200nm | 20nm | (60%:25%:15%) 30nm | 10nm | (50%:50%) 30nm | 1nm |
| E63 | SpMA1:F4T | --- | SpMA1 | SpMA2 | IC2:9a:TEG2 | --- | ST2:LiQ | --- |
| | (95%:5%) 20nm | | 210nm | 20nm | (57%:28%:15%) 30nm | | (50%:50%) 40nm | |
| E64 | SpMA1:F4T | --- | --- | SpMA1 | 3a:L3:TEG2 | --- | ST2:LiQ | --- |
| | (95%:5%) 20nm | | | 220nm | (35%:45%:20%) | | (50%:50%) 40nm | |
| E65 | SpMA1:F4T | --- | --- | SpMA1 | 3a:L3:TEG2 | --- | ST2:LiQ | --- |
| | (95%:5%) 20nm | | | 220nm | (45%:45%:10%) | | (50%:50%) 40nm | |
| E66 | SpMA1:F4T | --- | --- | SpMA1 | 3a:L3:TEG2 | --- | ST2:LiQ | --- |
| | (95%:5%) 20nm | | | 220nm | (40%:45%:15%) | | (50%:50%) 40nm | |
| E67 | SpMA1:F4T | --- | SpMA1 | SpMA2 | 3a:L3:TEG2 | --- | ST2:LiQ | --- |
| | (95%:5%) 20nm | | 210nm | 10nm | (35%:45%:20%) | | (50%:50%) 40nm | |
| E68 | SpMA1:F4T | --- | SpMA1 | SpMA2 | 3a:L3:TEG2 | --- | ST2:LiQ | --- |
| | (95%:5%) 20nm | | 210nm | 10nm | (40%:45%:15%) | | (50%:50%) 40nm | |
| E69 | SpMA1:F4T | --- | SpMA1 | SpMA2 | L2:3a:TEG2 | ST2 | ST2:LiQ | LiQ |
| | (95%:5%) 20nm | | 200nm | 20nm | (63%:25%:12%) | 10nm | (50%:50%) 30nm | 1nm |
| E70 | SpMA1:F4T | --- | SpMA1 | SpMA2 | L2:3a:TEG2 | ST2 | ST2:LiQ | LiQ |
| | (95%:5%) 20nm | | 200nm | 20nm | (58%:25%:17%) | 10nm | (50%:50%) 30nm | 1nm |
| E71 | SpMA1:F4T | --- | SpMA1 | SpMA2 | L2:3a:TEG2 | ST2 | ST2:LiQ | LiQ |
| | (95%:5%) 20nm | | 200nm | 20nm | (38%:45%:17%) | 10nm | (50%:50%) 30nm | 1nm |
| E72 | SpMA1:F4T | --- | SpMA1 | SpMA2 | L2:3a:TEG2 | ST2 | ST2:LiQ | LiQ |
| | (95%:5%) 20nm | | 200nm | 20nm | (18%:65%:17%) | 10nm | (50%:50%) 30nm | 1nm |
| E73 | SpMA1:F4T | --- | SpMA1 | SpMA4 | L2:3a:TEG2 | ST2 | ST2:LiQ | LiQ |
| | (95%:5%) 20nm | | 200nm | 20nm | (63%:25%:12%) | 10nm | (50%:50%) 30nm | 1nm |
| E74 | SpMA1:F4T | --- | SpMA1 | SpMA4 | L2:3a:TEG2 | ST2 | ST2:LiQ | LiQ |
| | (95%:5%) 20nm | | 200nm | 20nm | (43%:45%:12%) | 10nm | (50%:50%) 30nm | 1nm |
| E75 | SpMA1:F4T | --- | SpMA1 | SpMA4 | L2:3a:TEG2 | ST2 | ST2:LiQ | LiQ |
| | (95%:5%) 20nm | | 200nm | 20nm | (58%:25%:17%) | 10nm | (50%:50%) 30nm | 1nm |
| E76 | SpMA1:F4T | --- | SpMA1 | SpMA4 | L2:3a:TEG2 | ST2 | ST2:LiQ | LiQ |
| | (95%:5%) 20nm | | 200nm | 20nm | (38%:45%:17%) | 10nm | (50%:50%) 30nm | 1nm |
| E77 | SpMA1:F4T | --- | SpMA1 | SpMA4 | L2:3a:TEG2 | ST2 | ST2:LiQ | LiQ |
| | (95%:5%) 20nm | | 200nm | 20nm | (18%:65%:17%) | 10nm | (50%:50%) 30nm | 1nm |

**Tabelle 2: Daten der OLEDs**

| Bsp. | U1000 (V) | SE1000 (cd/A) | LE1000 (Im/VW) | EQE 1000 | CIE x/y bei 1000 cd/m² | L0;j0 | L1 % | LD (h) |
|---|---|---|---|---|---|---|---|---|
| V1 | 3.6 | 56 | 49 | 15.5% | 0.33/0.62 | 20mA/cm² | 80 | 155 |
| V2 | 3.3 | 61 | 58 | 15.8% | 0.34/0.62 | 40mA/cm² | 80 | 100 |
| V3 | 3.3 | 57 | 54 | 14.9% | 0.34/0.62 | 40mA/cm² | 80 | 190 |
| V4 | 3.3 | 60 | 56 | 16.6% | 0.33/0.62 | 20mA/cm² | 80 | 130 |
| V5 | 5.3 | 9.5 | 5.7 | 10.3% | 0.67/0.33 | 4000cd/m² | 80 | 335 |
| V6 | 3.4 | 61 | 56 | 17.3% | 0.34/0.62 | 20mA/cm² | 80 | 160 |
| V7 | 3.3 | 56 | 54 | 15.7% | 0.33/0.62 | 20mA/cm² | 80 | 310 |
| E1 | 3.3 | 61 | 58 | 16.8% | 0.35/0.61 | 20mA/cm² | 80 | 210 |
| E2 | 3.2 | 63 | 63 | 16.4% | 0.34/0.62 | 40mA/cm² | 80 | 205 |
| E3 | 3.3 | 64 | 61 | 16.6% | 0.34/0.62 | 40mA/cm² | 80 | 210 |
| E4 | 3.3 | 64 | 61 | 16.6% | 0.34/0.62 | 20mA/cm² | 80 | 230 |
| E5 | 3.4 | 58 | 54 | 16.2% | 0.33/0.62 | 20mA/cm² | 80 | 185 |
| E6 | 3.2 | 61 | 59 | 16.9% | 0.35/0.62 | 20mA/cm² | 80 | 200 |
| E7 | 3.4 | 68 | 63 | 17.6% | 0.34/0.62 | 20mA/cm² | 80 | 175 |
| E8 | 3.3 | 54 | 51 | 14.9% | 0.35/0.61 | 20mA/cm² | 80 | 220 |
| E9 | 3.5 | 56 | 51 | 15.5% | 0.33/0.62 | 20mA/cm² | 80 | 180 |
| E10 | 3.2 | 65 | 64 | 16.9% | 0.34/0.62 | 20mA/cm² | 80 | 220 |
| E11 | 3.5 | 55 | 49 | 15.2% | 0.33/0.61 | 20mA/cm² | 80 | 190 |
| E12 | 3.1 | 59 | 60 | 16.3% | 0.34/0.62 | 20mA/cm² | 80 | 205 |
| E13 | 3.3 | 67 | 65 | 18.0% | 0.34/0.62 | 20mA/cm² | 80 | 140 |
| E14 | 3.1 | 68 | 68 | 18.2% | 0.34/0.62 | 20mA/cm² | 80 | 245 |
| E15 | 3.4 | 57 | 54 | 16.2% | 0.33/0.62 | 20mA/cm² | 80 | 155 |
| E16 | 3.2 | 66 | 64 | 17.8% | 0.33/0.62 | 20mA/cm² | 80 | 270 |
| E17 | 3.1 | 58 | 59 | 15.8% | 0.34/0.62 | 20mA/cm² | 80 | 325 |
| E18 | 3.5 | 61 | 55 | 16.5% | 0.34/0.62 | 20mA/cm² | 80 | 340 |
| E19 | 3.3 | 63 | 68 | 16.6% | 0.34/0.62 | 20mA/cm² | 80 | 290 |
| E20 | 3.2 | 65 | 64 | 18.3% | 0.33/0.62 | 20mA/cm² | 80 | 190 |
| E21 | 3.2 | 57 | 55 | 16.0% | 0.34/0.63 | 20mA/cm² | 80 | 425 |
| E22 | 3.1 | 66 | 68 | 18.5% | 0.33/0.62 | 20mA/cm² | 80 | 170 |
| E23 | 3.8 | 54 | 45 | 15.4% | 0.34/0.62 | 20mA/cm² | 80 | 190 |
| E24 | 3.3 | 62 | 59 | 17.5% | 0.34/0.62 | 20mA/cm² | 80 | 205 |
| E25 | 4.4 | 8.7 | 6.2 | 6.6% | 0.14/0.16 | 6000cd/m² | 70 | 185 |
| E26 | 3.2 | 58 | 58 | 15.7% | 0.34/0.62 | 40mA/cm² | 80 | 190 |
| E27 | 3.1 | 59 | 59 | 15.9% | 0.34/0.62 | 40mA/cm² | 80 | 180 |
| E28 | 3.2 | 59 | 57 | 16.3% | 0.33/0.62 | 40mA/cm² | 80 | 205 |
| E29 | 3.2 | 54 | 54 | 15.5% | 0.34/0.62 | 40mA/cm² | 80 | 165 |
| E30 | 4.8 | 9.8 | 6.4 | 10.6% | 0.67/0.33 | 4000cd/m² | 80 | 360 |
| E31 | 4.6 | 11.0 | 7.5 | 11.9% | 0.67/0.33 | 4000cd/m² | 80 | 380 |
| E32 | 5.0 | 9.4 | 5.9 | 10.2% | 0.67/0.33 | 4000cd/m² | 80 | 440 |
| E33 | 4.6 | 10.4 | 7.1 | 11.3% | 0.67/0.33 | 4000cd/m² | 80 | 480 |
| E34 | 4.7 | 11.4 | 7.7 | 12.3% | 0.67/0.33 | 4000cd/m² | 80 | 515 |
| E35 | 3.2 | 55 | 54 | 15.3% | 0.34/0.62 | 20mA/cm² | 80 | 165 |
| E36 | 3.0 | 63 | 65 | 17.5% | 0.33/0.61 | 20mA/cm² | 80 | 200 |
| E37 | 3.4 | 68 | 64 | 18.9% | 0.34/0.62 | 20mA/cm² | 80 | 140 |
| E38 | 3.2 | 54 | 52 | 15.0% | 0.34/0.62 | 20mA/cm² | 80 | 150 |
| E39 | 3.3 | 58 | 55 | 16.1% | 0.33/0.62 | 20mA/cm² | 80 | 330 |
| E40 | 3.2 | 62 | 61 | 17.2% | 0.34/0.63 | 20mA/cm² | 80 | 390 |
| E41 | 3.4 | 65 | 62 | 18.4% | 0.33/0.62 | 20mA/cm² | 80 | 410 |
| E42 | 4.4 | 11.6 | 8.2 | 12.5% | 0.67/0.33 | 4000cd/m² | 80 | 540 |
| E43 | 3.0 | 58 | 61 | 15.7% | 0.34/0.62 | 40mA/cm² | 80 | 200 |
| E44 | 3.3 | 56 | 54 | 15.3% | 0.34/0.62 | 40mA/cm² | 80 | 175 |
| E45 | 3.2 | 58 | 57 | 16.1% | 0.34/0.62 | 40mA/cm² | 80 | 185 |
| E46 | 3.3 | 60 | 57 | 16.3% | 0.34/0.62 | 40mA/cm² | 80 | 230 |
| E47 | 3.1 | 64 | 64 | 17.2% | 0.35/0.62 | 40mA/cm² | 80 | 270 |
| E48 | 3.2 | 60 | 62 | 16.5% | 0.34/0.62 | 40mA/cm² | 80 | 245 |
| E49 | 3.2 | 62 | 61 | 17.0% | 0.34/0.62 | 40mA/cm² | 80 | 200 |
| E50 | 2.9 | 87 | 95 | 25.6% | 0.44/0.55 | 50mA/cm² | 90 | 345 |
| E51 | 2.8 | 86 | 96 | 25.5% | 0.45/0.55 | 50mA/cm² | 90 | 480 |
| E52 | 2.9 | 88 | 94 | 26.0% | 0.44/0.55 | 50mA/cm² | 90 | 210 |
| E53 | 2.9 | 85 | 92 | 25.1% | 0.44/0.55 | 50mA/cm² | 90 | 400 |
| E54 | 3.2 | 85 | 83 | 25.0% | 0.44/0.55 | 50mA/cm² | 90 | 185 |
| E55 | 2.9 | 87 | 95 | 25.4% | 0.45/0.55 | 50mA/cm² | 90 | 110 |
| E56 | 3.3 | 71 | 68 | 18.9% | 0.35/0.62 | 40mA/cm² | 80 | 235 |
| E57 | 3.4 | 70 | 65 | 18.8% | 0.35/0.62 | 40mA/cm² | 80 | 170 |
| E58 | 3.3 | 71 | 68 | 18.9% | 0.34/0.63 | 40mA/cm² | 80 | 270 |
| E59 | 3.2 | 74 | 72 | 19.7% | 0.34/0.63 | 40mA/cm² | 80 | 210 |
| E60 | 3.3 | 74 | 71 | 19.7% | 0.34/0.63 | 40mA/cm² | 80 | 165 |
| E61 | 3.4 | 70 | 64 | 18.6% | 0.34/0.62 | 40mA/cm² | 80 | 220 |
| E62 | 3.1 | 71 | 72 | 19.0% | 0.34/0.62 | 40mA/cm² | 80 | 170 |
| E63 | 3.0 | 76 | 79 | 20.3% | 0.34/0.63 | 40mA/cm² | 80 | 180 |
| E64 | 3.1 | 52 | 53 | 14.4% | 0.31/0.64 | 20mA/cm² | 90 | 395 |
| E65 | 3.2 | 53 | 52 | 14.4% | 0.31/0.64 | 20mA/cm² | 90 | 420 |
| E66 | 3.1 | 58 | 60 | 16.0% | 0.31/0.64 | 20mA/cm² | 90 | 460 |
| E67 | 3.3 | 58 | 56 | 15.7% | 0.31/0.64 | 20mA/cm² | 90 | 365 |
| E68 | 3.2 | 65 | 64 | 17.9% | 0.31/0.64 | 20mA/cm² | 90 | 300 |
| E69 | 3.5 | 69 | 63 | 18.4% | 0.35/0.62 | 40mA/cm² | 80 | 235 |
| E70 | 3.4 | 65 | 60 | 17.3% | 0.35/0.62 | 40mA/cm² | 80 | 405 |
| E71 | 3.3 | 68 | 64 | 18.2% | 0.35/0.62 | 40mA/cm² | 80 | 320 |
| E72 | 3.3 | 71 | 69 | 19.0% | 0.35/0.62 | 40mA/cm² | 80 | 220 |
| E73 | 3.2 | 67 | 67 | 18.0% | 0.35/0.62 | 40mA/cm² | 80 | 325 |
| E74 | 3.1 | 70 | 71 | 18.8% | 0.35/0.61 | 40mA/cm² | 80 | 245 |
| E75 | 3.1 | 67 | 69 | 18.0% | 0.35/0.62 | 40mA/cm² | 80 | 425 |
| E76 | 3.2 | 67 | 67 | 18.1% | 0.35/0.61 | 40mA/cm² | 80 | 315 |
| E77 | 3.0 | 69 | 72 | 18.5% | 0.35/0.61 | 40mA/cm² | 80 | 220 |

**Tabelle 3: Strukturformeln der Materialien für die OLEDs**

| | |
|---|---|
| 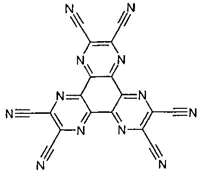 | 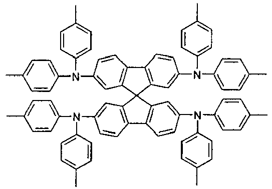 |
| HATCN | SpA1 |
| 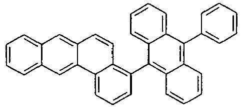 | 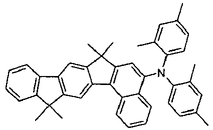 |
| M1 | D1 |
| 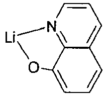 | 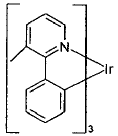 |
| LiQ | TEG1 |
| 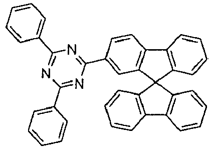 | 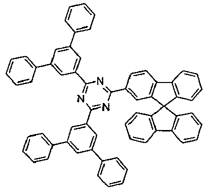 |
| ST1 | ST2 |
| 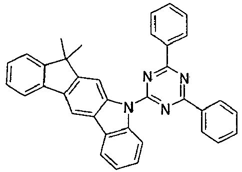 | 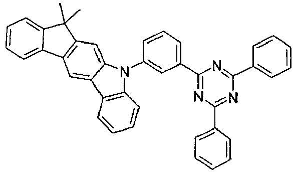 |
| IC1 | IC2 |
| 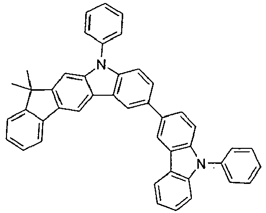 | 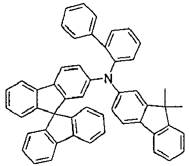 |
| IC3 | SpMA1 |
| 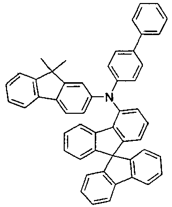 | 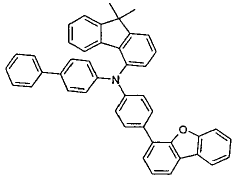 |
| SpMA2 | SpMA4 |
| 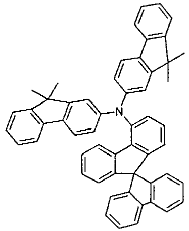 | 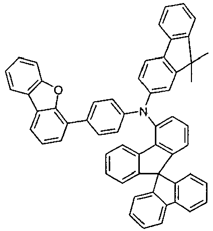 |
| SpMA5 | SpMA6 |
| 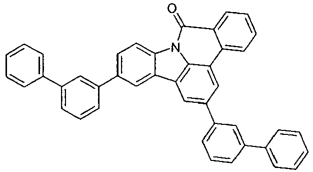 | 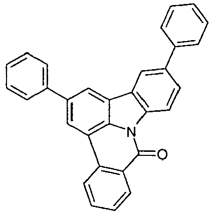 |
| L2 | L1 |
| 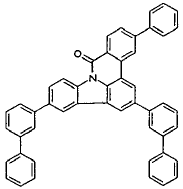 | 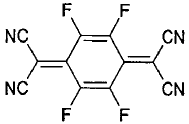 |
| L3 | F4T |
| 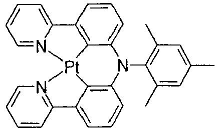 | 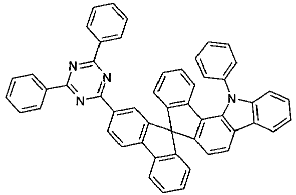 |
| TER1 | StdT1 |
| 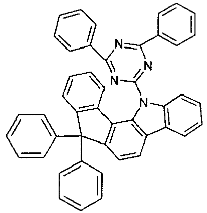 | 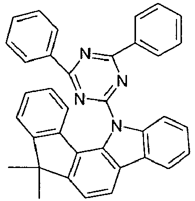 |
| StdT2 | StdT3 |
| 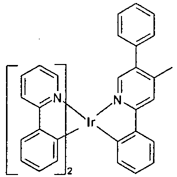 | 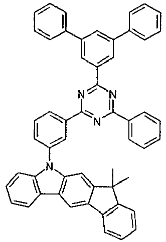 |
| TEG2 | IC5 |
| 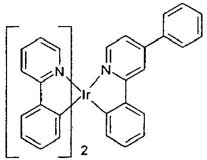 | 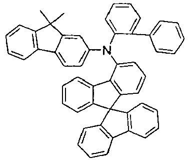 |
| TEY1 | SpMA3 |
| 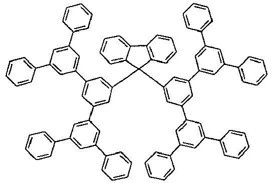 | 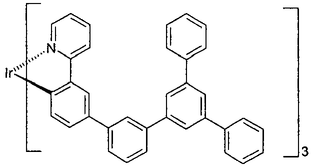 |
| WB1 | TEG3 |
| 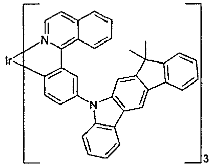 | 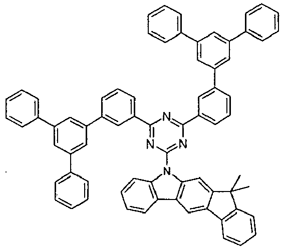 |
| TER2 | IC4 |

### Beispiel 17: OLEDs mit aus Lösung prozessierter Lochtransport- und Emissionsschicht

Viele der erfindungsgemäßen Materialien können auch aus Lösung verarbeitet werden und führen gegenüber vakuumprozessierten OLEDs zu wesentlich einfacher herstellbaren OLEDs mit dennoch guten Eigenschaften. Insbesondere haben erfindungsgemäße Matrixmaterialien positiven Einfluss auf Betriebslebensdauer und Effizienz von Bauteilen mit lösungsprozessierter Emissionsschicht. Die Herstellung vollständig lösungsbasierter OLEDs ist in der Literatur bereits vielfach beschrieben, z. B. in WO 2004/037887.

In den im Folgenden diskutierten Beispielen werden lösungsbasiert und vakuumbasiert aufgebrachte Schichten innerhalb einer OLED kombiniert, so dass die Prozessierung bis einschließlich zur Emissionsschicht aus Lösung und in den darauffolgenden Schichten durch thermisches Verdampfen im Vakuum erfolgt. Die vorbeschriebenen allgemeinen Verfahren werden dafür wie folgt auf die hier beschriebenen Gegebenheiten (Schichtdickenvariation, Materialien) angepasst und kombiniert.

Gereinigte Glasplättchen (Reinigung in Miele Laborspülmaschine, Reiniger Merck Extran), die mit strukturiertem ITO (Indium Zinn Oxid) der Dicke 50 nm beschichtet sind, werden zur verbesserten Prozessierung mit 20 nm (für grün emittierende OLEDs) bzw. 80 nm (für rot emittierende OLEDs) PEDOT:PSS beschichtet (Poly(3,4-ethylendioxythiophen)poly(styrol-sulfonat), bezogen als CLEVIOS™ P VP Al 4083 von Heraeus Precious Metals GmbH Deutschland, aus wässriger Lösung aufgeschleudert). Diese Substrate werden anschließend bei 180 °C für 10 min ausgeheizt.

Auf diese Substrate wird eine Lochtransportschicht der Dicke 20 nm aufgebracht. Sie besteht aus einem Polymer der folgenden Strukturformel, 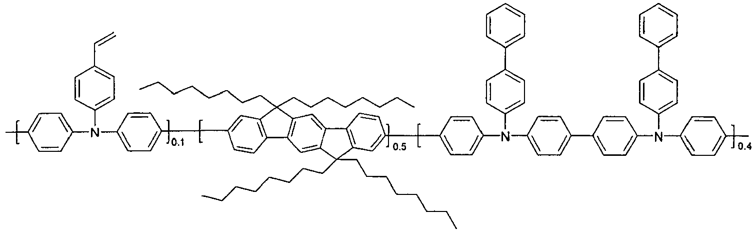 das gemäß WO 2010/097155 synthetisiert wurde. Das Material wird in Toluol gelöst. Der Feststoffgehalt der Lösung liegt bei 5 g/l. Mittels Spincoating wird hieraus in einer Stickstoffatmosphäre eine 20 nm dicke Schicht aufgebracht. Anschließend wird die Probe für 60 Minuten bei 180 °C in einer Stickstoffatmosphäre ausgeheizt.

Im Anschluss wird die Emissionsschicht aufgebracht. Diese setzt sich immer aus mindestens einem Matrixmaterial (Hostmaterial, Wirtsmaterial) und einem emittierenden Dotierstoff (Dotand, Emitter) zusammen. Weiterhin aufreten können Mischungen aus mehreren Matrixmaterialien sowie Co-Dotanden. Eine Angabe wie Matrix(92%):Dotand(8%) bedeutet, dass das Material Matrix in einem Gewichtsanteil von 92% und Dotand in einem Gewichtsanteil von 8% in der Lösung vorliegt, aus der die Emissionsschicht hergestellt wird. Eine entsprechende Feststoffmischung für die Emissionsschicht wird in Toluol gelöst. Der Feststoffgehalt liegt bei 18 g/l. Die Emissionsschicht wird in einer Stickstoffatmosphäre aufgeschleudert und 10 Minuten bei 180°C in der Stickstoffatmosphäre ausgeheizt.

Im Anschluss werden die Proben ohne Kontakt zu Luft in eine Vakuumkammer eingebracht und zwei weitere Schichten durch Verdampfen aufgebracht. Besteht eine solche Schicht aus mehreren Materialien, gilt für die Mischungsverhältnisse der Einzelkomponenten die weiter oben beschriebene Nomenklatur.

Die OLEDs werden wie oben beschrieben charakterisiert. Zur Lebensdauermessung werden die OLEDs mit einem konstanten Strom betrieben, der so eingestellt wird, dass eine gewisse Anfangsleuchtdichte erreicht wird. Die Lebensdauer LD80@8000 ist definiert als die Zeit, bis die Leuchtdichte von ihrem Anfangswert von 8000 cd/m² auf 80 %, also auf 6400 cd/m², abgefallen ist. Entsprechend sind Lebensdauerwerte bei anderen Anfangshelligkeiten oder anderen prozentualen Endleuchtdichten definiert.

**Vergleichsbeispiel LV1:** Gemäß Stand der Technik wird für die Emissionsschicht eine Feststoffmischung IC4(40%):WB1(40%):TEG3(20%) verwendet. Hieraus wird wie oben beschrieben eine 60 nm dicke Emissionsschicht hergestellt. Anschließend wird eine 10 nm dicke Schicht des Materials ST2 und danach eine 40 nm dicke Schicht ST2:LiQ(50%:50%) durch thermisches Verdampfen im Vakuum aufgebracht. Anschließend wird als Kathode eine 100 nm dicke Aluminiumschicht durch Verdampfen im Vakuum aufgebracht. Die OLED emittiert grün und man erhält U1000 = 4. 6V, EQE1000 = 17.8 %, LD80@10000 = 115 h, LD90@10000 = 28 h.

**Erfindungsgemäßes Beispiel LE1:** Die OLED entspricht dem Beispiel LV1, mit dem Unterschied, dass statt der Mischung IC4(40%):WB1(40%):TEG3(20%) die Mischung 3q(40%):WB1(40%):TEG3(20%) verwendet wird. Die OLED emittiert grün, und man erhält U1000 = 4.6 V, EQE1000 = 18.3 %, LD80@10000 = 120 h, LD90@10000 = 37 h.

**Erfindungsgemäßes Beispiel LE2:** Die OLED entspricht dem Beispiel LV1, mit dem Unterschied, dass statt der Mischung IC4(40%):WB1(40%):TEG3(20%) die Mischung 3p(40%):WB1(40%):TEG3(20%) verwendet wird. Die OLED emittiert grün, und man erhält U1000 = 4.5 V, EQE1000 = 18.4 %, LD80@10000 = 130 h, LD90@10000 = 38 h.

**Erfindungsgemäßes Beispiel LE3:** Die OLED entspricht dem Beispiel LV1, mit dem Unterschied, dass statt der Mischung IC4(40%):WB1(40%):TEG3(20%) die Mischung 3a(40%):WB1(40%):TEG3(20%) verwendet wird. Die OLED emittiert grün, und man erhält U1000 = 4.4 V, EQE1000 = 18.8 %, LD80@10000 = 240 h, LD90@10000 = 75 h.

**Vergleichsbeispiel LV2:** Die OLED entspricht dem Beispiel LV1, mit dem Unterschied, dass statt der Mischung IC4(40%):WB1(40%):TEG3(20%) die Mischung IC5(40%):WB1(30%):TEG3(30%) verwendet wird. Die OLED emittiert grün, und man erhält U1000 = 4.0 V, EQE1000 = 19.7 %, LD80@10000 = 335 h, LD90@10000 = 87 h.

**Erfindungsgemäßes Beispiel LE4:** Die OLED entspricht dem Beispiel LV2, mit dem Unterschied, dass statt der Mischung IC5(40%):WB1(30%):TEG3(30%) die Mischung 3a(40%):WB1(30%):TEG3(30%) verwendet wird. Die OLED emittiert grün, und man erhält U1000 = 4.0 V, EQE1000 = 21.4 %, LD80@10000 = 520 h, LD90@10000 = 195 h.

**Vergleichsbeispiel LV3:** Die OLED entspricht dem Beispiel LV1, mit dem Unterschied, dass statt der Mischung IC4(40%):WB1(40%):TEG3(20%) die Mischung IC5(40%):WB1(24%):TEG3(30%):TER2(6%) verwendet wird. Die OLED emittiert rot, und man erhält U1000 = 6.0 V, EQE1000 = 13.5 %, LD80@8000 = 180 h, LD90@8000 = 48 h.

**Erfindungsgemäßes Beispiel LE5:** Die OLED entspricht dem Beispiel LV3, mit dem Unterschied, dass statt der Mischung IC5(40%):WB1(24%):TEG3(30%):TER2(6%) die Mischung 3a(40%):WB1(24%):TEG3(30%):TER2(6%) verwendet wird. Die OLED emittiert rot, und man erhält U1000 = 5.6 V, EQE1000 = 13.8 %, LD80@8000 = 255 h, LD90@8000 = 82 h.

**Erfindungsgemäßes Beispiel LE6:** Die OLED entspricht dem Beispiel LV3, mit dem Unterschied, dass statt der Mischung IC5(40%):WB1(24%):TEG3(30%):TER2(6%) die Mischung 3p(40%):WB1(24%):TEG3(30%):TER2(6%) verwendet wird. Die OLED emittiert rot, und man erhält U1000 = 6.0 V, EQE1000 = 13.8 %, LD80@8000 = 275 h, LD90@8000 = 81 h.

**Erfindungsgemäßes Beispiel LE7:** Die OLED entspricht dem Beispiel LV3, mit dem Unterschied, dass statt der Mischung IC5(40%):WB1(24%):TEG3(30%):TER2(6%) die Mischung 3q(40%):WB1(24%):TEG3(30%):TER2(6%) verwendet wird. Die OLED emittiert rot, und man erhält U1000 = 6.0 V, EQE1000 = 14.0 %, LD80@8000 = 250 h, LD90@8000 = 68 h.

**Vergleichsbeispiel LV4:** Gemäß Stand der Technik wird für die Emissionsschicht eine Feststoffmischung IC4(40%):WB1(40%):TEG3(20%) verwendet. Hieraus wird wie oben beschrieben eine 60 nm dicke Emissionsschicht hergestellt. Anschließend wird eine 20 nm dicke Schicht M1:D1(95%:5%) und anschließend eine 20 nm dicke Schicht ST2:LiQ(50%:50%) durch thermisches Verdampfen im Vakuum aufgebracht. Anschließend wird als Kathode eine 100 nm dicke Aluminiumschicht durch Verdampfen im Vakuum aufgebracht. Die OLED emittiert grün, und man erhält U1000 = 5.0 V, EQE1000 = 16.7 %, LD80@10000 = 33 h, LD90@10000 = 8 h.

**Vergleichsbeispiel LV5:** Die OLED entspricht dem Beispiel LV4, mit dem Unterschied, dass statt der Mischung IC4(40%):WB1(40%):TEG3(20%) die Mischung IC5(40%):WB1(40%):TEG3(20%) verwendet wird. Die OLED emittiert grün, und man erhält U1000 = 4.3 V, EQE1000 = 15.0 %, LD80@10000 = 29 h, LD90@10000 = 8 h.

**Erfindungsgemäßes Beispiel LE8:** Die Die OLED entspricht dem Beispiel LV4, mit dem Unterschied, dass statt der Mischung IC4(40%):WB1(40%):TEG3(20%) die Mischung 3a(40%):WB1(40%):TEG3(20%) verwendet wird. Die OLED emittiert grün, und man erhält U1000 = 4.3 V, EQE1000 = 16.9 %, LD80@10000 = 88 h, LD90@10000 = 25 h.

Wie man durch Vergleich der Beispiele LV1 mit LE1-LE3, LV2 mit LE4, LV3 mit LE5-LE7 sowie LV4 und LV5 mit LE8 sieht, erhält man mit Mischungen enthaltend erfindungsgemäße Verbindungen Verbesserungen in allen Parametern, insbesondere eine sehr deutliche Steigerung der für Displayanwendungen wichtigen LD90.

## Patentansprüche

1. Verbindung gemäß Formel (1) bzw. Formel (1A), 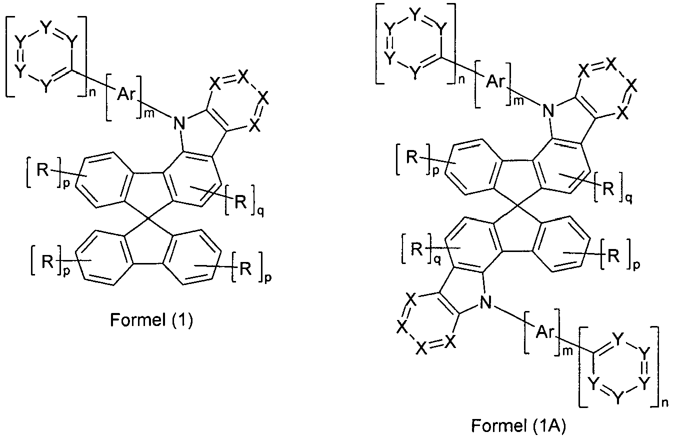 wobei für die verwendeten Symbole und Indizes gilt:
Y ist bei jedem Auftreten gleich oder verschieden CR¹ oder N, mit der Maßgabe, dass mindestens eine Gruppe Y für N steht;
X ist bei jedem Auftreten gleich oder verschieden CR¹ oder N; oder zwei benachbarte X stehen für S, O oder NR¹, so dass ein Fünfring entsteht; oder zwei benachbarte X stehen für eine Gruppe der folgenden Formel (2), (3) oder (4), 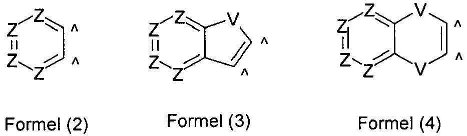 wobei ^ die entsprechenden benachbarten Gruppen X in der Formel (1) andeutet;
V ist bei jedem Auftreten gleich oder verschieden C(R¹)₂, NR¹, O, S, BR¹, Si(R¹)₂ oder C=O;
Z ist bei jedem Auftreten gleich oder verschieden CR¹ oder N;
Ar ist bei jedem Auftreten gleich oder verschieden ein aromatisches oder heteroaromatisches Ringsystem mit 5 bis 40 aromatischen Ringatomen, das mit einem oder mehreren Resten R¹ substituiert sein kann;
R ist bei jedem Auftreten gleich oder verschieden ausgewählt aus der Gruppe bestehend aus H, D, F, Cl, Br, I, CN, N(Ar¹)₂, einer gerad-kettigen Alkylgruppe mit 1 bis 40 C-Atomen oder einer verzweigten oder cyclischen Alkylgruppe mit 3 bis 40 C-Atomen, die jeweils mit einem oder mehreren Resten R² substituiert sein kann, wobei eine oder mehrere nicht-benachbarte CH₂-Gruppen durch R²C=CR², C≡C oder O ersetzt sein können und wobei ein oder mehrere H-Atome durch D oder F ersetzt sein können, oder einem aromatischen Ring-system mit 6 bis 60 aromatischen Ringatomen, das mit einem oder mehreren Resten R² substituiert sein kann, wobei das aromatische Ringsystem keine Heteroarylgruppen enthält; dabei können zwei benachbarte Substituenten R ein monocyclisches oder poly-cyclisches, aliphatisches oder aromatisches Ringsystem bilden, das mit einem oder mehreren Resten R² substituiert sein kann;
R¹ ist bei jedem Auftreten gleich oder verschieden ausgewählt aus der Gruppe bestehend aus H, D, F, Cl, Br, I, CN, NO₂ N(Ar¹)₂, N(R²)₂, C(=O)Ar¹, C(=O)R², P(=O)(Ar¹)₂, P(Ar¹)₂, B(Ar¹)₂, Si(Ar¹)₃, Si(R²)₃, einer geradkettigen Alkyl-, Alkoxy- oder Thioalkylgruppe mit 1 bis 40 C-Atomen oder einer verzweigten oder cyclischen Alkyl-, Alkoxy-oder Thioalkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C=C, Si(R²)₂, C=O, C=S, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R² substituiert sein kann, einer Aryloxy- oder Heteroaryloxygruppe mit 5 bis 60 aromatischen Ringatomen, die mit einem oder mehreren Resten R² substituiert sein kann; dabei können optional zwei benachbarte Substituenten R¹ ein monocyclisches oder polycyclisches, aliphatisches, aromatisches oder heteroaromatisches Ringsystem bilden, das mit einem oder mehreren Resten R² substituiert sein kann;
Ar¹ ist bei jedem Auftreten gleich oder verschieden ein aromatisches oder heteroaromatisches Ringsystem mit 5-30 aromatischen Ringatomen, das mit einem oder mehreren nicht-aromatischen Resten R² substituiert sein kann; dabei können zwei Reste Ar¹, welche an dasselbe N-Atom oder P-Atom binden, auch durch eine Einfachbindung oder eine Brücke, ausgewählt aus N(R²), C(R²)₂, O oder S, miteinander verbrückt sein;
R² ist bei jedem Auftreten gleich oder verschieden ausgewählt aus der Gruppe bestehend aus H, D, F, CN, einem aliphatischen Kohlenwasserstoffrest mit 1 bis 20 C-Atomen, oder einem aromatischen oder heteroaromatischen Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R² miteinander ein mono- oder polycyclisches, aliphatisches Ringsystem bilden können;
m, n ist bei jedem Auftreten gleich oder verschieden 0 oder 1 mit der Maßgabe, dass m + n ≥ 1;
p ist bei jedem Auftreten gleich oder verschieden 0, 1, 2, 3 oder 4;
q ist 0, 1 oder 2.

2. Verbindung nach Anspruch 1, **dadurch gekennzeichnet,dass** X gleich oder verschieden bei jedem Auftreten für CR¹ oder N steht, wobei maximal eine Gruppe X pro Cyclus für N steht; oder zwei benachbarte Gruppen X stehen für eine Gruppe der Formel (2) oder (3), wobei Z gleich oder verschieden bei jedem Auftreten für CR¹ steht und V gleich oder verschieden bei jedem Auftreten für NR¹, C(R¹)₂, O oder S steht.

3. Verbindung nach Anspruch 1 oder 2, ausgewählt aus den Verbindungen der Formeln (5) bis (12), 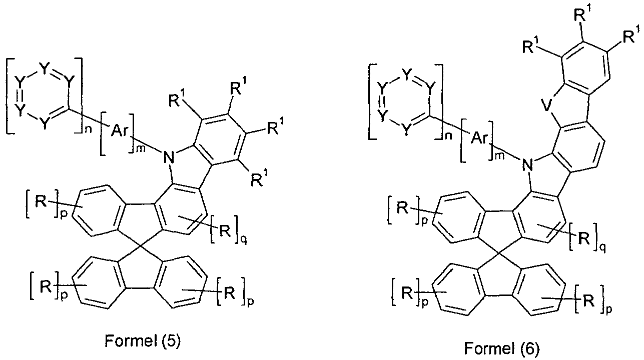 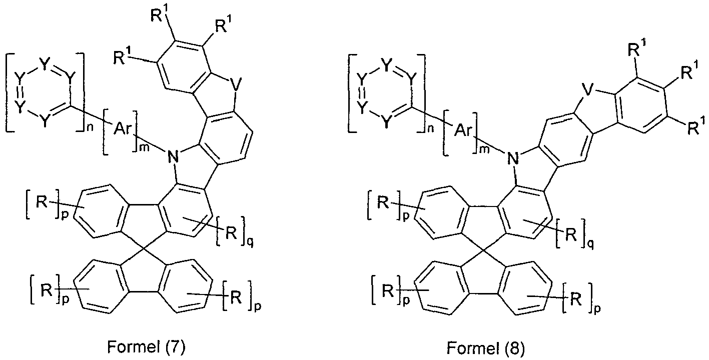 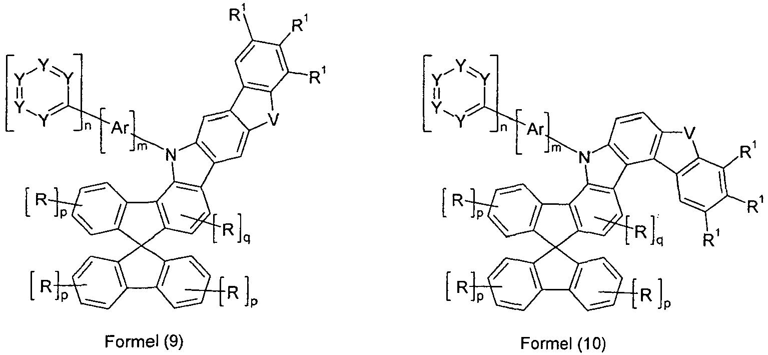 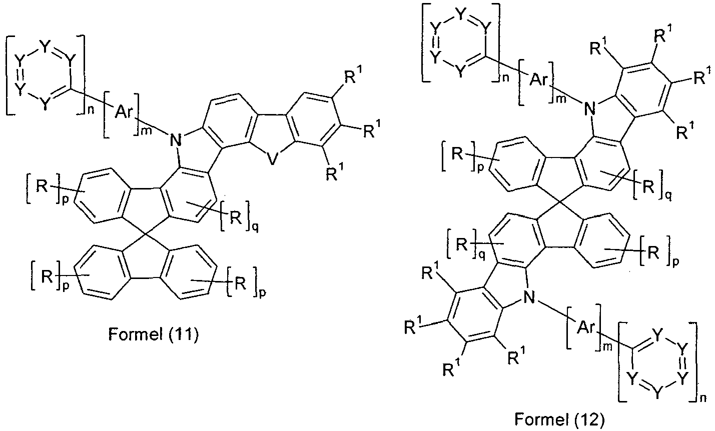 wobei die verwendeten Symbole und Indizes die in Anspruch 1 genannten Bedeutungen aufweisen und V bevorzugt für NR¹, C(R¹)₂, O oder S steht.

4. Verbindung nach einem oder mehreren der Ansprüche 1 bis 3, ausgewählt aus den Strukturen der Formeln (5a) bis (12a), 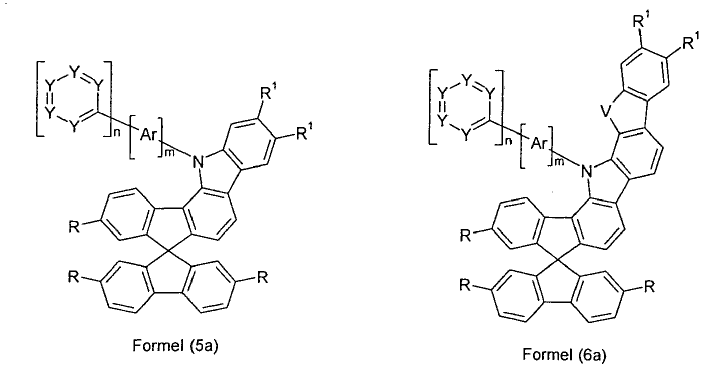 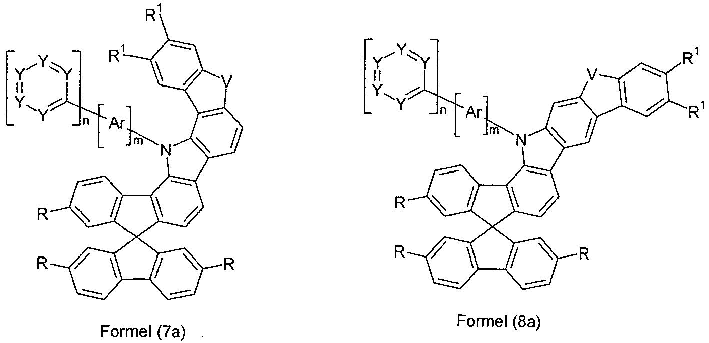 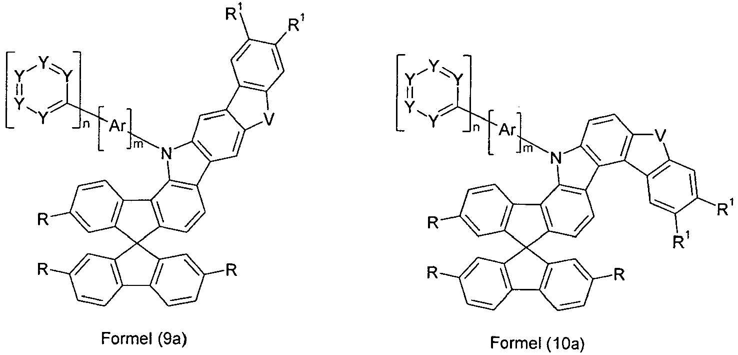 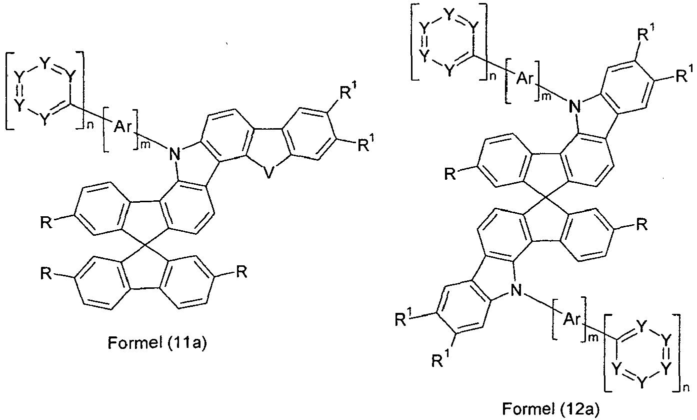 wobei die verwendeten Symbole und Indizes die in Anspruch 1 genannten Bedeutungen aufweisen.

5. Verbindung nach einem oder mehreren der Ansprüche 1 bis 4, **dadurch gekennzeichnet, dass** R gleich oder verschieden bei jedem Auftreten ausgewählt ist aus der Gruppe bestehend aus H, F, CN, N(Ar¹)₂, einer geradkettigen Alkylgruppe mit 1 bis 10 C-Atomen oder einer verzweigten oder cyclischen Alkylgruppe mit 3 bis 10 C-Atomen oder einem aromatischen Ringsystem mit 6 bis 30 aromatischen Ringatomen, das mit einem oder mehreren nicht-aromatischen Resten R² substituiert sein kann,
und dass R¹ gleich oder verschieden bei jedem Auftreten ausgewählt ist aus der Gruppe bestehend aus H, D, F, Br, CN, N(Ar¹)₂, C(=O)Ar¹, P(=O)(Ar¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenyl- oder Alkinylgruppe mit 2 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R² substituiert sein kann.

6. Verbindung nach einem oder mehreren der Ansprüche 1 bis 5, **dadurch gekennzeichnet, dass** Ar ausgewählt ist aus aromatischen oder heteroaromatischen Ringsystemen mit 5 bis 24 aromatischen Ringatomen, welche jeweils durch einen oder mehrere Reste R¹ substituiert sein können, bevorzugt Benzol, ortho-, meta- oder para-Biphenyl, ortho-, meta-, para- oder verzweigtem Terphenyl, ortho-, meta-, para- oder verzweigtem Quaterphenyl, 1-, 2- oder 3-Fluorenyl, 1-, 2-, 3- oder 4-Spirobifluorenyl, 1- oder 2-Naphthyl, Pyrrol, Furan, Thiophen, Indol, Benzofuran, Benzothiophen, 1-, 2- oder 3-Carbazol, 1-, 2- oder 3-Dibenzofuran, 1-, 2- oder 3-Dibenzothiophen, Indenocarbazol, Indolocarbazol, 2-, 3- oder 4-Pyridin, 2-, 4- oder 5-Pyrimidin, Pyrazin, Pyridazin, Triazin, Anthracen, Phenanthren, Triphenylen, Pyren, Benzanthracen oder Kombinationen aus zwei oder drei dieser Gruppen, welche jeweils mit einem oder mehreren Resten R¹ substituiert sein können.

7. Verbindung nach einem oder mehreren der Ansprüche 1 bis 6, **dadurch gekennzeichnet, dass** in der Gruppe der folgenden Formel (Het-Ar), 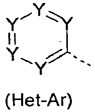 welche für n = 1 in der Verbindung nach einem oder mehreren der Ansprüche 1 bis 6 vorhanden ist und die an Ar bzw. für m = 0 an den Stickstoff gebunden ist, mindestens eine Gruppe Y und maximal drei Gruppen Y für N und die anderen Gruppen Y für CR¹ stehen.

8. Verbindung nach Anspruch 7, **dadurch gekennzeichnet, dass** die Gruppe (Het-Ar) ausgewählt ist aus den Gruppen der Formeln (Het-Ar-1) bis (Het-Ar-10), 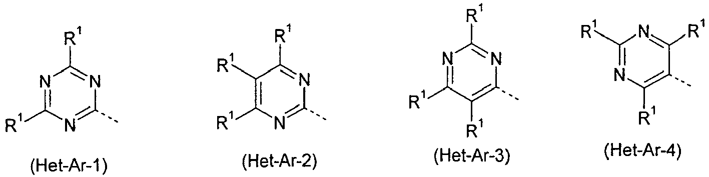 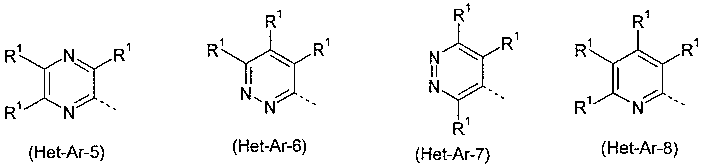 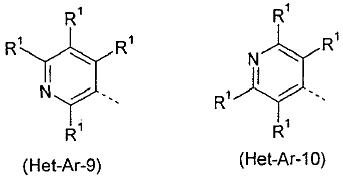 wobei die gestrichelte Bindung die Bindung an Ar bzw. für m = 0 die Bindung an den Stickstoff darstellt und die verwendeten Symbole die in Anspruch 1 genannten Bedeutungen aufweisen.

9. Verbindung nach einem oder mehreren der Ansprüche 1 bis 8, **dadurch gekennzeichnet, dass** sie keine kondensierten Aryl- bzw. Heteroarylgruppen enthält, in denen mehr als zwei Sechsringe direkt aneinander ankondensiert sind.

10. Verfahren zur Herstellung einer Verbindung nach einem oder mehreren der Ansprüche 1 bis 9, umfassend die Reaktionsschritte:
a) Synthese der Grundgerüsts der Verbindung (1) bzw. (1A), die noch keine Gruppe (Het-Ar) und/oder Ar enthält; und
b) Umsetzung der Grundgerüsts aus a) in einer C-C-Kupplung, wie Suzuki-, Negishi-, Yamamoto-, Grignard-Cross- oder Stille-Kupplung, etc., oder C-N-Kupplung, wie Buchwald- oder Ullmann-Kupplung.

11. Oligomer, Polymer oder Dendrimer enthaltend eine oder mehrere der Verbindungen nach einem oder mehreren der Ansprüche 1 bis 9, wobei an einer oder mehreren Positionen statt Substituenten eine oder mehrere Bindungen der Verbindung zum Polymer, Oligomer oder Dendrimer vorhanden sind.

12. Formulierung, enthaltend mindestens eine Verbindung nach einem oder mehreren der Ansprüche 1 bis 9 oder ein Oligomer, Polymer oder Dendrimer nach Anspruch 11 und mindestens ein Lösemittel.

13. Verwendung einer Verbindung nach einem oder mehreren der Ansprüche 1 bis 9 oder eines Oligomers, Polymers oder Dendrimers nach Anspruch 11 in einer elektronischen Vorrichtung.

14. Elektronische Vorrichtung enthaltend mindestens eine Verbindung nach einem oder mehreren der Ansprüche 1 bis 9 oder ein Oligomer, Polymer oder Dendrimer nach Anspruch 11, insbesondere ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, farbstoffsensibilisierten organischen Solarzellen, organischen optischen Detektoren, organischen Photorezeptoren, organischen Feld-Quench-Devices, lichtemittierenden elektrochemischen Zellen, organischen Laserdioden und "organic plasmon emitting devices".

15. Elektronische Vorrichtung nach Anspruch 14, wobei es sich um eine organische Elektrolumineszenzvorrichtung handelt, **dadurch gekennzeichnet, dass** die Verbindung nach einem oder mehreren der Ansprüche 1 bis 9 als Matrixmaterial für phosphoreszierende oder fluoreszierende Emitter und/oder in einer elektronenblockierenden bzw. exzitonenblockierenden Schicht und/oder in einer Lochtransportschicht und/oder in einer Lochblockierschicht und/oder in einer Lochblockier- oder Elektronentransportschicht eingesetzt wird.

## Claims

1. Compound of the formula (1) or formula (1A), 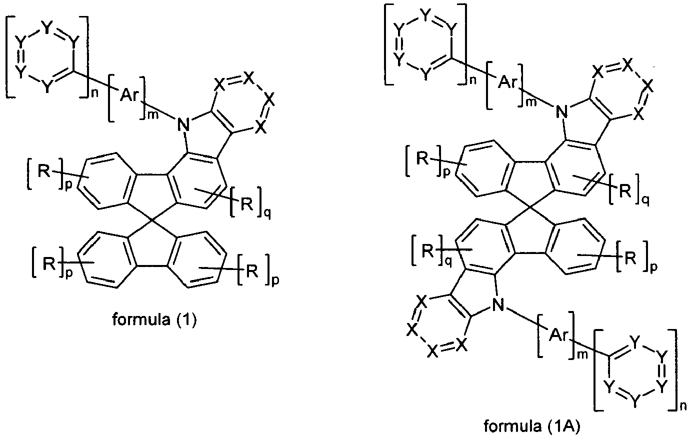 where the following applies to the symbols and indices used:
Y is on each occurrence, identically or differently, CR¹ or N, with the proviso that at least one group Y stands for N;
X is on each occurrence, identically or differently, CR¹ or N; or two adjacent X stand for S, O or NR¹, so that a five-membered ring forms; or two adjacent X stand for a group of the following formula (2), (3) or (4), 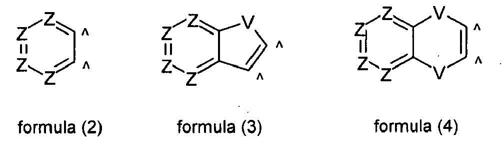 where ^ indicates the corresponding adjacent groups X in the formula (1);
V is on each occurrence, identically or differently, C(R¹)₂, NR¹, O, S, BR¹, Si(R¹)₂ or C=O;
Z is on each occurrence, identically or differently, CR¹ or N;
Ar is on each occurrence, identically or differently, an aromatic or heteroaromatic ring system having 5 to 40 aromatic ring atoms, which may be substituted by one or more radicals R¹;
R is selected on each occurrence, identically or differently, from the group consisting of H, D, F, Cl, Br, I, CN, N(Ar¹)₂, a straight-chain alkyl group having 1 to 40 C atoms or a branched or cyclic alkyl group having 3 to 40 C atoms, each of which may be substituted by one or more radicals R², where one or more non-adjacent CH₂ groups may be replaced by R²C=CR², C=C or O and where one or more H atoms may be replaced by D or F, or an aromatic ring system having 6 to 60 aromatic ring atoms, which may be substituted by one or more radicals R², where the aromatic ring system contains no heteroaryl groups; two adjacent substituents R here may form a monocyclic or polycyclic, aliphatic or aromatic ring system, which may be substituted by one or more radicals R²;
R¹ is selected on each occurrence, identically or differently, from the group consisting of H, D, F, Cl, Br, I, CN, NO₂, N(Ar¹)₂, N(R²)₂, C(=O)Ar¹, C(=O)R², P(=O)(Ar¹)₂, P(Ar¹)₂, B(Ar¹)₂, Si(Ar¹)₃, Si(R²)₃, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, each of which may be substituted by one or more radicals R², where one or more non-adjacent CH₂ groups may be replaced by R²C=CR², C=C, Si(R²)₂, C=O, C=S, C=NR², P(=O)(R²), SO, SO₂, NR², O, S or CONR² and where one or more H atoms may be replaced by D, F, Cl, Br, I, CN or NO₂, an aromatic or heteroaromatic ring system having 5 to 60 aromatic ring atoms, which may in each case be substituted by one or more radicals R², an aryloxy or heteroaryloxy group having 5 to 60 aromatic ring atoms, which may be substituted by one or more radicals R²; two adjacent substituents R¹ here may optionally form a monocyclic or polycyclic, aliphatic, aromatic or heteroaromatic ring system, which may be substituted by one or more radicals R²;
Ar¹ is on each occurrence, identically or differently, an aromatic or heteroaromatic ring system having 5-30 aromatic ring atoms, which may be substituted by one or more non-aromatic radicals R²; two radicals Ar¹ here which are bonded to the same N atom or P atom may also be bridged to one another by a single bond or a bridge selected from N(R²), C(R²)₂, O or S;
R² is selected on each occurrence, identically or differently, from the group consisting of H, D, F, CN, an aliphatic hydrocarbon radical having 1 to 20 C atoms, or an aromatic or heteroaromatic ring system having 5 to 30 aromatic ring atoms, in which one or more H atoms may be replaced by D, F, Cl, Br, I or CN, where two or more adjacent substituents R² may form a mono- or polycyclic, aliphatic ring system with one another;
m, n are on each occurrence, identically or differently, 0 or 1, with the proviso that m + n ≥ 1;
p is on each occurrence, identically or differently, 0, 1, 2, 3 or 4;
q is 0, 1 or 2.

2. Compound according to Claim 1, **characterised in that** X stands, identically or differently on each occurrence, for CR¹ or N, where a maximum of one group X per ring stands for N; or two adjacent groups X stand for a group of the formula (2) or (3), where Z stands, identically or differently on each occurrence, for CR¹ and V stands, identically or differently on each occurrence, for NR¹, C(R¹)₂, O or S.

3. Compound according to Claim 1 or 2, selected from the compounds of the formulae (5) to (12), 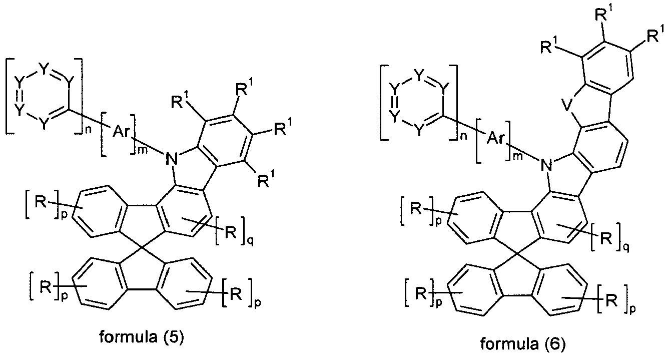 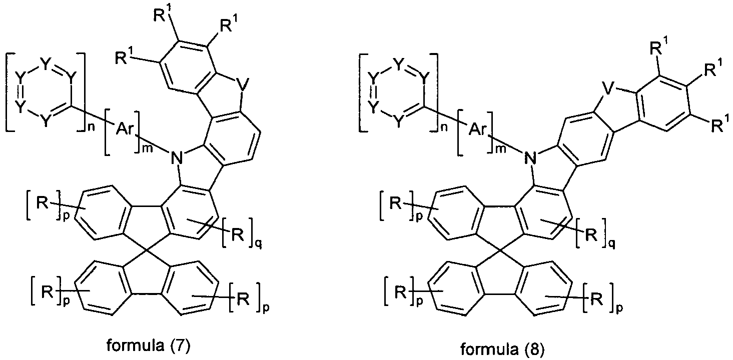 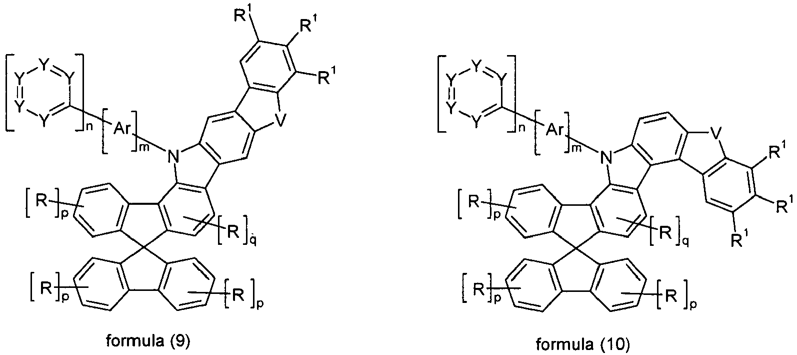 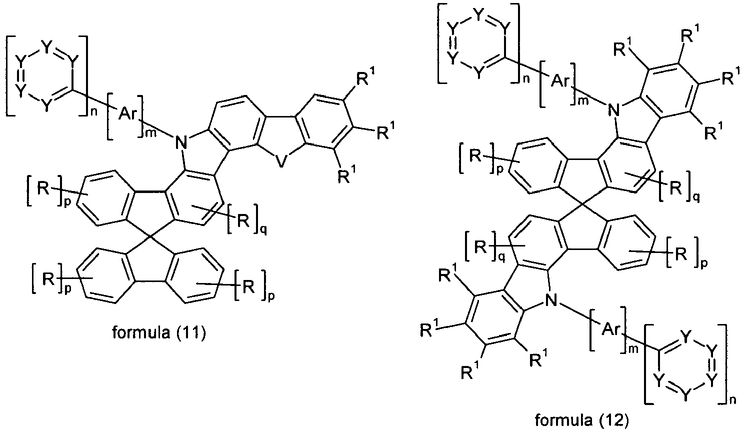 where the symbols and indices used have the meanings given in Claim 1, and V preferably stands for NR¹, C(R¹)₂, O or S.

4. Compound according to one or more of Claims 1 to 3, selected from the structures of the formulae (5a) to (12a), 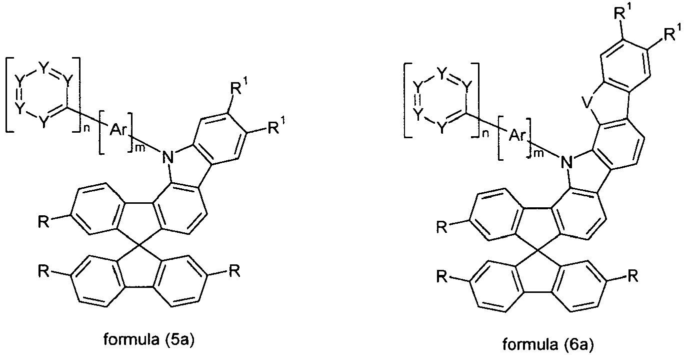 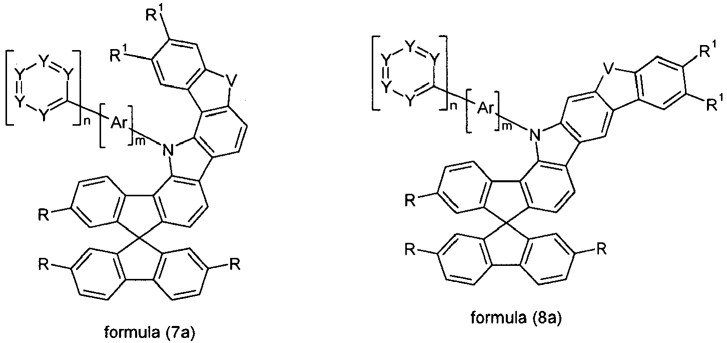 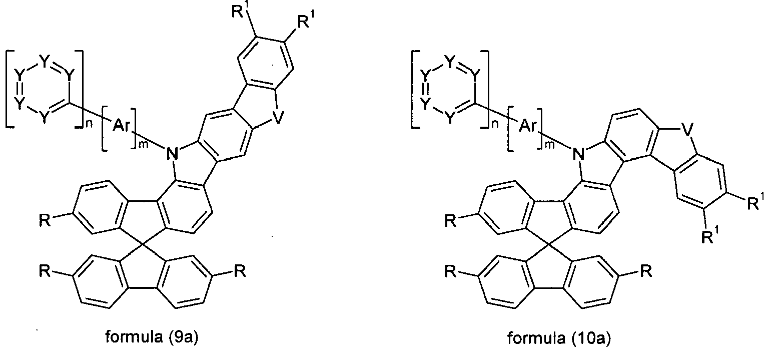 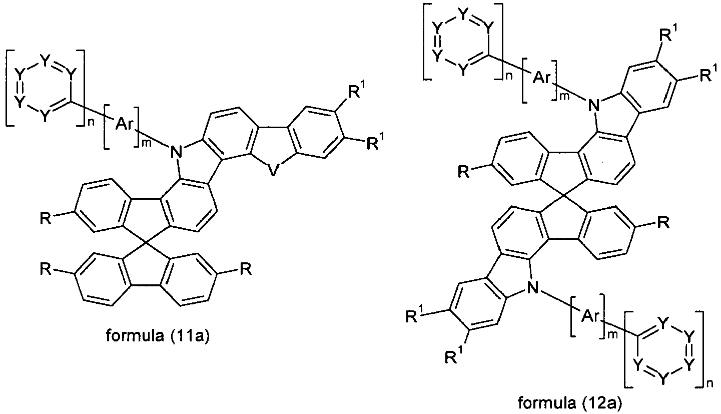 where the symbols and indices used have the meanings given in Claim 1.

5. Compound according to one or more of Claims 1 to 4, **characterised in that** R is selected, identically or differently on each occurrence, from the group consisting of H, F, CN, N(Ar¹)₂, a straight-chain alkyl group having 1 to 10 C atoms or a branched or cyclic alkyl group having 3 to 10 C atoms or an aromatic ring system having 6 to 30 aromatic ring atoms, which may be substituted by one or more non-aromatic radicals R²,
and **in that** R¹ is selected, identically or differently on each occurrence, from the group consisting of H, D, F, Br, CN, N(Ar¹)₂, C(=O)Ar¹, P(=O)-(Ar¹)₂, a straight-chain alkyl or alkoxy group having 1 to 10 C atoms or a branched or cyclic alkyl or alkoxy group having 3 to 10 C atoms or an alkenyl or alkynyl group having 2 to 10 C atoms, each of which may be substituted by one or more radicals R², where one or more non-adjacent CH₂ groups may be replaced by O and where one or more H atoms may be replaced by D or F, an aromatic or heteroaromatic ring system having 5 to 30 aromatic ring atoms, which may in each case be substituted by one or more radicals R².

6. Compound according to one or more of Claims 1 to 5, **characterised in that** Ar is selected from aromatic or heteroaromatic ring systems having 5 to 24 aromatic ring atoms, which may in each case be substituted by one or more radicals R¹, preferably benzene, ortho-, meta- or parabiphenyl, ortho-, meta-, para- or branched terphenyl, ortho-, meta-, para- or branched quaterphenyl, 1-, 2- or 3-fluorenyl, 1-, 2-, 3- or 4-spirobifluorenyl, 1- or 2-naphthyl, pyrrole, furan, thiophene, indole, benzofuran, benzothiophene, 1-, 2- or 3-carbazole, 1-, 2- or 3-dibenzofuran, 1-, 2- or 3-dibenzothiophene, indenocarbazole, indolocarbazole, 2-, 3- or 4-pyridine, 2-, 4- or 5-pyrimidine, pyrazine, pyridazine, triazine, anthracene, phenanthrene, triphenylene, pyrene, benzanthracene, or combinations of two or three of these groups, each of which may be substituted by one or more radicals R¹.

7. Compound according to one or more of Claims 1 to 6, **characterised in that**, in the group of the following formula (Het-Ar), 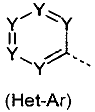 which is present in the compound according to one or more of Claims 1 to 6 for n = 1 and which is bonded to Ar or, for m = 0, to the nitrogen, at least one group Y and a maximum of three groups Y stand for N and the other groups Y stand for CR¹.

8. Compound according to Claim 7, **characterised in that** the group (Het-Ar) is selected from the groups of the formulae (Het-Ar-1) to (Het-Ar-10), 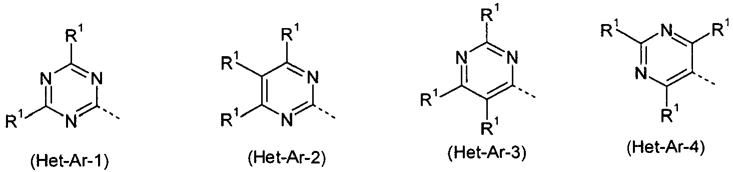 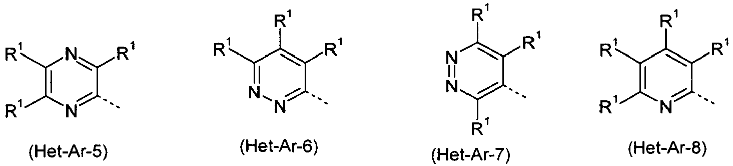 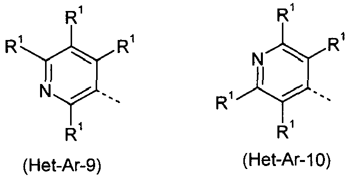 where the dashed bond represents the bond to Ar or, for m = 0, the bond to the nitrogen, and the symbols used have the meanings given in Claim 1.

9. Compound according to one or more of Claims 1 to 8, **characterised in that** it contains no condensed aryl or heteroaryl groups in which more than two six-membered rings are condensed directly onto one another.

10. Process for the preparation of a compound according to one or more of Claims 1 to 9, comprising the reaction steps:
a) synthesis of the skeleton of compound (1) or (1A) which as yet contains no group (Het-Ar) and/or Ar; and
b) reaction of the skeleton from a) in a C-C coupling, such as Suzuki, Negishi, Yamamoto, Grignard-Cross or Stille coupling, etc., or C-N coupling, such as Buchwald or Ullmann coupling.

11. Oligomer, polymer or dendrimer containing one or more of the compounds according to one or more of Claims 1 to 9, where one or more bonds from the compound to the polymer, oligomer or dendrimer are present instead of substituents at one or more positions.

12. Formulation comprising at least one compound according to one or more of Claims 1 to 9 or an oligomer, polymer or dendrimer according to Claim 11 and at least one solvent.

13. Use of a compound according to one or more of Claims 1 to 9 or an oligomer, polymer or dendrimer according to Claim 11 in an electronic device.

14. Electronic device comprising at least one compound according to one or more of Claims 1 to 9 or an oligomer, polymer or dendrimer according to Claim 11, in particular selected from the group consisting of organic electroluminescent devices, organic integrated circuits, organic field-effect transistors, organic thin-film transistors, organic light-emitting transistors, organic solar cells, dye-sensitised organic solar cells, organic optical detectors, organic photoreceptors, organic field-quench devices, light-emitting electrochemical cells, organic laser diodes and "organic plasmon emitting devices".

15. Electronic device according to Claim 14, which is an organic electroluminescent device, **characterised in that** the compound according to one or more of Claims 1 to 9 is employed as matrix material for phosphorescent or fluorescent emitters and/or in an electron-blocking or exciton-blocking layer and/or in a hole-transport layer and/or in a hole-blocking layer and/or in a hole-blocking or electron-transport layer.

## Revendications

1. Composé de la formule (1) ou de la formule (1A) : 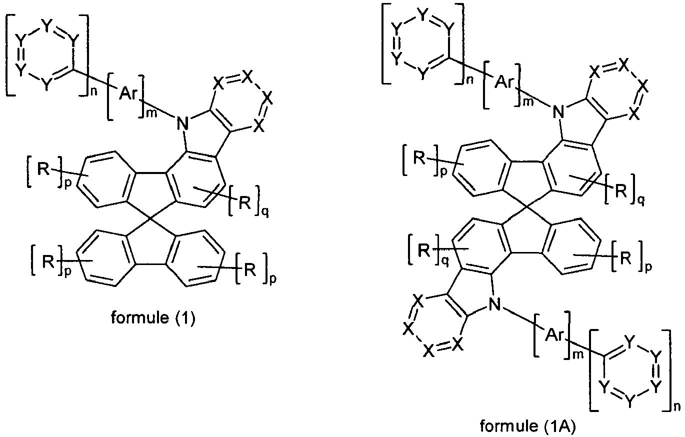 dans lesquelles ce qui suit s'applique aux symboles et indices utilisés :
Y est, pour chaque occurrence, de manière identique ou différente, CR¹ ou N, étant entendu qu'au moins un groupe Y représente N ;
X est, pour chaque occurrence, de manière identique ou différente, CR¹ ou N ; ou deux X adjacents représentent S, O ou NR¹, de telle sorte qu'un cycle à cinq éléments se forme ; ou deux X adjacents représentent un groupe de la formule (2), (3) ou (4) qui suit : 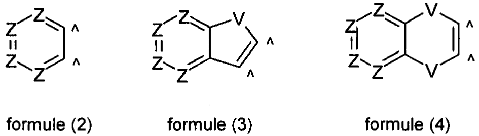 dans lesquelles ^ représente les groupes X adjacents correspondants dans la formule (1) ;
V est, pour chaque occurrence, de manière identique ou différente, C(R¹)₂, NR¹, O, S, BR¹, Si(R¹)₂ ou C=O ;
Z est, pour chaque occurrence, de manière identique ou différente, CR¹ ou N ;
Ar est, pour chaque occurrence, de manière identique ou différente, un système aromatique ou hétéroaromatique qui comporte 5 à 40 atomes de cycle aromatique, lequel peut être substitué par un radical ou plusieurs radicaux R¹;
R est sélectionné pour chaque occurrence, de manière identique ou différente, parmi le groupe qui est constitué par H, D, F, Cl, Br, I, CN, N(Ar¹)₂, un groupe alkyle en chaîne droite qui comporte 1 à 40 atome(s) de C ou un groupe alkyle ramifié ou cyclique qui comporte 3 à 40 atomes de C, dont chacun peut être substitué par un radical ou plusieurs radicaux R², où un ou plusieurs groupe(s) CH₂ non adjacents peut/peuvent être remplacé(s) par R²C=CR², C=C ou O et où un ou plusieurs atome(s) de H peut/peuvent être remplacé(s) par D ou F, ou un système de cycle aromatique qui comporte 6 à 60 atomes de cycle aromatique, lequel peut être substitué par un radical ou plusieurs radicaux R², où le système de cycle aromatique ne contient pas de groupes hétéroaryle ; deux substituants R adjacents peuvent ici former un système de cycle aliphatique ou aromatique monocyclique ou polycyclique, lequel peut être substitué par un radical ou plusieurs radicaux R²;
R¹ est sélectionné pour chaque occurrence, de manière identique ou différente, parmi le groupe qui est constitué par H, D, F, Cl, Br, I, CN, NO₂, N(Ar¹)₂, N(R²)₂, C(=O)Ar¹, C(=O)R², P(=O)(Ar¹)₂, P(Ar¹)₂, B(Ar¹)₂, Si(Ar¹)₃, Si(R²)₃, un groupe alkyle, alcoxy ou thioalkyle en chaîne droite qui comporte 1 à 40 atome(s) C ou un groupe alkyle, alcoxy ou thioalkyle ramifié ou cyclique qui comporte 3 à 40 atomes de C ou un groupe alkényle ou alkynyle qui comporte 2 à 40 atomes de C, dont chacun peut être substitué par un radical ou plusieurs radicaux R², où un ou plusieurs groupe(s) CH₂ non adjacents peut/peuvent être remplacé(s) par R²C=CR², C≡C, Si(R²)₂, C=O, C=S, C=NR², P(=O)(R²), SO, SO₂, NR², O, S ou CONR² et où un ou plusieurs atome(s) de H peut/peuvent être remplacé(s) par D, F, Cl, Br, I, CN ou NO₂, un système de cycle aromatique ou hétéroaromatique qui comporte 5 à 60 atomes de cycle aromatique, lequel peut dans chaque cas être substitué par un radical ou plusieurs radicaux R², un groupe aryloxy ou hétéroaryloxy qui comporte 5 à 60 atomes de cycle aromatique, lequel peut être substitué par un radical ou plusieurs radicaux R²; deux substituants R¹ adjacents peuvent ici en option former un système de cycle aromatique ou hétéroaromatique, aliphatique, monocyclique ou polycyclique, lequel peut être substitué par un radical ou plusieurs radicaux R²;
Ar¹ est pour chaque occurrence, de manière identique ou différente, un système de cycle aromatique ou hétéroaromatique qui comporte 5 à 30 atomes de cycle aromatique, lequel peut être substitué par un radical ou plusieurs radicaux non aromatique(s) R²; deux radicaux Ar¹ ici qui sont liés au même atome de N ou de P peuvent également être pontés l'un à l'autre au moyen d'une liaison simple ou d'un pont qui est sélectionné parmi N(R²), C(R²)₂, O ou S;
R² est sélectionné pour chaque occurrence, de manière identique ou différente, parmi le groupe qui est constitué par H, D, F, CN, un radical hydrocarbone aliphatique qui comporte 1 à 20 atome(s) de C, ou un système de cycle aromatique ou hétéroaromatique qui comporte 5 à 30 atomes de cycle aromatique, dans lequel un ou plusieurs atome(s) de H peut/peuvent être remplacé(s) par D, F, Cl, Br, I ou CN, où deux substituants R² adjacents ou plus peuvent former un système de cycle aliphatique monocyclique ou polycyclique l'un avec l'autre ou les uns avec les autres ;
m, n sont pour chaque occurrence, de manière identique ou différente, 0 ou 1, étant entendu que m + n ≥ 1 ;
p est pour chaque occurrence, de manière identique ou différente, 0, 1, 2, 3 ou 4 ;
q est 0, 1 ou 2.

2. Composé selon la revendication 1, **caractérisé en ce que** X représente, de manière identique ou différente pour chaque occurrence, CR¹ ou N, où un maximum d'un groupe X par cycle représente N ; ou deux groupes adjacents X représentent un groupe de la formule (2) ou (3), où Z représente, de manière identique ou différente pour chaque occurrence, CR¹ et V représente, de manière identique ou différente pour chaque occurrence, NR¹, C(R¹)₂, O ou S.

3. Composé selon la revendication 1 ou 2, qui est sélectionné parmi les composés des formules (5) à (12) : 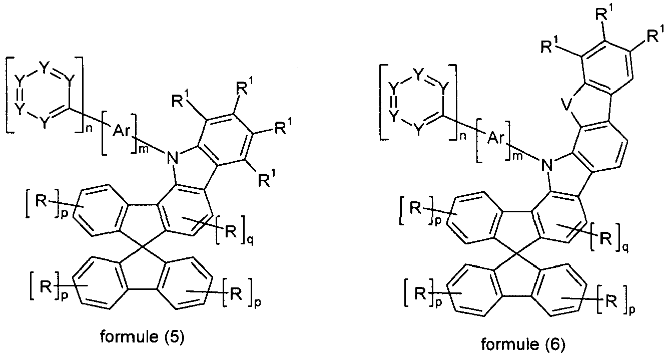 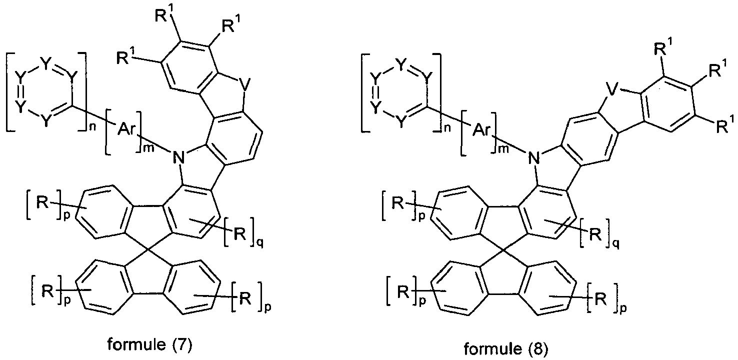 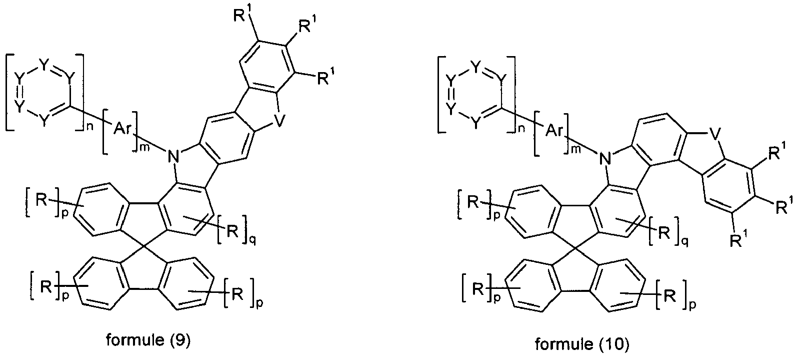 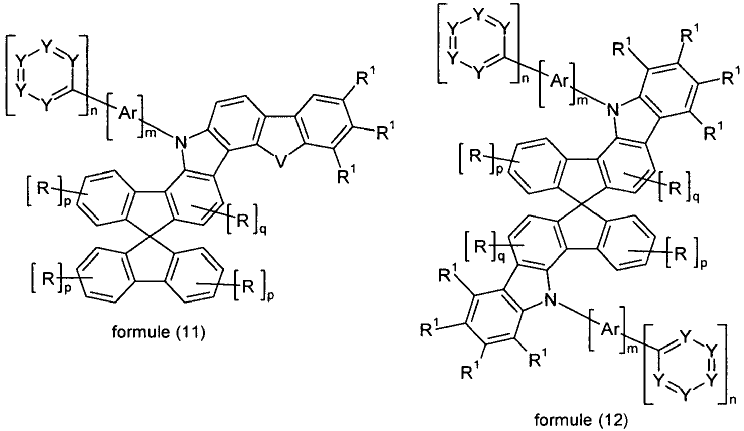 dans lesquelles les symboles et les indices utilisés présentent les significations données selon la revendication 1, et V représente de façon préférable NR¹, C(R¹)₂, O ou S.

4. Composé selon une ou plusieurs des revendications 1 à 3, qui est sélectionné parmi les structures des formules (5a) à (12a) : 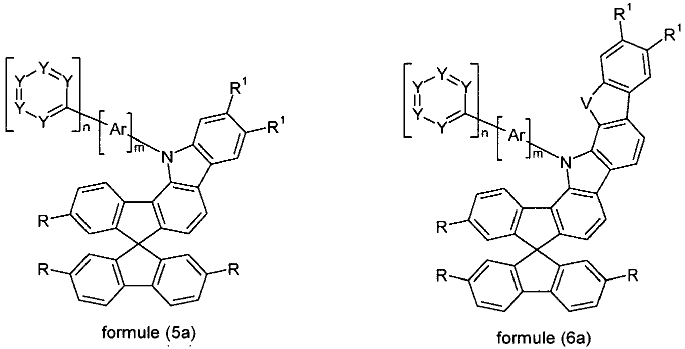 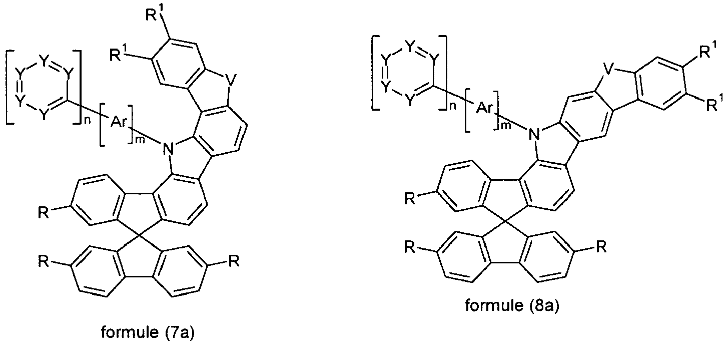 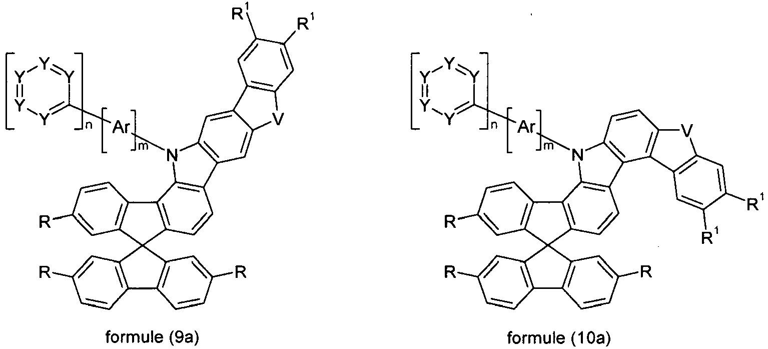 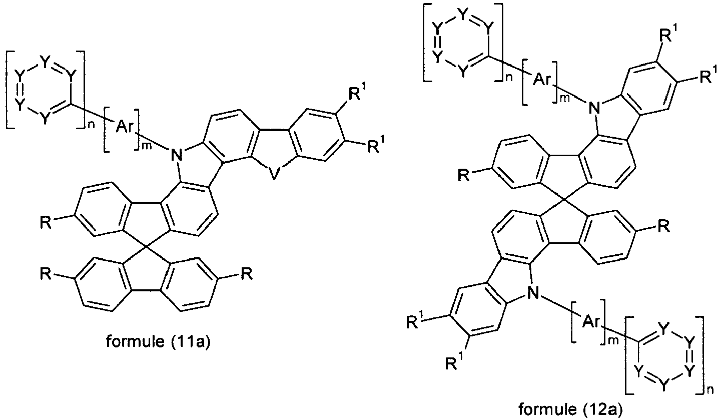 dans lesquelles les symboles et les indices utilisés présentent les significations données selon la revendication 1.

5. Composé selon une ou plusieurs des revendications 1 à 4, **caractérisé en ce que** R est sélectionné, de manière identique ou différente pour chaque occurrence, parmi le groupe qui est constitué par H, F, CN, N(Ar¹)₂, un groupe alkyle en chaîne droite qui comporte 1 à 10 atome(s) C ou un groupe alkyle ramifié ou cyclique qui comporte 3 à 10 atomes C ou un système de cycle aromatique qui comporte 6 à 30 atomes de cycle aromatique, lequel peut être substitué par un radical ou plusieurs radicaux non aromatique(s) R²,
et **en ce que** R¹ est sélectionné, de manière identique ou différente pour chaque occurrence, parmi le groupe qui est constitué par H, D, F, Br, CN, N(Ar¹)₂, C(=O)Ar¹, P(=O)(Ar¹)₂, un groupe alkyle ou alcoxy en chaîne droite qui comporte 1 à 10 atome(s) de C ou un groupe alkyle ou alcoxy ramifié ou cyclique qui comporte 3 à 10 atomes de C ou un groupe alkényle ou alkynyle qui comporte 2 à 10 atomes de C, dont chacun peut être substitué par un radical ou plusieurs radicaux R², où un ou plusieurs groupe(s) CH₂ non adjacents peut/peuvent être remplacé(s) par O et où un ou plusieurs atome(s) de H peut/peuvent être remplacé(s) par D ou F, un système de cycle aromatique ou hétéroaromatique qui comporte 5 à 30 atomes de cycle aromatique, lequel peut dans chaque cas être substitué par un radical ou plusieurs radicaux R².

6. Composé selon une ou plusieurs des revendications 1 à 5, **caractérisé en ce que** Ar est sélectionné parmi des systèmes de cycle aromatique ou hétéroaromatique qui comportent 5 à 24 atomes de cycle aromatique, lesquels peuvent dans chaque cas être substitués par un radical ou plusieurs radicaux R¹, de façon préférable benzène, orthobiphényle, méta-biphényle ou para-biphényle, ortho-terphényle, métaterphényle, para-terphényle ou terphényle ramifié, ortho-quaterphényle, méta-quaterphényle, para-quaterphényle ou quaterphényle ramifié, 1- fluorényle, 2- fluorényle ou 3-fluorényle, 1-, 2- spirobifluorényle, 3- spirobifluorényle ou 4-spirobifluorényle, 1-naphtyle ou 2-naphtyle, pyrrole, furane, thiophène, indole, benzofurane, benzothiophène, 1- carbazole, 2- carbazole ou 3-carbazole, 1- dibenzofurane, 2-dibenzofurane ou 3-dibenzofurane, 1- dibenzothiophène, 2- dibenzothiophène ou 3-dibenzothiophène, indénocarbazole, indolocarbazole, 2- pyridine, 3- pyridine ou 4-pyridine, 2- pyrimidine, 4- pyrimidine ou 5-pyrimidine, pyrazine, pyridazine, triazine, anthracène, phénanthrène, triphénylène, pyrène, benzanthracène, ou des combinaisons de deux ou trois de ces groupes, dont chacun peut être substitué par un radical ou plusieurs radicaux R¹.

7. Composé selon une ou plusieurs des revendications 1 à 6, **caractérisé en ce que**, dans le groupe de la formule (Het-Ar) qui suit : 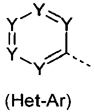 lequel est présent dans le composé selon une ou plusieurs des revendications 1 à 6 pour n = 1 et lequel est lié à Ar ou, pour m = 0, à l'azote, au moins un groupe Y et un maximum de trois groupes Y représentent N et les autres groupes Y représentent CR¹.

8. Composé selon la revendication 7, **caractérisé en ce que** le groupe (Het-Ar) est sélectionné parmi les groupes des formules (Het-Ar-1) à (Het-Ar-10) : 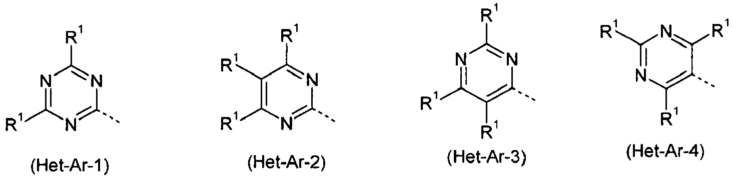 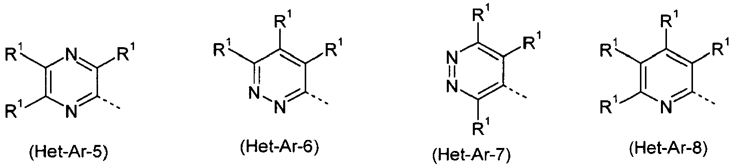 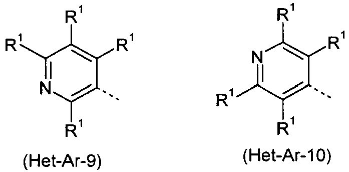 dans lesquelles la liaison en pointillés représente la liaison sur Ar ou, pour m = 0, la liaison sur l'azote, et les symboles utilisés présentent les significations données selon la revendication 1.

9. Composé selon une ou plusieurs des revendications 1 à 8, **caractérisé en ce qu'**il ne contient pas de groupes aryle ou hétéroaryle condensés dans lesquels plus de deux cycles à six éléments sont condensés directement les uns sur les autres.

10. Procédé pour la préparation d'un composé selon une ou plusieurs des revendications 1 à 9, comprenant les étapes de réaction qui suivent :
a) synthèse du squelette du composé (1) ou (1A) qui ne contient pas pour l'instant de groupe (Het-Ar) et/ou Ar ; et
b) réaction du squelette issu de a) selon un couplage C-C, tel qu'un couplage de Suzuki, de Negishi, de Yamamoto, de Grignard-Cross ou de Stille, etc., ou selon un couplage C-N, tel qu'un couplage de Buchwald ou de Ullmann.

11. Oligomère, polymère ou dendrimère qui contient un ou plusieurs des composés selon une ou plusieurs des revendications 1 à 9, où une ou plusieurs liaison(s) depuis le composé sur le polymère, l'oligomère ou le dendrimère est/sont présentes en lieu et place de substituants au niveau d'une ou de plusieurs position(s).

12. Formulation comprenant au moins un composé selon une ou plusieurs des revendications 1 à 9 ou un oligomère, un polymère ou un dendrimère selon la revendication 11 et au moins un solvant.

13. Utilisation d'un composé selon une ou plusieurs des revendications 1 à 9 ou d'un oligomère, d'un polymère ou d'un dendrimère selon la revendication 11 dans un dispositif électronique.

14. Dispositif électronique comprenant au moins un composé selon une ou plusieurs des revendications 1 à 9 ou un oligomère, un polymère ou un dendrimère selon la revendication 11, en particulier sélectionné parmi le groupe qui est constitué par les dispositifs électroluminescents organiques, les circuits intégrés organiques, les transistors à effet de champ organiques, les transistors à film mince organiques, les transistors à émission de lumière organiques, les cellules solaires organiques, les cellules solaires organiques sensibilisées par colorant, les détecteurs optiques organiques, les photorécepteurs organiques, les dispositifs à extinction de champ organiques, les cellules électrochimiques à émission de lumière, les diodes laser organiques et les "dispositifs à émission de plasmons organiques".

15. Dispositif électronique selon la revendication 14, lequel est un dispositif électroluminescent organique, **caractérisé en ce que** le composé selon une ou plusieurs des revendications 1 à 9 est utilisé en tant que matériau de matrice pour des émetteurs phosphorescents ou fluorescents et/ou dans une couche de blocage d'électrons ou de blocage d'excitons et/ou dans une couche de transport de trous et/ou dans une couche de blocage de trous et/ou dans une couche de blocage de trous ou de transport d'électrons.
